# NEW EUROPEAN PATENT SPECIFICATION

(11) **EP 0 601 106 B2**
(45) Date of publication and mention of the opposition decision: **05.11.2003**
(45) Mention of the grant of the patent: 17.05.2000
(21) Application number: 92919544.4
(22) Date of filing: 28.08.1992
(51) Int. Cl.: A61K 38/00

(54) **MORPHOGEN-INDUCED MODULATION OF INFLAMMATORY RESPONSE**
Morphogen-induzierte Modulation von entzündlichen Antworten
MODULATION, INDUITE PAR MORPHOGENE, DE REACTION INFLAMMATOIRE

(30) Priority: 30.08.1991 US 752764; 30.08.1991 US 752861; 30.08.1991 US 753059
(43) Date of publication of application: 15.06.1994
(73) Proprietor: Curis, Inc., Cambridge, MA 02138 (US)
(72) Inventor: KUBERASAMPATH, Thangavel, Medway, MA 02053 (US); PANG, Roy, H., L., Etna, NH 03750 (US); OPPERMANN, Hermann, Medway, MA 02053 (US); RUEGER, David, C., Hopkinton, MA 01748 (US); COHEN, Charles, M., Medway, MA 02053 (US); OZKAYNAK, Engin, Milford, MA 01757 (US); SMART, John, E., Weston, MA 02193 (US)
(74) Representative: Price, Vincent Andrew
(86) International application number: US9207358
(87) International publication number: WO93004692

(56) References cited:
- EP-A- 0 269 408
- EP-A- 0 429 570
- EP-A- 0 665 739
- WO-A-01/74379
- WO-A-84/01106
- WO-A-88/00205
- WO-A-89/09788
- WO-A-90/00900
- WO-A-91/05802
- WO-A-92/07073
- WO-A-92/15323
- WO-A-99/21574
- US-A- 4 981 787
- US-A- 5 008 240
- SCIENCE vol. 249, no. 4964, 6 July 1990, LANCASTER, PA US pages 61 - 64 LEFER A.M. ET AL 'Mediation of cardioprotection by Transforming growth factor-beta' cited in the application
- PROCEEDINGS OF THE NATIONAL ACADEMY OF SCIENCES OF USA. vol. 88, no. 7, April 1991, WASHINGTON US pages 2918 - 2921 KURUVILLA, A.P. 'Protective effect of transforming growth factor beta1 on experimental autoimmune diseases in mice' cited in the application
- EMBO JOURNAL vol. 9, no. 7, 1990, EYNSHAM, OXFORD GB pages 2085 - 2093 ZKAYNAK, E. ET AL 'OP-1 cDNA encodes an osteogenic protein in the TGF-beta family'
- Clinical Orthopaedics and Related Research, 250, Jan. 1990, Bonewald L.F. and Mundy, G.R.
- Alignment to the homology
- Pschyrembel, Def. of Ulcus, p. 1736
- Genealogy. net.
- Roche Lexikon, 1984, p. 412, def. for "pus"
- Pschyrembel, Def. for "pus", p. 410
- Internetpublikation, def. of "periodontitis"
- Annals of Periodontology, Vol. 4, no. 1, Dec.1990

## Description

### Field of the Invention

The present invention finds generally utility in a method for modulating the inflammatory response induced in a mammal following tissue injury. More particularly, this invention finds utility in a method for alleviating immune-cell mediated tissue destruction associated with the inflammatory response.

### Background of the Invention

The body's inflammatory response to tissue injury can cause significant tissue destruction, leading to loss of tissue function. Damage to cells resulting from the effects of inflammatory response e.g., by immune-cell mediated tissue destruction, has been implicated as the cause of reduced tissue function or loss of tissue function in diseases of the joints (e.g., rheumatoid and osteo-arthritis) and of many organs, including the kidney, pancreas, skin, lung and heart. For example, glomular nephritis, diabetes, inflammatory bowel disease, vascular diseases such as atheroclerosis and vasculitis, and skin diseases such as psoriasis and dermatitis are believed to result in large part from unwanted acute inflammatory reaction and fibrosis. A number of these diseases, including arthritis, psoriasis and inflammatory bowel disease are considered to be chronic inflammatory diseases. The damaged tissue also often is replaced by fibrotic tissue, e.g., scar tissue, which further reduces tissue function. Graft and transplanted organ rejection also is believed to be primarily due to the action of the body's immune/inflammatory response system.

The immune-cell mediated tissue destruction often follows an initial tissue injury or insult. The secondary damage, resulting from the inflammatory response, often is the source of significant tissue damage. Among the factors thought to mediate these damaging effects are those associated with modulating the body's inflammatory response following tissue injury, e.g., cytokines such as interleukin-1 (IL-1) and tumor necrosis factor (TNF), and oxygen-derived free radicals such as superoxide anions. These humoral agents are produced by adhering neutrophilic leukocytes or by endothelial cells and have been identified at ischemic sites upon reperfusion. Moreover, TNF concentrations are increased in humans after myocardial infarction.

A variety of lung diseases are characterized by airway inflammation, including chronic bronchitis, emphysema, idiopathic pulmonary fibrosis and asthma. Another type of lung-related inflammation disorders are inflammatory diseases characterized by a generalized, wide-spread acute inflammatory response such as adult respiratory distress syndrome. Another dysfunction associated with the inflammatory response is that mounted in response to injury caused by hyperoxia, e.g., prolonged exposure to lethally high concentrations of O₂ (95-100% O₂). Similarly, reduced blood flow to a tissue (and, therefore reduced or lack of oxygen to tissues), as described below, also can induce a primary tissue injury that stimulates the inflammatory response.

It is well known that damage occurs to cells in mammals which have been deprived of oxygen. In fact, the interruption of blood flow, whether partial (hypoxia) or complete (ischemia) and the ensuing inflammatory responses may be the most important cause of coagulative necrosis or cell death in human disease. The complications of atherosclerosis, for example, are generally the result of ischemic cell injury in the brain, heart, small intestines, kidneys, and lower extremities. Highly differentiated cells, such as the proximal tubular cells of the kidney, cardiac myocytes, and the neurons of the central nervous system, all depend on aerobic respiration to produce ATP, the energy necessary to carry out their specialized functions. When ischemia limits the oxygen supply and ATP is depleted, the affected cells may become irreversibly injured. The ensuing inflammatory responses to this initial injury provide additional insult to the affected tissue. Examples of such hypoxia or ischemia are the partial or total loss of blood supply to the body as a whole, an organ within the body, or a region within an organ, such as occurs in cardiac arrest, pulmonary embolus, renal artery occlusion, coronary occlusion or occlusive stroke.

The tissue damage associated with ischemia-reperfusion injury is believed to comprise both the initial cell damage induced by the deprivation of oxygen to the cell and its subsequent recirculation, as well as the damage caused by the body's response to this initial damage. It is thought that reperfusion injury may result in dysfunction to the endothelium of the vasculature as well as injury to the surrounding tissue. In idiopathic pulmonary fibrosis, for example, scar tissue accumulates on the lung tissue lining, inhibiting the tissue's elasticity. The tissue damage associated with hyperoxia injury is believed to follow a similar mechanism, where the initial damage is mediated primarily through the presence of toxic oxygen metabolites followed by an inflammatory response to this initial injury.

Similarly, tissues and organs for transplantation also are subject to the tissue destructive effects associated with the recipient host body's inflammatory response following transplantation. It is currently believed that the initial destructive response is due in large part to reperfusion injury to the transplanted organ after it has been transplanted to the organ recipient.

Accordingly, the success of organ or tissue transplantation depends greatly on the preservation of the tissue activity (e.g., tissue or organ viability) at the harvest of the organ, during storage of the harvested organ, and at transplantation. To date, preservation of organs such as lungs, pancreas, heart and liver remains a significant stumbling block to the successful transplantation of these organs. U.S. Patent No. 4,952,409 describes a superoxide dismutase-containing liposome to inhibit reperfusion injury. U.S. Patent No. 5,002,965 describes the use of ginkolides, known platelet activating factor antagonists, to inhibit reperfusion injury. Both of these factors are described working primarily by inhibiting the release of and/or inhibiting the damaging effects of free oxygen radicals. A number of patents also have issued on the use of immunosuppressants for inhibiting graft rejection. A representative listing includes U.S. Patent Nos. 5,104,858, 5,008,246 and 5,068,323. A significant problem with many immunosuppressants is their low therapeutic index, requiring the administration of high doses that can have significant toxic side effects.

Rheumatoid and osteoarthritis are prevalent diseases characterized by chronic inflammation of the synovial membrane lining the afflicted joint. A major consequence of chronic inflammatory joint disease (e.g., rheumatoid arthritis) and degenerative arthritis (e.g., osteoarthritis) is loss of function of those affected joints. This loss of function is due primarily to destruction of the major structural components of the joint, cartilage and bone, and subsequent loss of the proper joint anatomy. As a consequence of chronic disease, joint destruction ensues and can lead to irreversible and permanent damage to the joint and loss of function. Current treatment methods for severe cases of rheumatoid arthritis typically include the removal of the synovial membrane, e.g., synovectomy. Surgical synovectomy has many limitations, including the risk of the surgical procedure itself, and the fact that a surgeon often cannot remove all of the diseased membrane. The diseased tissue remaining typically regenerates, causing the same symptoms which the surgery was meant to alleviate.

Psoriasis is a chronic, recurrent, scaling skin disease of unknown etiology characterized by chronic inflammation of the skin. Erythematous eruptions, often in papules or plaques, and usually having a white silvery scale, can affect any part of the skin, but most commonly affect the scalp, elbows, knees and lower back. The disease usually occurs in adults, but children may also be affected. Patients with psoriasis have a much greater incidence of arthritis (psoraitic arthritis), and generalized exfoliation and even death can threaten afflicted individuals.

Current therapeutic regimens include topical or intralesional application of corticosteroids, topical administration of keratolytics, and use of tar and UV light on affected areas. No single therapy is ideal, and it is rare for a patient not to be treated with several alternatives during the relapsing and remitting course of the disease. Whereas systematic treatment can induce prompt resolution of psoriatic lesions, suppression often requires ever-increasing doses, sometimes with toxic side effect, and tapering of therapy may result in rebound phenomena with extensions of lesions, possibly to exfoliation.

Inflammatory bowel disease (IBD) describes a class of clinical disorders of the gastrointestinal mucosa characterized by chronic inflammation and severe ulceration of the mucosa. The two major diseases in this classification are ulcerative colitis and regional enteritis (Crohn's Disease). Like oral mucositis, the diseases classified as IBD are associated with severe mucosal ulceration (frequently penetrating the wall of the bowel and forming strictures and fistulas), severe mucosal and submucosal inflammation and edema, and fibrosis (e.g., scar tissue formation which interferes with the acid protective function of the gastrointestinal lining.) Other forms of IBD include regional ileitis and proctitis. Clinically, patients with fulminant IBD can be severely ill with massive diarrhea, blood loss, dehydration, weight loss and fever. The prognosis of the disease is not good and frequently requires resection of the diseased tissue.

The present invention finds application in a method for protecting mammalian tissue, particularly human tissue, from the damage associated with the inflammatory response following a tissue injury. The inflammatory reaction may be in response to an initial tissue injury or insult. The original injury may be chemically, mechanically, biologically or immunologically related. Another application is in methods and compositions for protecting tissue from the tissue destructive effects associated with chronic inflammatory diseases, including arthritis (e.g., reheumatoid or osteoarthritis), psoriatic arthritis, psoriasis and dermatitis, inflammatory bowel disease and other autoimmune diseases. Yet another application is in methods and compositions for enhancing the viability of mammalian tissues and organs to be transplanted, including protecting the transplanted organs from immune cell-mediated tissue destruction, such as the tissue damage associated with ischemia-reperfusion injury. This tissue damage may occur during donor tissue or organ harvesting and transport, as well as following initiation of blood flow after transplantation of the organ or tissue in the recipient host.

The invention also finds application in a method for alleviating tissue damage associated with ischemic-reperfusion injury in a mammal following a deprivation of oxygen to a tissue in the mammal. Other applications include providing a method for alleviating tissue damage associated with ischemic-reperfusion injury in a human which has suffered from hypoxia or ischemia following cardiac arrest, pulmonary embolus, renal artery occlusion, coronary occlusion or occlusive stroke. A further application is to provide a method for alleviating tissue damage associated with hyperoxia-induced tissue injury, e.g., lethally high oxygen concentrations.

Still another application of the invention is to provide a method for modulating inflammatory responses in general, particularly those induced in a human following tissue injury.

These and other objects and features of the invention will be apparent from the description, drawings and claims which follow.

Each of Kurvilla et al. (1991), 88 Proc. Natl. Acad. Sci. USA 2918-2921, Lefer et al. (1990), 249 Science 61-64; Shepard et. al., EP 0,269,408; Nathan et al., W090/00900; and, Bentz et al., U.S. Pat. 4,971,952 describe studies testing whether TGFβ can mitigate specific types of inflammatory or ischemia reperfusion damage to mammalian cells or tissues. TGF-β is not a member of the class of proteins defined herein as morphogens.

Each of Oppermann et. al., W091/05802; Kuberasampath et al., WO89/09787; Oppermann et al., WO89/09788; and Oppermann et al. W092/07073 teach that OP-1 and related proteins may be used in stimulating tissue-specific regeneration of mammalian cartilage and bone tissue. In particular, these references teach that OP-1, when adsorbed on a suitable supporting matrix, induces the developmental cascade of cellular and molecular events that culminates in endochondral bone morphogenesis. The biologically active OP-1 preparations are taught to be, at best, sparingly soluble in physiologically compatible solutions. OP-1-charged matrix devices thus are disclosed as useful for inducing local morphogenesis of bone and/or cartilage.

Cohen et al., W092/15323 teaches that OP-1 induces tissue specific morphogenesis in diverse mammalian body tissues, and that morphogen can be used in the replacement or repair of damaged tissues.

### Summary of the Invention

The present invention is defined in the claims. It finds utility in a method for alleviating the tissue destructive effects associated with activation of the inflammatory response following tissue injury. The method comprises the step of providing to the affected tissue a therapeutically effective concentration of a morphogenic protein ("morphogen", as defined herein) upon tissue injury or in anticipation of tissue injury, sufficient to substantially inhibit or reduce the tissue destructive effects of the inflammatory response.

Also described herein are compositions and therapeutic treatment methods that comprise the step of administering to a mammal a therapeutically effective amount of a morphogenic protein ("morphogen"), as defined herein, upon injury to a tissue, or in anticipation of such injury, for a time and at a concentration sufficient to inhibit the tissue destructive effects associated with the body's inflammatory response, including repairing damaged tissue, and/or inhibiting additional damage thereto.

As embodied herein, the term "ischemic-reperfusion injury" refers to the initial damage associated with oxygen deprivation of a cell and the subsequent damage associated with the inflammatory response when the cell is resupplied with oxygen. As embodied herein, the term "hyperoxia-induced injury" refers to the tissue damage associated with prolonged exposure to lethally high doses of oxygen, e.g., greater than 95% O₂, including the tissue damage associated with the inflammatory response to the toxically high oxygen dose. Accordingly, as used herein, "toxic oxygen concentrations" refers to the tissue damage associated withthe injury induced by both lethally low oxygen concentrations of oxygen (including a complete lack of oxygen), and by lethally high oxygen concentrations. The expression "alleviating" means the protection from, reduction of and/or elimination of undesired tissue destruction, particularly immune cell-mediated tissue destruction. The tissue destruction may be in response to an initial tissue injury, which may be mechanical, chemical or immunological in origin. The expression "enhance the viability of" living tissues or organs, as used herein, means protection from, reduction of and/or elimination of reduced or lost tissue or organ function as a result of tissue death, particularly immune cell-mediated tissue death. "Transplanted" living tissue encompasses both tissue transplants (e.g., as in the case of bone marrow transplants) and tissue grafts. Finally, a "free oxygen radical inhibiting agent" means a molecule capable of inhibiting the release of and/or inhibiting tissue damaging effects of free oxygen radicals.

Described herein are methods and compositions for alleviating the ischemic-reperfusion injury in mammalian tissue resulting from a deprivation of, and subsequent reperfusion of, oxygen to the tissue. Also described is a method for alleviating the tissue-destructive effects associated with hyperoxia. Methods and compositions for maintaining the viability of tissues and organs, particularly living tissues and organs to be transplanted, including protecting them from ischemia-reperfusion injury, together with methods for protecting tissues and organs from the tissue destructive effects of chronic inflammatory diseases, such as arthritis, psoriasis, dermatitis, including contact dermatitis, IBD and other chronic inflammatory diseases of the gastrointestinal tract, as well as the tissue destructive effects associated with other, known autoimmune diseases, such as diabetes, multiple sclerosis, amyotrophic lateral sclerosis (ALS), and other autoimmune neurodegenerative diseases are also described herein.

The morphogen may be provided to the damaged tissue following an initial injury to the tissue. The morphogen may be provided directly to the tissue, as by injection to the damaged tissue site or by topical administration, or may be provided indirectly, e.g., systemically by oral or parenteral means.

The morphogen may also be provided to tissue at risk of damage due to immune cell-mediated tissue destruction. Examples of such tissues include tissue grafts and tissue or organ transplants, as well as any tissue or organ about to undergo a surgical procedure or other clinical procedure likely to either inhibit blood flow to the tissue or otherwise induce an inflammatory response. Here the morphogen preferably is provided to the patient prior to induction of the injury, e.g., as a prophylactic, to provide a cyto-protective effect to the tissue at risk.

Where the tissue at risk comprises a tissue or organ to be transplanted, the tissue or organ to be transplanted preferably is exposed to a morphogen prior to transplantation. Most preferably, the tissue or organ is exposed to the morphogen prior to its removal from the donor, by providing the donor with a composition comprising a morphogen. Alternatively or, in addition, once removed from the donor, the organ or tissue is placed in a preservation solution containing a morphogen. In addition, the recipient also preferably is provided with a morphogen just prior to, or concommitant with, transplantation. In all cases, the morphogen may be administered directly to the tissue at risk, as by injection or topical administration to the tissue, or it may be provided systemically, either by oral or parenteral administration.

The morphogens described herein are envisioned to be useful in enhancing viability of any organ or living tissue to be transplanted. The morphogens may be used to particular advantage in lung, heart, liver, kidney or pancreas transplants, as well as in transplantation and/or grafting of bone marrow, skin, gastrointestinal mucosa, and other living tissues.

Where the patient suffers from a chronic inflammatory disease, such as diabetes, arthritis, psoriasis, IBD, and the like, the morphogen preferably is administered at regular intervals as a prophylactic, to prevent and/or inhibit the tissue damage normally associated with the disease during flare periods. As above, the morphogen may be provided directly to the tissue at risk, for example by injection or by topical administration, or indirectly, as by systemic e.g., oral or parenteral administration.

Among the morphogens useful in this invention are proteins originally identified as osteogenic proteins, such as the OP-1, OP-2 and CBMP2 proteins, as well as amino acid sequence-related proteins such as DPP (from Drosophila), Vgl (from Xenopus), Vgr-1 (from mouse, see U.S. 5,011,691 to Oppermann et al.), GDF-1 (from mouse, see Lee (1991) PNAS 88:4250-4254), all of which are presented in Table II and Seq. ID Nos.5-14), and the recently identified 60A protein (from Drosophila, Seq. ID No. 24, see Wharton et al. (1991) PNAS 88:9214-9218.) The members of this family, which include members of the TGF-β super-family of proteins, share substantial amino acid sequence homology in their C-terminal regions. The proteins are translated as a precursor, having an N-terminal signal peptide sequence, typically less than about 30 residues, followed by a "pro" domain that is cleaved to yield the mature sequence. The signal peptide is cleaved rapidly upon translation, at a cleavage site that can be predicted in a given sequence using the method of Von Heijne ((1986) Nucleic Acids Research 14:4683-4691.) Table I, below, describes the various morphogens identified to date, including their nomenclature as used herein, their Seq. ID references, and publication sources for the amino acid sequences for the full length proteins not included in the Seq. Listing.

**TABLE I**

| | |
|---|---|
| "OP-1" | Refers generically to the group of morphogenically active proteins expressed from part or all of a DNA sequence encoding OP-1 protein, including allelic and species variants thereof, e.g., human |
| | OP-1 ("hOP-1", Seq. ID No. 5, mature protein amino acid sequence), or mouse OP-1 ("mOP-1", Seq. ID No. 6, mature protein amino acid sequence.) The conserved seven cysteine skeleton is defined by residues 38 to 139 of Seq. ID Nos. 5 and 6. The cDNA sequences and the amino acids encoding the full length proteins are provided in Seq. Id Nos. 16 and 17 (hOP1) and Seq. ID Nos. 18 and 19 (mOP1.) The mature proteins are defined by residues 293-431 (hOP1) and 292-430 (mOP1). The "pro" regions of the proteins, cleaved to yield the mature, morphogenically active proteins are defined essentially by residues 30-292 (hOP1) and residues 30-291 (mOP1). |
| "OP-2" | refers generically to the group of active proteins expressed from part or all of a DNA sequence encoding OP-2 protein, including allelic and species variants thereof, e.g., human OP-2 ("hOP-2", Seq. ID No. 7, mature protein amino acid sequence) or mouse OP-2 ("mOP-2", Seq. ID No. 8, mature protein amino acid sequence). The conserved seven cysteine skeleton is defined by residues 38 to 139 of Seq. ID Nos. 7 and 8. The cDNA sequences and the amino acids encoding the full length proteins are provided in Seq. ID Nos. 20 and 21 (hOP2) and Seq. ID Nos. 22 and 23 (mOP2.) The mature proteins are defined essentially by residues 264-402 (hOP2) and 261-399 (mOP2). The "pro" |
| | regions of the proteins, cleaved to yield the mature, morphogenically active proteins likely are defined essentially by residues 18-263 (hOP2) and residues 18-260 (mOP2). (Another cleavage site also occurs 21 residues upstream for both OP-2 proteins.) |
| "CBMP2" | refers generically to the morphogenically active proteins expressed from a DNA sequence encoding the CBMP2 proteins, including allelic and species variants thereof, e.g., human CBMP2A ("CBMP2A(fx)", Seq ID No. 9) or human CBMP2B DNA ("CBMP2B(fx)", Seq. ID No. 10). The amino acid sequence for the full length proteins, referred to in the literature as BMP2A and BMP2B, or BMP2 and BMP4, appear in Wozney, et al. (1988) Science 242:1528-1534. The pro domain for BMP2 (BMP2A) likely includes residues 25-248 or 25-282; the mature protein, residues 249-396 or 283-396. The pro domain for BMP4 (BMP2B) likely includes residues 25-256 or 25-292; the mature protein, residues 257-408 or 293-408. |
| "DPP(fx)" | refers to protein sequences encoded by the Drosophila DPP gene and defining the conserved seven cysteine skeleton (Seq. ID No. 11). The amino acid sequence for the full length protein appears in Padgett, et |
| | al (1987) Nature 325: 81-84. The pro domain likely extends from the signal peptide cleavage site to residue 456; the mature protein likely is defined by residues 457-588. |
| "Vgl(fx)" | refers to protein sequences encoded by the Xenopus Vgl gene and defining the conserved seven cysteine skeleton (Seq. ID No. 12). The amino acid sequence for the full length protein appears in Weeks (1987) Cell 51: 861-867. The prodomain likely extends from the signal peptide cleavage site to residue 246; the mature protein likely is defined by residues 247-360. |
| "Vgr-1(fx)" | refers to protein sequences encoded by the murine Vgr-1 gene and defining the conserved seven cysteine skeleton (Seq. ID No. 13). The amino acid sequence for the full length protein appears in Lyons, et al, (1989) PNAS 86: 4554-4558. The prodomain likely extends from the signal peptide cleavage site to residue 299; the mature protein likely is defined by residues 300-438. |
| "GDF-1(fx)" | refers to protein sequences encoded by the human GDF-1 gene and defining the conserved seven cysteine skeleton (Seq. ID No. 14). The cDNA and encoded amino sequence for the full length protein is |
| | provided in Seq. ID. No. 32. The prodomain likely extends from the signal peptide clavage site to residue 214; the mature protein likely is defined by residues 215-372. |
| "60A" | refers generically to the morphogenically active proteins expressed from part or all of a DNA sequence (from the Drosophila 60A gene) encoding the 60A proteins (see Seq. ID No. 24 wherein the cDNA and encoded amino acid sequence for the full length protein is provided). "60A(fx)" refers to the protein sequences defining the conserved seven cysteine skeleton (residues 354 to 455 of Seq. ID No. 24.) The prodomain likely extends from the signal peptide cleavage site to residue 324; the mature protein likely is defined by residues 325-455. |
| "BMP3(fx)" | refers to protein sequences encoded by the human BMP3 gene and defining the conserved seven cysteine skeleton (Seq. ID No. 26). The amino acid sequence for the full length protein appears in Wozney et al. (1988) Science 242: 1528-1534. The pro domain likely extends from the signal peptide cleavage site to residue 290; the mature protein likely is defined by residues 291-472. |
| "BMP5(fx)" | refers to protein sequences encoded by the human BMP5 gene and defining the conserved seven cysteine skeleton (Seq. ID No. 27). The amino acid sequence for the full length protein appears in Celeste, et al. (1991) PNAS 87: 9843-9847. The pro domain likely extends from the signal peptide cleavage site to residue 316; the mature protein likely is defined by residues 317-454. |
| "BMP6(fx)" | refers to protein sequences encoded by the human BMP6 gene and defining the conserved seven cysteine skeleton (Seq. ID No. 28). The amino acid sequence for the full length protein appears in Celeste, et al. (1990) PNAS 87: 9843-5847. The pro domain likely includes extends from the signal peptide cleavage site to residue 374; the mature sequence likely includes residues 375-513. |

The OP-2 proteins have an additional cysteine residue in this region (e.g., see residue 41 of Seq. ID Nos. 7 and 8), in addition to the conserved cysteine skeleton in common with the other proteins in this family. The GDF-1 protein has a four amino acid insert within the conserved skeleton (residues 44-47 of Seq. ID No. 14) but this insert likely does not interfere with the relationship of the cysteines in the folded structure. In addition, the CBMP2 proteins are missing one amino acid residue within the cysteine skeleton.

The morphogens are inactive when reduced, but are active as oxidized homodimers and when oxidized in combination with other morphogens (e.g., as heterodimers). Thus, as defined herein, a morphogen is a dimeric protein comprising a pair of polypeptide chains, wherein each polypeptide chain comprises at least the C-terminal six cysteine skeleton defined by residues 43-139 of Seq. ID No. 5 including functionally equivalent arrangements of these cysteines (e.g., amino acid insertions or deletions which alter the linear arrangement of the cysteines in the sequence but not their relationship in the folded structure), such that, when the polypeptide chains are folded, the dimeric protein species comprising the pair of polypeptide chains has the appropriate three-dimensional structure, including the appropriate intra-or inter-chain disulfide bonds such that the protein is capable of acting as a morphogen as defined herein. Specifically, the morphogens generally are capable of all of the following biological functions in a morphogenically permissive environment: stimulating proliferation of progenitor cells; stimulating the differentiation of progenitor cells; stimulating the proliferation of differentiated cells; and supporting the growth and maintenance of differentiated cells, including the "redifferentiation" of transformed cells. In addition, it is also anticipated that these morphogens are capable of inducing redifferentiation of committed cells under appropriate environmental conditions.

The morphogens describes herein may comprise one of two species of generic amino acid sequences: Generic Sequence 1 (Seq. ID No. 1) or Generic Sequence 2 (Seq. ID No. 2); where each Xaa indicates one of the 20 naturally-occurring L-isomer, α-amino acids or a derivative thereof. Generic Sequence 1 comprises the conserved six cysteine skeleton and Generic Sequence 2 comprises the conserved six cysteine skeleton plus the additional cysteine identified in OP-2 (see residue 36 Seq. ID No. 2). These sequences may further comprise the following additional sequence at their N-terminus:

Amino acid sequences within the foregoing generic sequences include: Generic Sequence 3 (Seq. ID No. 3), Generic Sequence 4 (Seq. ID No. 4), Generic Sequence 5 (Seq. ID No. 30) and Generic Sequence 6 (Seq. ID No. 31), listed below. These Generic Sequences accommodate the homologies shared among the various preferred members of this morphogen family identified in Table II, as well as the amino acid sequence variation among them. Specifically, Generic Sequences 3 and 4 are composite amino acid sequences of the following proteins presented in Table II and identified in Seq. ID Nos. 5-14: human OP-1 (hOP-1, Seq. ID Nos. 5 and 16-17), mouse OP-1 (mOP-1, Seq. ID Nos. 6 and 18-19), human and mouse OP-2 (Seq. ID Nos. 7, 8, and 20-22), CBMP2A (Seq. ID No. 9), CBMP2B (Seq. ID No. 10), DPP (from Drosophila, Seq. ID No. 11), Vgl, (from Xenopus, Seq. ID No. 12), Vgr-1 (from mouse, Seq. ID No. 13), and GDF-1 (from mouse, Seq. ID No. 14.) The generic sequences include both the amino acid identity shared by the sequences in Table II, as well as alternative residues for the variable positions within the sequence. Note that these generic sequences allow for an additional cysteine at position 41 or 46 in Generic Sequences 3 or 4, respectively, providing an appropriate cysteine skeleton where inter- or intramolecular disulfide bonds can form, and contain certain critical amino acids which influence the tertiary structure of the proteins. wherein each Xaa is independently selected from a group of one or more specified amino acids defined as follows: "Res." means "residue" and Xaa at res.4 = (Ser, Asp or Glu); Xaa at res.6 = (Arg, Gln, Ser or Lys); Xaa at res.7 = (Asp or Glu); Xaa at res.8 = (Leu or Val); Xaa at res.11 = (Gln, Leu, Asp, His or Asn); Xaa at res.12 = (Asp, Arg or Asn); Xaa at res.14 = (Ile or Val); Xaa at res.15 = (lie or Val); Xaa at res.18 = (Glu, Gln, Leu, Lys, Pro or Arg); Xaa at res.20 = (Tyr or Phe); Xaa at res.21 = (Ala, Ser, Asp, Met, His, Leu or Gln); Xaa at res.23 = (Tyr, Asn or Phe); Xaa at res.26 = (Glu, His, Tyr, Asp or Gln); Xaa at res.28 = (Glu, Lys, Asp or Gln); Xaa at res.30 = (Ala, Ser, Pro or Gln); Xaa at res.31 = (Phe, Leu or Tyr); Xaa at res.33 = (Leu or Val); Xaa at res.34 = (Asn, Asp, Ala or Thr); Xaa at res.35 = (Ser, Asp, Glu, Leu or Ala); Xaa at res.36 = (Tyr, Cys, His, Ser or lle); Xaa at res.37 = (Met, Phe, Gly or Leu); Xaa at res.38 = (Asn or Ser); Xaa at res.39 = (Ala, Ser or Gly); Xaa at res.40 = (Thr, Leu or Ser); Xaa at res.44 = (lle or Val); Xaa at res.45 = (Val or Leu); Xaa at res.46 = (Gln or Arg); Xaa at res.47 = (Thr, Ala or Ser); Xaa at res.49 = (Val or Met); Xaa at res.50 = (His or Asn); Xaa at res.51 = (Phe, Leu, Asn, Ser, Ala or Val); Xaa at res.52 = (lle, Met, Asn, Ala or Val); Xaa at res.53 = (Asn, Lys, Ala or Glu); Xaa at res.54 = (Pro or Ser); Xaa at res.55 = (Glu, Asp, Asn, or Gly); Xaa at res.56 = (Thr, Ala, Val, Lys, Asp, Tyr, Ser or Ala); Xaa at res.57 = (Val, Ala or lle); Xaa at res.58 = (Pro or Asp); Xaa at res.59 = (Lys or Leu); Xaa at res.60 = (Pro or Ala); Xaa at res.63 = (Ala or Val); Xaa at res.65 = (Thr or Ala); Xaa at res.66 = (Gln, Lys, Arg or Glu); Xaa at res.67 = (Leu, Met or Val); Xaa at res.68 = (Asn, Ser or Asp); Xaa at res.69 = (Ala, Pro or Ser); Xaa at res.70 = (lle, Thr or Val); Xaa at res.71 = (Ser or Ala); Xaa at res.72 = (Val or Met); Xaa at res.74 = (Tyr or Phe); Xaa at res.75 = (Phe, Tyr or Leu); Xaa at res.76 = (Asp or Asn); Xaa at res.77 = (Asp, Glu, Asn or Ser); Xaa at res.78 = (Ser, Gln, Asn or Tyr); Xaa at res.79 = (Ser, Asn, Asp or Glu); Xaa at res.80 = (Asn, Thr or Lys); Xaa at res.82 = (lle or Val); Xaa at res.84 = (Lys or Arg); Xaa at res.85 = (Lys, Asn, Gln or His); Xaa at res.86 = (Tyr or His); Xaa at res.87 = (Arg, Gln or Glu); Xaa at res.88 = (Asn, Glu or Asp); Xaa at res.90 = (Val, Thr or Ala); Xaa at res.92 = (Arg, Lys, Val, Asp or Glu); Xaa at res.93 = (Ala, Gly or Glu); and Xaa at res.97 = (His or Arg); wherein each Xaa is independently selected from a group of one or more specified amino acids as defined by the following: "Res." means "residue" and Xaa at res.2 = (Lys or Arg); Xaa at res.3 = (Lys or Arg); Xaa at res.4 = (His or Arg); Xaa at res.5 = (Glu, Ser, His, Gly, Arg or Pro); Xaa at res.9 = (Ser, Asp or Glu); Xaa at res.11 = (Arg, Gln, Ser or Lys); Xaa at res.12 = (Asp or Glu); Xaa at res.13 = (Leu or Val); Xaa at res.16 = (Gln, Leu, Asp, His or Asn); Xaa at res.17 = (Asp, Arg, or Asn); Xaa at res.19 = (lie or Val); Xaa at res.20 = (lie or Val); Xaa at res.23 = (Glu, Gln, Leu, Lys, Pro or Arg); Xaa at res.25 = (Tyr or Phe); Xaa at res.26 = (Ala, Ser, Asp, Met, His, Leu, or Gln); Xaa at res.28 = (Tyr, Asn or Phe); Xaa at res.31 = (Glu, His, Tyr, Asp or Gln); Xaa at res.33 = Glu, Lys, Asp or Gln); Xaa at res.35 = (Ala, Ser or Pro); Xaa at res.36 = (Phe, Leu or Tyr); Xaa at res.38 = (Leu or Val); Xaa at res.39 = (Asn, Asp, Ala or Thr); Xaa at res.40 = (Ser, Asp, Glu, Leu or Ala); Xaa at res.41 = (Tyr, Cys, His, Ser or lle); Xaa at res.42 = (Met, Phe, Gly or Leu); Xaa at res.44 = (Ala, Ser or Gly); Xaa at res.45 = (Thr, Leu or Ser); Xaa at res.49 = (lie or Val); Xaa at res.50 = (Val or Leu); Xaa at res.51 = (Gln or Arg); Xaa at res.52 = (Thr, Ala or Ser); Xaa at res.54 = (Val or Met); Xaa at res.55 = (His or Asn); Xaa at res.56 = (Phe, Leu, Asn, Ser, Ala or Val); Xaa at res.57 = (lle, Met, Asn, Ala or Val); Xaa at res.58 = (Asn, Lys, Ala or Glu); Xaa at res.59 = (Pro or Ser); Xaa at res.60 = (Glu, Asp, or Gly); Xaa at res.61 = (Thr, Ala, Val, Lys, Asp, Tyr, Ser or Ala); Xaa at res.62 = (Val, Ala or lle); Xaa at res.63 = (Pro or Asp); Xaa at res.64 = (Lys or Leu); Xaa at res.65 = (Pro or Ala); Xaa at res.68 = (Ala or Val); Xaa at res.70 = (Thr or Ala); Xaa at res.71 = (Gln, Lys, Arg or Glu); Xaa at res.72 = (Leu, Met or Val); Xaa at res.73 = (Asn, Ser or Asp); Xaa at res.74 = (Ala, Pro or Ser); Xaa at res.75 = (Ile, Thr or Val); Xaa at res.76 = (Ser or Ala); Xaa at res.77 = (Val or Met); Xaa at res.79 = (Tyr or Phe); Xaa at res.80 = (Phe, Tyr or Leu); Xaa at res.81 = (Asp or Asn); Xaa at res.82 = (Asp, Glu, Asn or Ser); Xaa at res.83 = (Ser, Gln, Asn or Tyr); Xaa at res.84 = (Ser, Asn, Asp or Glu); Xaa at res.85 = (Asn, Thr or Lys); Xaa at res.87 = (lie or Val); Xaa at res.89 = (Lys or Arg); Xaa at res.90 = (Lys, Asn, Gln or His); Xaa at res.91 = (Tyr or His); Xaa at res.92 = (Arg, Gln or Glu); Xaa at res.93 = (Asn, Glu or Asp); Xaa at res.95 = (Val, Thr or Ala); Xaa at res.97 = (Arg, Lys, Val, Asp or Glu); Xaa at res.98 = (Ala, Gly or Glu); and Xaa at res.102 = (His or Arg).

Similarly, Generic Sequence 5 (Seq. ID No. 30) and Generic Sequence 6 (Seq. ID No. 31) accommodate the homologies shared among all the morphogen protein family members identified in Table II. Specifically, Generic Sequences 5 and 6 are composite amino acid sequences of human OP-1 (hOP-1, Seq. ID Nos. 5 and 16-17), mouse OP-1 (mOP-1, Seq. ID Nos. 6 and 18-19), human and mouse OP-2 (Seq. ID Nos. 7, 8, and 20-22), CBMP2A (Seq. ID No. 9), CBMP2B (Seq. ID No. 10), DPP (from Drosophila, Seq. ID No. 11), Vgl, (from Xenopus, Seq. ID No. 12), Vgr-1 (from mouse, Seq. ID No. 13), and GDF-1 (from mouse, Seq. ID No. 14), human BMP3 (Seq. ID No. 26), human BMP5 (Seq. ID No. 27), human BMP6 (Seq. ID No. 28) and 60(A) (from Drosophila, Seq. ID Nos. 24-25). The generic sequences include both the amino acid identity shared by these sequences in the C-terminal domain, defined by the six and seven cysteine skeletons (Generic Sequences 5 and 6, respectively), as well as alternative residues for the variable positions within the sequence. As for Generic Sequences 3 and 4, Generic Sequences 5 and 6 allow for an additional cysteine at position 41 (Generic Sequence 5) or position 46 (Generic Sequence 6), providing an appropriate cysteine skeleton where inter- or intramolecular disulfide bonds can form, and containing certain critical amino acids which influence the tertiary structure of the proteins. wherein each Xaa is independently selected from a group of one or more specified amino acids defined as follows: "Res." means "residue" and Xaa at res.2 = (Tyr or Lys); Xaa at res.3 = Val or lle); Xaa at res.4 = (Ser, Asp or Glu); Xaa at res.6 = (Arg, Gln, Ser, Lys or Ala); Xaa at res.7 = (Asp, Glu or Lys); Xaa at res.8 = (Leu, Val or lle); Xaa at res.11 = (Gln, Leu, Asp, His, Asn or Ser); Xaa at res.12 = (Asp, Arg, Asn or Glu); Xaa at res.14 = (lie or Val); Xaa at res.15 = (lie or Val); Xaa at res.16 (Ala or Ser); Xaa at res.18 = (Glu, Gln, Leu, Lys, Pro or Arg); Xaa at res.19 = (Gly or Ser); Xaa at res.20 = (Tyr or Phe); Xaa at res.21 = (Ala, Ser, Asp, Met, His, Gln, Leu or Gly); Xaa at res.23 = (Tyr, Asn or Phe); Xaa at res.26 = (Glu, His, Tyr, Asp, Gln or Ser); Xaa at res.28 = (Glu, Lys, Asp, Gln or Ala); Xaa at res.30 = (Ala, Ser, Pro, Gln or Asn); Xaa at res.31 = (Phe, Leu or Tyr); Xaa at res.33 = (Leu, Val or Met); Xaa at res.34 = (Asn, Asp, Ala, Thr or Pro); Xaa at res.35 = (Ser, Asp, Glu, Leu, Ala or Lys); Xaa at res.36 = (Tyr, Cys, His, Ser or lle); Xaa at res.37 = (Met, Phe, Gly or Leu); Xaa at res.38 = (Asn, Ser or Lys); Xaa at res.39 = (Ala, Ser, Gly or Pro); Xaa at res.40 = (Thr, Leu or Ser); Xaa at res.44 = (lle, Val or Thr); Xaa at res.45 = (Val, Leu or Ile); Xaa at res.46 = (Gin or Arg); Xaa at res.47 = (Thr, Ala or Ser); Xaa at res.48 = (Leu or lle); Xaa at res.49 = (Val or Met); Xaa at res.50 = (His, Asn or Arg); Xaa at res.51 = (Phe, Leu, Asn, Ser, Ala or Val); Xaa at res.52 = (Ile, Met, Asn, Ala, Val or Leu); Xaa at res.53 = (Asn, Lys, Ala, Glu, Gly or Phe); Xaa at res.54 = (Pro, Ser or Val); Xaa at res.55 = (Glu, Asp, Asn, Gly, Val or Lys); Xaa at res.56 = (Thr, Ala, Val, Lys, Asp, Tyr, Ser, Ala, Pro or His); Xaa at res.57 = (Val, Ala or lle); Xaa at res.58 = (Pro or Asp); Xaa at res.59 = (Lys, Leu or Glu); Xaa at res.60 = (Pro or Ala); Xaa at res.63 = (Ala or Val); Xaa at res.65 = (Thr, Ala or Glu); Xaa at res.66 = (Gln, Lys, Arg or Glu); Xaa at res.67 = (Leu, Met or Val); Xaa at res.68 = (Asn, Ser, Asp or Gly); Xaa at res.69 = (Ala, Pro or Ser); Xaa at res.70 = (Ile, Thr, Val or Leu); Xaa at res.71 = (Ser, Ala or Pro); Xaa at res.72 = (Val, Met or Ile); Xaa at res.74 = (Tyr or Phe); Xaa at res.75 = (Phe, Tyr, Leu or His); Xaa at res.76 = (Asp, Asn or Leu); Xaa at res.77 = (Asp, Glu, Asn or Ser); Xaa at res.78 = (Ser, Gln, Asn, Tyr or Asp); Xaa at res.79 = (Ser, Asn, Asp, Glu or Lys); Xaa at res.80 = (Asn, Thr or Lys); Xaa at res.82 = (lle, Val or Asn); Xaa at res.84 = (Lys or Arg); Xaa at res.85 = (Lys, Asn, Gln, His or Val); Xaa at res.86 = (Tyr or His); Xaa at res.87 = (Arg, Gln, Glu or Pro); Xaa at res.88 = (Asn, Glu or Asp); Xaa at res.90 = (Val, Thr, Ala or lle); Xaa at res.92 = (Arg, Lys, Val, Asp or Glu); Xaa at res.93 = (Ala, Gly, Glu or Ser); Xaa at res.95 = (Gly or Ala) and Xaa at res.97 = (His or Arg). wherein each Xaa is independently selected from a group of one or more specified amino acids as defined by the following: "Res." means "residue" and Xaa at res.2 = (Lys, Arg, Ala or Gln); Xaa at res.3 = (Lys, Arg or Met); Xaa at res.4 = (His, Arg or Gln); Xaa at res.5 = (Glu, Ser, His, Gly, Arg, Pro, Thr, or Tyr); Xaa at res.7 = (Tyr or Lys); Xaa at res.8 = (Val or lle); Xaa at res.9 = (Ser, Asp or Glu); Xaa at res.11 = (Arg, Gln, Ser, Lys or Ala); Xaa at res.12 = (Asp, Glu, or Lys); Xaa at res.13 = (Leu, Val or Ile); Xaa at res.16 = (Gln, Leu, Asp, His, Asn or Ser); Xaa at res.17 = (Asp, Arg, Asn or Glu); Xaa at res.19 = (lle or Val); Xaa at res.20 = (Ile or Val); Xaa at res.21 = (Ala or Ser); Xaa at res.23 = (Glu, Gln, Leu, Lys, Pro or Arg); Xaa at res.24 = (Gly or Ser); Xaa at res.25 = (Tyr or Phe); Xaa at res.26 = (Ala, Ser, Asp, Met, His, Gln, Leu, or Gly); Xaa at res.28 = (Tyr, Asn or Phe); Xaa at res.31 = (Glu, His, Tyr, Asp, Gln or Ser); Xaa at res.33 = Glu, Lys, Asp, Gln or Ala); Xaa at res.35 = (Ala, Ser, Pro, Gln or Asn); Xaa at res.36 = (Phe, Leu or Tyr); Xaa at res.38 = (Leu, Val or Met); Xaa at res.39 = (Asn, Asp, Ala, Thr or Pro); Xaa at res.40 = (Ser, Asp, Glu, Leu, Ala or Lys); Xaa at res.41 = (Tyr, Cys, His, Ser or lle); Xaa at res.42 = (Met, Phe, Gly or Leu); Xaa at res.43 = (Asn, Ser or Lys); Xaa at res.44 = (Ala, Ser, Gly or Pro); Xaa at res.45 = (Thr, Leu or Ser); Xaa at res.49 = (lle, Val or Thr); Xaa at res.50 = (Val, Leu or lle); Xaa at res.51 = (Gin or Arg); Xaa at res.52 = (Thr, Ala or Ser); Xaa at res.53 = (Leu or lle); Xaa at res.54 = (Val or Met); Xaa at res.55 = (His, Asn or Arg); Xaa at res.56 = (Phe, Leu, Asn, Ser, Ala or Val); Xaa at res.57 = (Ile, Met, Asn, Ala, Val or Leu); Xaa at res.58 = (Asn, Lys, Ala, Glu, Gly or Phe); Xaa at res.59 = (Pro, Ser or Val); Xaa at res.60 = (Glu, Asp, Gly, Val or Lys); Xaa at res.61 = (Thr, Ala, Val, Lys, Asp, Tyr, Ser, Ala, Pro or His); Xaa at res.62 = (Val, Ala or lle); Xaa at res.63 = (Pro or Asp); Xaa at res.64 = (Lys, Leu or Glu); Xaa at res.65 = (Pro or Ala); Xaa at res.68 = (Ala or Val); Xaa at res.70 = (Thr, Ala or Glu); Xaa at res.71 = (Gln, Lys, Arg or Glu); Xaa at res.72 = (Leu, Met or Val); Xaa at res.73 = (Asn, Ser, Asp or Gly); Xaa at res.74 = (Ala, Pro or Ser); Xaa at res.75 = (Ile, Thr, Val or Leu); Xaa at res.76 = (Ser, Ala or Pro); Xaa at res.77 = (Val, Met or lle); Xaa at res.79 = (Tyr or Phe); Xaa at res.80 = (Phe, Tyr, Leu or His); Xaa at res.81 = (Asp, Asn or Leu); Xaa at res.82 = (Asp, Glu, Asn or Ser); Xaa at res.83 = (Ser, Gln, Asn, Tyr or Asp); Xaa at res.84 = (Ser, Asn, Asp, Glu or Lys); Xaa at res.85 = (Asn, Thr or Lys); Xaa at res.87 = (lle, Val or Asn); Xaa at res.89 = (Lys or Arg); Xaa at res.90 = (Lys, Asn, Gln, His or Val); Xaa at res.91 = (Tyr or His); Xaa at res.92 = (Arg, Gin, Glu or Pro); Xaa at res.93 = (Asn, Glu or Asp); Xaa at res.95 = (Val, Thr, Ala or lle); Xaa at res.97 = (Arg, Lys, Val, Asp or Glu); Xaa at res.98 = (Ala, Gly, Glu or Ser); Xaa at res.100 = (Gly or Ala); and Xaa at res.102 = (His or Arg).

Particularly useful sequences for use as morphogens in this invention include the C-terminal domains, e.g., the C-terminal 96-102 amino acid residues of Vgl, Vgr-1, DPP, OP-1, OP-2, CBMP-2A, CBMP-2B, GDF-1 (see Table II, below, and Seq. ID Nos. 5-14), as well as proteins comprising the C-terminal domains of 60A, BMP3, BMP5 and BMP6 (see Seq. ID Nos. 24-28), all of which include at least the conserved six or seven cysteine skeleton. In addition, biosynthetic constructs designed from the generic sequences, such as COP-1, 3-5, 7, 16, disclosed in U.S. Pat. No. 5,011,691, also are useful. In another preferred aspect of the invention, useful morphogens include active proteins comprising species of polypeptide chains having the generic amino acid sequence herein referred to as "OPX", which accommodates the homologies between the various identified species of OP1 and OP2 (Seq. ID No. 29).

The morphogens described herein include proteins comprising any of the polypeptide chains described above, whether isolated from naturally-occurring sources, or produced by recombinant DNA or other synthetic techniques, and includes allelic and species variants of these proteins, naturally-occurring or biosynthetic mutants thereof, as well as various truncated and fusion constructs. Deletion or addition mutants also are envisioned to be active, including those which may alter the conserved C-terminal cysteine skeleton, provided that the alteration does not functionally disrupt the relationship of these cysteines in the folded structure. Accordingly, such active forms are considered the equivalent of the specifically described constructs disclosed herein.

The proteins may include forms having varying glycosylation patterns, varying N-termini, a family of related proteins having regions of amino acid sequence homology, and active truncated or mutated forms of native or biosynthetic proteins, produced by expression of recombinant DNA in host cells.

The morphogenic proteins can be expressed from intact or truncated cDNA or from synthetic DNAs in procaryotic or eucaryotic host cells, and purified, cleaved, refolded, and dimerized to form morphogenically active compositions. Currently preferred host cells include E. coli or mammalian cells, such as CHO, COS or BSC cells.

Thus, in view of this disclosure, skilled genetic engineers can isolate genes from cDNA or genomic libraries of various different species which encode appropriate amino acid sequences, or construct DNAs from oligonucleotides, and then can express them in various types of host cells, including both procaryotes and eucaryotes, to produce large quantities of active proteins capable of protecting tissues and organs from immune cell-mediated tissue destruction, including substantially inhibiting such damage and/or regenerating the damaged tissue in a variety of mammals, including humans.

The foregoing and other objects, features and advantages of the present invention will be made more apparent from the following detailed description of the invention.

### Brief Description of the Drawings

- FIG 1: shows the cardioprotective effects of morphogen (hOP1) in a rat myocardial ischemia-reperfusion model, as evidenced by the smaller loss of myocardial creatine kinase in hOP1-treated rats;
- FIG 2: shows the effects of 20 µg of morphogen (hOP1 given 24 hours prior to isolation of rat heart on endothelial-dependent vasorelaxation to acetycholine following induced ischemia-reperfusion injury;
- FIG 3: shows the effect of morphogen (hOP1) on neutrophil adherence to LTB₄-stimulated mesenteric artery endothelium in neutrophil-activated rats;
- FIG 4: (A and B) are schematic representations of morphogen inhibition of early mononuclear phagocytic multinu-clearization in vivo;
- FIG 5: graphs the effect of a morphogen (e.g., OP-1) and a placebo control on mucositic lesion formation; and
- FIG 6: (A-D) graphs the effects of a morphogen (eg., OP-1, Figs. 6A and 6C) and TGF-β (Fig. 6B and 6D) on collagen (6A and 6B) and hyaluronic acid (6C and 6D) production in primary fibroblast cultures.

### Detailed Description of the Invention

It now has been surprisingly discovered that the morphogens defined herein are effective agents in alleviating the tissue destructive effects associated with the body's inflammatory response to tissue injury. In particular, as disclosed herein, the morphogens are capable of alleviating the necrotic tissue effects associated with the ensuing inflammatory responses that occur following an initial tissue injury.

When tissue injury occurs, whether caused by bacteria, trauma, chemicals, heat, or any other phenomenon, the body's inflammatory response is stimulated. In response to signals released from the damaged cells (e.g., cytokines), extravascularization of immune effector cells is induced. Under ordinary circumstances these invading immune effector cells kill the infectious agent and/or infected or damaged cells (through the release of killing substances such as superoxides, performs, and other antimicrobial agents stored in granules), remove the dead tissues and organisms (through phagocytosis), release various biological response modifiers that promote rapid healing and covering of the wound (quite often resulting in the formation of fibrotic scar tissue), and then, after the area is successfully healed, exit from the site of the initial insult Once the site is perceived to be normal, the local release of inflammatory cytokines ceases and the display of adhesion molecules on the vessel endothelium returns to basal levels. In some cases, however, the zeal of these interacting signals and cellular systems, which are designed to capture and contain very rapidly multiplying infectious agents, act to the detriment of the body, killing additional, otherwise healthy, surrounding tissue. This additional unnecessary tissue death further compromises organ function and sometimes results in death of the individual. In addition, the resulting scar tissue that often forms can interfere with normal tissue function as occurs, for example, in idiopathic pulmonary fibrosis, IBD and organ cirrhosis.

The vascular endothelium constitutes the first barrier between circulating immune effector cells and extravascular tissues. Extravasation of these circulating cells requires that they bind to the vascular endothelial cells, cross the basement membrane, and enter insulted tissues e.g, by phagocytosis or protease-mediated extracellular matrix degradation. Without being limited to a particular theory, it is believed that the morphogens of this invention may modulate the inflammatory response in part by modulating the attachment of immune effector cells to the luminal side of the endothelium of blood vessels at or near sites of tissue damage and/or inflammatory lesions. Because the method reduces or prevents the attachment of immune effector cells at these sites, it also prevents the subsequent release of tissue destructive agents by these same immune effector cells at sites of tissue damage and/or inflammatory lesions. Because attachment of immune effector cells to the endothelium must precede their extravascularization, the method also prevents the initial or continued entry of these cells into extravascular sites of tissue destruction or ongoing inflammatory lesions. Therefore, the invention not only finds utility in a method to reduce or prevent the immune cell-mediated cellular destruction at extravascular sites of recent tissue destruction, but also finds utility in a method to prevent or reduce the continued entry of immune effector cells into extravascular sites of ongoing inflammatoly cascades. As will be appreciated by those skilled in the art, the morphogens of this invention also may be contemplated in mechanisms for disrupting the functional interaction of immune effector cells with endothelium where the adhesion molecules are induced by means other than in response to tissue injury.

One source of tissue injury is induced by cell exposure to toxic oxygen concentrations, such as ischemic-reperfusion tissue injury (oxygen deprivation), and following hyperoxia injury (lethally high oxygen concentrations). Accordingly, the present invention finds utility in a method for alleviating the tissue damage induced by ischemic-reperfusion injury or hyperoxia-induced injury comprising the step of administering to the afflicted individual a therapeutic amount of a morphogen prior to, during, or after damage to the affected tissue. Where the toxic oxygen concentrations may be deliberately induced, as by a surgical or clinical procedure, the morphogen preferably is administered prior to induction.

In addition, the morphogens described herein, in contrast to fibrogenic growth factors such as TGF-β, stimulate tissue morphogenesis and do not stimulate fibrosis or scar tissue formation (see Example 9, below.) Accordingly, in addition to inhibiting the tissue destructive effects associated with the inflammatory response, the morphogens further enhance the viability of damaged tissue and/or organs by stimulating the regeneration of the damaged tissue and preventing fibrogenesis.

The morphogens described herein also can inhibit epithelial cell proliferation (see Example 10, below.) This activity of the morphogens also may be particularly useful in the treatment of psoriasis and other inflammatory diseases that involve epithelial cell populations.

Provided below are detailed descriptions of suitable morphogens useful in the invention, as well as methods for their administration and application, and numerous, nonlimiting examples which 1) illustrate the suitability of the morphogens described herein as therapeutic agents for protecting tissue from the tissue destructive effects associated with the body's inflammatory response; and 2) provide assays with which to test candidate morphogens for their efficacy. I. Useful Morphogens

As defined herein a protein is morphogenic if it is capable of inducing the developmental cascade of cellular and molecular events that culminate in the formation of new, organ-specific tissue and comprises at least the conserved C-terminal six cysteine skeleton or its functional equivalent (see supra). Specifically, the morphogens generally are capable of all of the following biological functions in a morphogenically permissive environment: stimulating proliferation of progenitor cells; stimulating the differentiation of progenitor cells; stimulating the proliferation of differentiated cells; and supporting the growth and maintenance of differentiated cells, including the "redifferentiation" of transformed cells. Details of how the morphogens useful in the invention first were identified, as well as a description on how to make, use and test them for morphogenic activity are disclosed in USSN 667,274, filed March 11, 1991 and USSN 752,764, filed August 30, 1991. As disclosed therein, the morphogens may be purified from naturally-sourced material or recombinantly produced from procaryotic or eucaryotic host cells, using the genetic sequences disclosed therein. Alternatively, novel morphogenic sequences may be identified following the procedures disclosed therein.

Particularly useful proteins include those which comprise the naturally derived sequences disclosed in Table II. Other useful sequences include biosynthetic constructs such as those disclosed in U.S. Pat. 5,011,691 (e.g., COP-1, COP-3, COP-4, COP-5, COP-7, and COP-16).

The morphogens useful in the invention also can be described by any of the 6 generic sequences described herein (Generic Sequences 1, 2, 3, 4, 5 and 6). Generic sequences 1 and 2 also may include, at their N-terminus, the sequence

Table II, set forth below, compares the amino acid sequences of the active regions of native proteins that have been identified as morphogens, including human OP-1 (hOP-1, Seq. ID Nos. 5 and 16-17), mouse OP-1 (mOP-1, Seq. ID Nos. 6 and 18-19), human and mouse OP-2 (Seq. ID Nos. 7, 8, and 20-23), CBMP2A (Seq. ID No. 9), CBMP2B (Seq. ID No. 10), BMP3 (Seq. ID No. 26), DPP (from Drosophila, Seq. ID No. 11), Vgl, (from Xenopus, Seq. ID No. 12), Vgr-1 (from mouse, Seq. ID No. 13), GDF-1 (from mouse, Seq. ID Nos. 14, 32 and 33), 60A protein (from Drosophila, Seq. ID Nos. 24 and 25), BMP5 (Seq. ID No. 27) and BMP6 (Seq. ID No. 28). The sequences are aligned essentially following the method of Needleman et al. (1970) J. Mol. Biol., 48:443-453, calculated using the Align Program (DNAstar, Inc.) In the table, three dots indicates that the amino acid in that position is the same as the amino acid in hOP-1. Three dashes indicates that no amino acid is present in that position, and are included for purposes of illustrating homologies. For example, amino acid residue 60 of CBMP-2A and CBMP-2B is "missing". Of course, both these amino acid sequences in this region comprise Asn-Ser (residues 58, 59), with CBMP-2A then comprising Lys and lle, whereas CBMP-2B comprises Ser and lle.

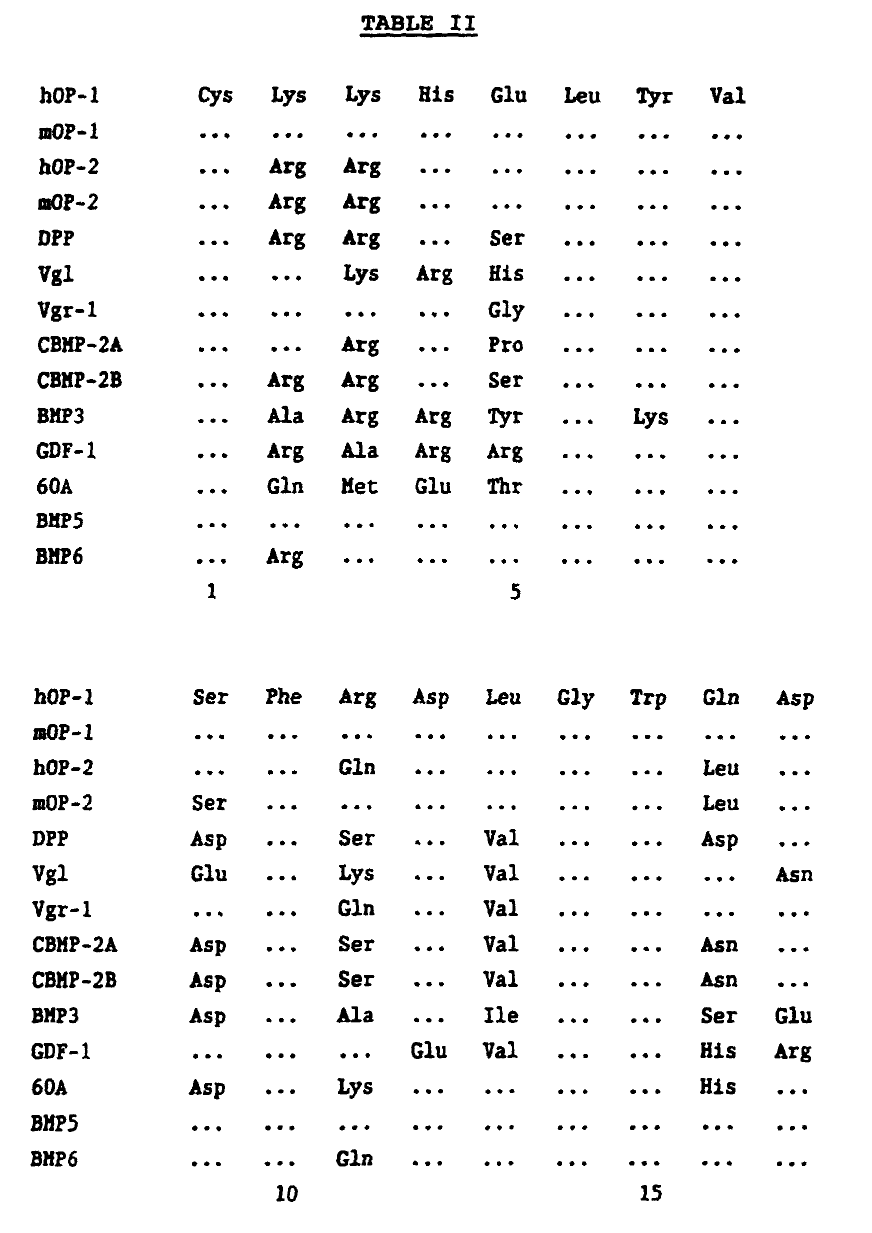

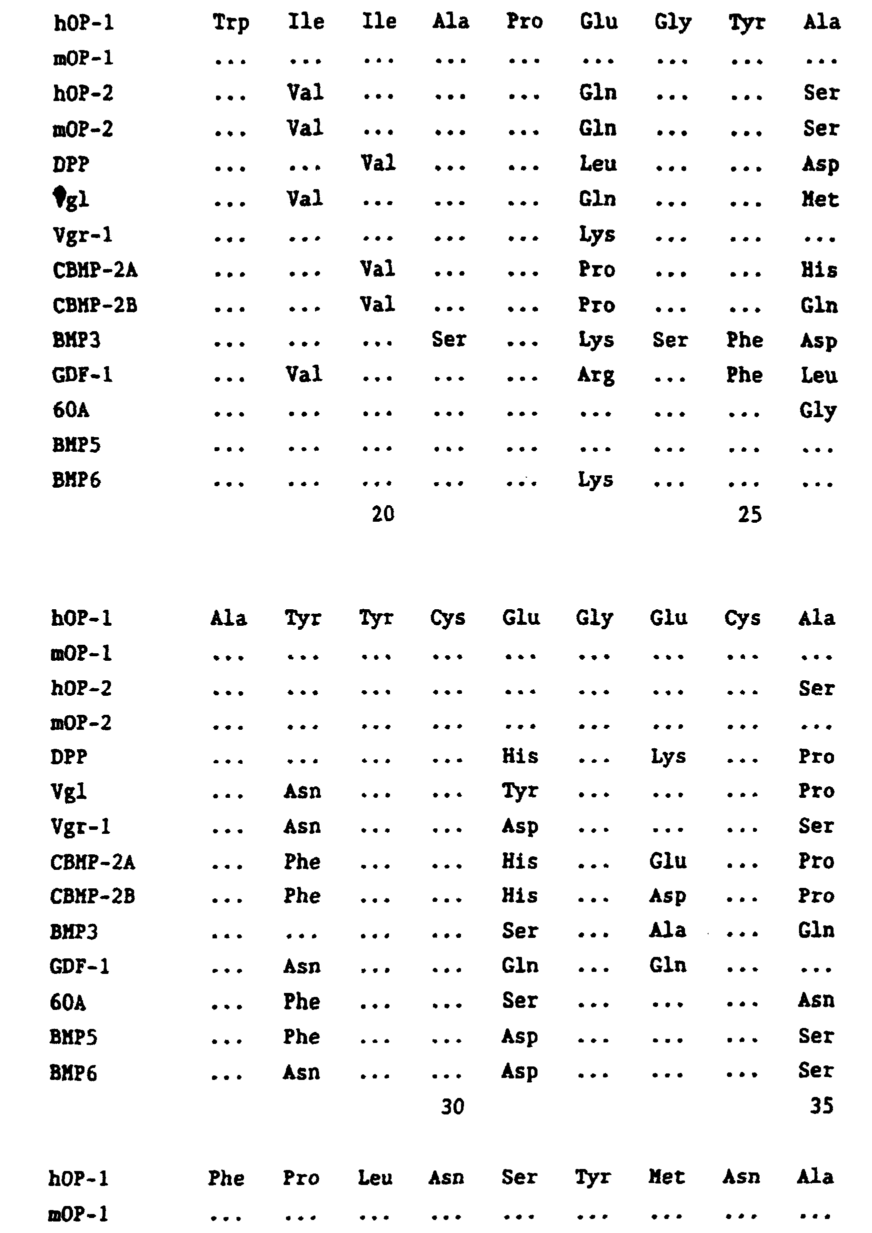

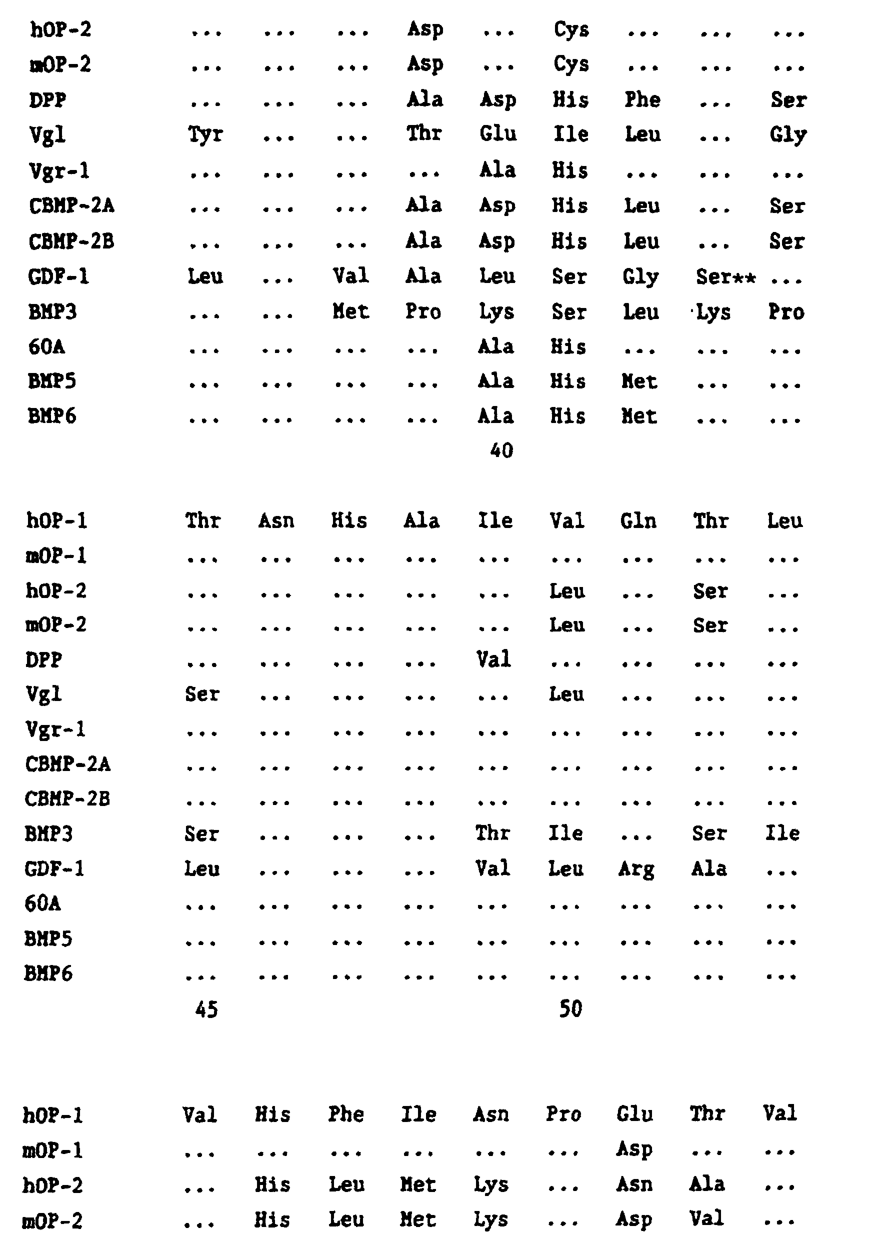

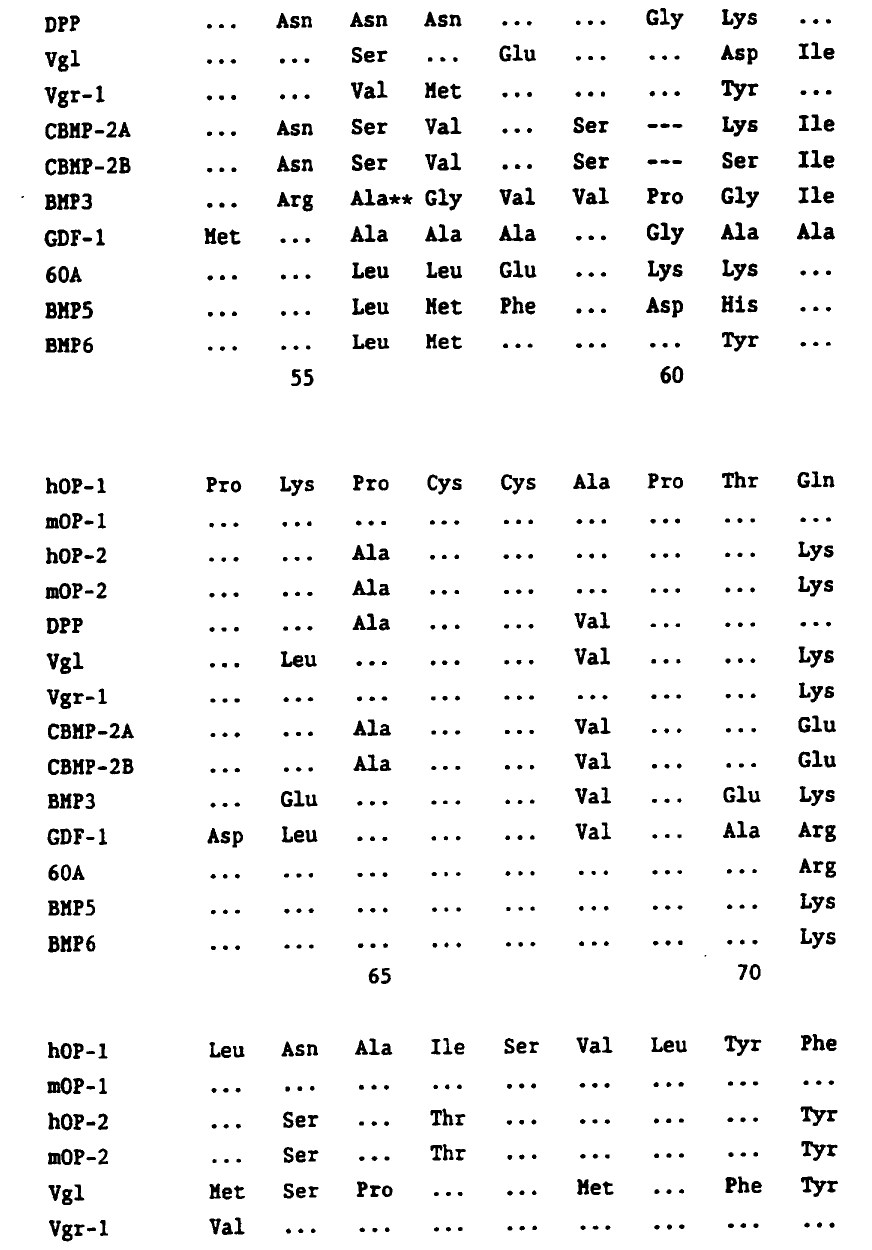

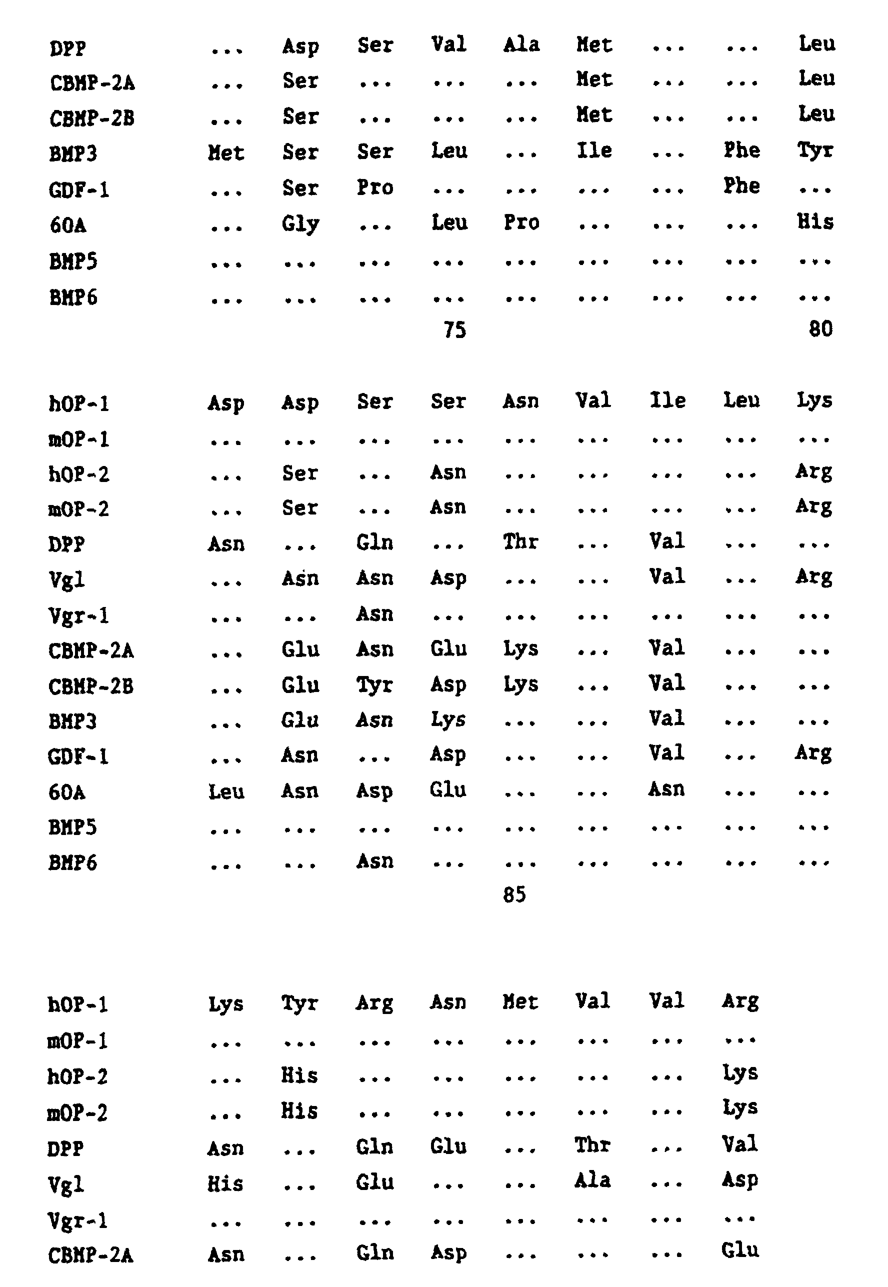

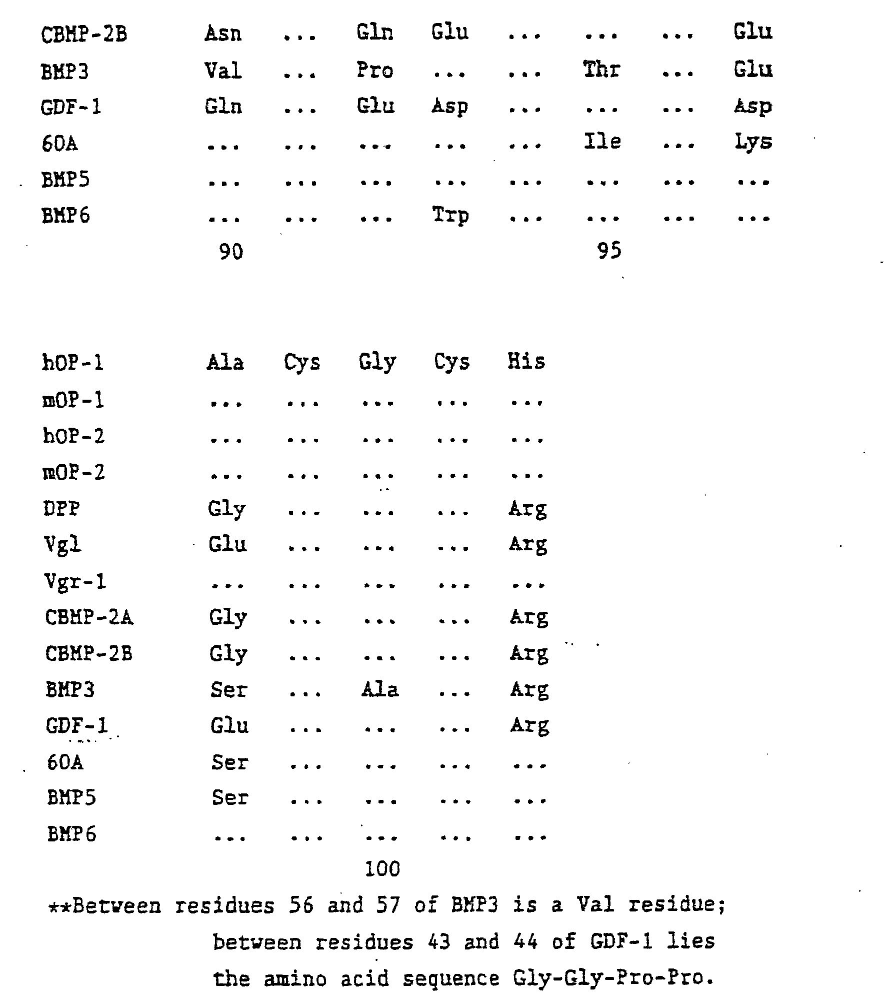

The currently most preferred protein sequences useful as morphogens in this invention include the amino acid sequence defining the conserved six cysteine skeleton of hOP1 (e.g., residues 43-139 of Seq. ID No. 5). In addition, the most preferred sequences include both allelic and species variants of the OP-1 and OP-2 proteins, including the Drosophila 60A protein. Accordingly, in still another preferred aspect, the invention includes morphogens comprising species of polypeptide chains having the generic amino acid sequence referred to herein as "OPX", which defines the seven cysteine skeleton and accommodates the identities between the various identified mouse and human OP1 and OP2 proteins. OPX is presented in Seq. ID No. 29. As described therein, each Xaa at a given position independently is selected from the residues occurring at the corresponding position in the C-terminal sequence of mouse or human OP1 or OP2 (see Seq. ID Nos. 5-8 and/or Seq. ID Nos. 16-23).

### II. Formulations and Methods for Administering Therapeutic Agents

The morphogens may be provided to an individual by any suitable means, preferably directly (e.g., locally, as by injection or topical administration to a tissue locus) or systemically (e.g., parenterally or orally). Where the morphogen is to be provided parenterally, such as by intravenous, subcutaneous, intramuscular, intraorbital, ophthalmic, intraventricular, intracranial, intracapsular, intraspinal, intracisternal, intraperitoneal, buccal, rectal, vaginal, intranasal or by aerosol administration, the morphogen preferably comprises part of an aqueous solution. The solution is physiologically acceptable so that in addition to delivery of the desired morphogen to the patient, the solution does not otherwise adversely affect the patient's electrolyte and volume balance. The aqueous medium for the morphogen thus may comprise normal physiologic saline (9.85% NaCl, 0.15M), pH 7-7.4. The aqueous solution containing the morphogen can be made, for example, by dissolving the protein in 50% ethanol containing acetonitrile in 0.1% trifluoroacetic acid (TFA) or 0.1% HCl, or equivalent solvents. One volume of the resultant solution then is added, for example, to ten volumes of phosphate buffered saline (PBS), which further may include 0.1-0.2% human serum albumin (HSA). The resultant solution preferably is vortexed extensively. If desired, a given morphogen may be made more soluble by association with a suitable molecule. For example, association of the mature dimer with the pro domain of the morphogen keeps the morphogen soluble in physiological buffers. In fact, the endogenous protein is thought to be transported in this form. Another molecule capable of enhancing solubility and particularly useful for oral administrations, is casein. For example, addition of 0.2% casein increases solubility of the mature active form of OP-1 by 80%. Other components found in milk and/or various serum proteins also may be useful.

Useful solutions for parenteral administration may be prepared by any of the methods well known in the pharmaceutical art, described, for example, in Remington's Pharmaceutical Sciences (Gennaro, A., ed.), Mack Pub., 1990. Formulations may include, for example, polyalkylene glycols such as polyethylene glycol, oils of vegetable origin, hydrogenated naphthalenes, and the like. Formulations for direct administration, in particular, may include glycerol and other compositions of high viscosity to help maintain the morphogen at the desired locus. Biocompatible, preferably bioresorbable, polymers, including, for example, hyaluronic acid, collagen, tricalcium phosphate, polybutyrate, lactide and glycolide polymers, and lactide/glycolide copolymers, may be useful excipients to control the release of the morphogen in vivo. Other potentially useful parenteral delivery systems for these morphogens include ethylene-vinyl acetate copolymer particles, osmotic pumps, implantable infusion systems, and liposomes. Formulations for inhalation administration contain as excipients, for example, lactose, or may be aqueous solutions containing, for example, polyoxyethylene-9-lauryl ether, glycocholate and deoxycholate, or oily solutions for administration in the form of nasal drops, or as a gel to be applied intranasally. Formulations for parenteral administration may also include glycocholate for buccal administration, methoxysalicylate for rectal administration, or cutric acid for vaginal administration.

Suppositories for rectal administration also may be prepared by mixing the morphogen or morphogen-stimulating agent with a non-irritating excipient such as cocoa butter or other compositions which are solid at room temperature and liquid at body temperatures.

Formulations for topical administration to the skin surface may be prepared by dispersing the morphogen or morphogen-stimulating agent with a dermally acceptable carrier such as a lotion, cream, ointment or soap. Particularly useful are carriers capable of forming a film or layer over the skin to localize application and inhibit removal. For topical administration to internal tissue surfaces, the morphogen may be dispersed in a liquid tissue adhesive or other substance known to enhance adsorption to a tissue surface. For example, hydroxypropylcellulose or fibrinogen/thrombin solutions may be used to advantage. Alternatively, tissue-coating solutions, such as pectin-containing formulations, may be used.

Alternatively, the morphogens described herein may be administered orally. Oral administration of proteins as therapeutics generally is not practiced as most proteins are readily degraded by digestive enzymes and acids in the mammalian digestive system before they can be absorbed into the bloodstream. However, the morphogens described herein typically are acid stable and protease-resistant (see, for example, U.S. Pat.No. 4,968,590.) In addition, at least one morphogen, OP-1, has been identified in mammary gland extract, colostrum and 57-day milk. Moreover, the OP-1 purified from mammary gland extract is morphogenically active. Specifically, this protein induces endochondral bone formation in mammals when implanted subcutaneously in association with a suitable matrix material, using a standard in vivo bone assay, such as is disclosed in U.S. Pat.No. 4,968,590. Moreover, the morphogen also is detected in the bloodstream. Finally, soluble form morphogen, e.g., mature morphogen associated with the pro domain, is morphogenically active. These findings indicate that oral and parenteral administration are viable means for administering morphogens to an individual. In addition, while the mature forms of certain morphogens described herein typically are sparingly soluble, the morphogen form found in milk (and mammary gland extract and colostrum) is readily soluble, probably by association of the mature, morphogenically active form with part or all of the pro domain of the intact sequence and/or by association with one or more milk components. Accordingly, the compounds provided herein also may be associated with molecules capable of enhancing their solubility in vitro or in vivo.

Where the morphogen or morphogen-stimulating agent comprises part of a tissue or organ preservation solution, any commercially available preservation solution may be used to advantage. For example, useful solutions known in the art include Collins solution, Wisconsin solution, Belzer solution, Eurocollins solution and lactated Ringer's solution. Generally, an organ preservation solution usually possesses one or more of the following properties: (a) an osmotic pressure substantially equal to that of the inside of a mammalian cell,(solutions typically are hyperosmolar and have K+ and/or Mg++ ions present in an amount sufficient to produce an osmotic pressure slightly higher than the inside of a mammalian cell; (b) the solution typically is capable of maintaining substantially normal ATP levels in the cells; and (c) the solution usually allows optimum maintenance of glucose metabolism in the cells. Organ preservation solutions also may contain anticoagulants, energy sources such as glucose, fructose and other sugars, metabolites, heavy metal chelators, glycerol and other materials of high viscosity to enhance survival at low temperatures, free oxygen radical inhibiting agents and a pH indicator. A detailed description of preservation solutions and useful components may be found, for example, in US Patent No. 5,002,965.

The compounds provided herein also may be associated with molecules capable of targeting the morphogen or morphogen-stimulating agent to the desired tissue. For example, an antibody, antibody fragment, or other binding protein that interacts specifically with a surface molecule on cells of the desired tissue, may be used. Useful targeting molecules may be designed, for example, using the single chain binding site technology disclosed, for example, in U.S. Pat. No. 5,091,513.

As described above, the morphogens provided herein share significant sequence homology in the C-terminal active domains. By contrast, the sequences typically diverge significantly in the sequences which define the pro domain. Accordingly, the pro domain is thought to be morphogen-specific. As described above, it is also known that the various morphogens identified to date are differentially expressed in the different tissues. Accordingly, without being limited to any given theory, it is likely that, under natural conditions in the body, selected morphogens typically act on a given tissue. Accordingly, part or all of the pro domains which have been identified associated with the active form of the morphogen in solution, may serve as targeting molecules for the morphogens described herein. For example, the pro domains may interact specifically with one or more molecules at the target tissue to direct the morphogen associated with the pro domain to that tissue. Accordingly, another useful targeting molecule for targeting morphogen to a tissue of interest is part or all of a morphogen pro domain. For example, part or all of the pro domain of GDF-1 may be used to target a morphogen to nerve tissue. Alternatively, part or all of the pro domain of OP-1 or CBMP2 may be used to target a morphogen to bone tissue, both of which proteins are found naturally associated with bone tissue.

The morphogens described herein are useful for providing neuroprotective effects to alleviate neural pathway damage associated with the body's immune/inflammatory response to an initial injury to nerve tissue. As used herein, a "neural pathway" describes a nerve circuit for the passage of electric signals from a source to a target cell site and includes both the central nervous system (CNS) and peripheral nervous system (PNS). The pathway includes the neurons through which the electric impulse is transported, including groups of interconnecting neurons, the nerve fibers formed by bundled neuronal axons, and the glial cells surrounding and associated with the neurons. An inflammatory response to nerve tissue injury may follow trauma to nerve tissue, caused, for example, by an autoimmune (including autoantibody) dysfunction, neoplastic lesion, infection, chemical or mechanical trauma, or other disease. An exemplary nerve-related inflammatory disease is multiple sclerosis. Neural pathway damage also can result from a reduction or interruption, e.g., occlusion, of a neural blood supply, as in an embolic stroke, (e.g, ischemia or hypoxia-induced injury), or by other trauma to the nerve or surrounding material. In addition, at least part of the damage associated with a number of primary brain tumors also appears to be immunologically related. Application of the morphogen directly to the cells to be treated, or providing the morphogen to the mammal systemically, for example, intravenously or indirectly by oral administration, may be used to alleviate and/or inhibit the immunologically related response to a neural injury. Alternatively, administration of an agent capable of stimulating morphogen expression and/or secretion in vivo, preferably at the site of injury, also may be used. Where the injury is to be induced, as during surgery or other aggressive clinical treatment, the morphogen or agent may be provided prior to induction of the injury to provide a neuroprotective effect to the nerve tissue at risk.

Where the morphogen is intended for use as a therapeutic to alleviate tissue damage associated with an immune/inflammatory condition of the CNS, an additional problem must be addressed: overcoming the so-called "blood-brain barrier", the brain capillary wall structure that effectively screens out all but selected categories of molecules present in the blood, preventing their passage into the brain. The blood-brain barrier may be bypassed effectively by direct infusion of the morphogen or morphogen-stimulating agent into the brain. Alternatively, the morphogen or morphogen-stimulating agent may be modified to enhance its transport across the blood-brain barrier. For example, truncated forms of the morphogen or a morphogen-stimulating agent may be most successful. Alternatively, the morphogen or morphogen-stimulating agent may be modified to render it more lipophilic, or it may be conjugated to another molecule which is naturally transported across the barrier, using standard means known to those skilled in the art, as, for example, described in Pardridge, Endocrine Reviews 7:314-330 (1986) and U.S. Pat. No. 4,801,575.

Finally, the morphogens provided herein may be administered alone or in combination with other molecules known to be beneficial in the treatment compositions and methods described herein, including, but not limited to anticoagulants, free oxygen radical inhibiting agents, salicylic acid, vitamin D, and other anti-inflammatory agents. Psoriais treatments also may include ultra-violet light treatment, zinc oxide and retinoids.

The compounds provided herein can be formulated into pharmaceutical compositions by admixture with pharmaceutically acceptable nontoxic excipients and carriers. As noted above, such compositions may be prepared for parenteral administration, particularly in the form of liquid solutions or suspensions; for oral administration, particularly in the form of tablets or capsules; or intranasally, particularly in the form of powders, nasal drops, or aerosols.

The compositions can be formulated for parenteral or oral administration to humans or other mammals in therapeutically effective amounts, e.g., amounts which provide appropriate concentrations for a time sufficient to alleivate the tissue destructive effects associated with the inflammatory response, including protecting tissue in anticipation of tissue damage.

As will be appreciated by those skilled in the art, the concentration of the compounds described in a therapeutic composition will vary depending upon a number of factors, including the dosage of the drug to be administered, the chemical characteristics (e.g., hydrophobicity) of the compounds employed, and the route of administration. The preferred dosage of drug to be administered also is likely to depend on such variables as the type and extent of progression of the tissue damage, the overall health status of the particular patient, the relative biological efficacy of the compound selected, the formulation of the compound excipients, and its route of administration. In general terms, the compounds of this invention may be provided in an aqueous physiological buffer solution containing about 0.001% to 10% w/v compound for parenteral administration. Typical dose ranges are from about 10 ng/kg to about 1 g/kg of body weight per day; a preferred dose range is from about 0.1 µg/kg to 100 mg/kg of body weight per day. Optimally, the morphogen dosage given is between 0.1-100 µg of protein per kilogram weight of the patient. No obvious morphogen induced pathological lesions are induced when mature morphogen (e.g., OP-1,20 µg) is administered daily to normal growing rats for 21 consecutive days. Moreover, 10 µg systemic injections of morphogen (e.g., OP-1) injected daily for 10 days into normal newborn mice does not produce any gross abnormalities.

In administering morphogens systemically, preferably a large volume loading dose is used at the start of the treatment. The treatment then is continued with a maintenance dose. Further administration then can be determined by monitoring at intervals the levels of the morphogen in the blood.

Where tissue injury is induced deliberately as part of, for example, a surgical procedure, the morphogen preferably is provided just prior to, or concomitant with induction of the trauma. Preferably, the morphogen is administered prophylactically in a surgical setting.

Alternatively, an effective amount of an agent capable of stimulating endogenous morphogen levels may be administered by any of the routes described above. For example, an agent capable of stimulating morphogen production and/or secretion from cells of affected tissue and/or transplant tissue may be provided to a mammal, e.g., by direct administration of the agent to the tissue to be treated. A method for identifying and testing agents capable of modulating the levels of endogenous morphogens in a given tissue is described generally herein in Example 15, and in detail in copending USSN 752,859, filed August 30, 1991. Briefly, candidate compounds can be identified and tested by incubating the compound in vitro with a test tissue or cells thereof, for a time sufficient to allow the compound to affect the production, i.e., the expression and/or secretion, of a morphogen produced by the cells of that tissue.

For purposes of the present invention, the above-described morphogens effective in alleviating tissue damage associated with ischemic-reperfusion injury are administered prior to or during the restoration of oxygen (e.g., restoration of blood flow, reperfusion.) Where treatment is to follow an existing injury, the therapeutic agent preferably is administered as an intravenous infusion provided acutely after the hypoxic or ischemic condition occurs. For example, the therapeutic agent can be administered by intravenous infusion immediately after a cerebral infarction, a myocardial infarction, asphyxia, or a cardiopulmonary arrest. Where ischemia or hypoxia injury is deliberately and/or unavoidably induced as part of, for example, a surgical procedure where circulation to an organ or organ system is deliberately and/or transiently interrupted, e.g., in carotid enterectomy, coronary artery bypass, grafting, organ transplanting, fibrinolytic therapy, etc., the therapeutic agent preferably is provided just prior to, or concomitant with, reduction of oxygen to the tissue. Preferably, the morphogen is administered prophylactically in a surgical setting.

Similarly, where hyperoxia-induced injury already has occurred, the morphogen is administered upon diagnosis. Where hyperoxia injury may be induced as, for example, during treatment of prematurely newborn babies, or patients suffering from pulmonary diseases such as emphysema, the therapeutic agent preferably is administered prior to administration of oxygen e.g., prophylactically.

### III. Examples

### Example 1. Identification of Morphogen-Expressing Tissue

Determining the tissue distribution of morphogens may be used to identify different morphogens expressed in a given tissue, as well as to identify new, related morphogens. Tissue distribution also may be used to identify useful morphogen-producing tissue for use in screening and identifying candidate morphogen-stimulating agents. The morphogens (or their mRNA transcripts) readily are identified in different tissues using standard methodologies and minor modifications thereof in tissues where expression may be low. For example, protein distribution may be determined using standard Western blot analysis or immunofluorescent techniques, and antibodies specific to the morphogen or morphogens of interest. Similarly, the distribution of morphogen transcripts may be determined using standard Northern hybridization protocols and transcript-specific probes.

Any probe capable of hybridizing specifically to a transcript, and distinguishing the transcript of interest from other, related transcripts may be used. Because the morphogens described herein share such high sequence homology in their active, C-terminal domains, the tissue distribution of a specific morphogen transcript may best be determined using a probe specific for the pro region of the immature protein and/or the N-terminal region of the mature protein. Another useful sequence is the 3' non-coding region flanking and immediately following the stop codon. These portions of the sequence vary substantially among the morphogens of this invention, and accordingly, are specific for each protein. For example, a particularly useful Vgr-1-specific probe sequence is the Pvull-Sacl fragment, a 265 bp fragment encoding both a portion of the untranslated pro region and the N-terminus of the mature sequence (see Lyons et al. (1989) PNAS 86:4554-4558 for a description of the cDNA sequence). Similarly, particularly useful mOP-1-specific probe sequences are the BstX1-BgII fragment, a 0.68 Kb sequence that covers approximately two-thirds of the mOP-1 pro region; a Stul-Stul fragment, a 0.2 Kb sequence immediately upstream of the 7-cysteine domain; and the Ear1-Pst1 fragment, an 0.3 Kb fragment containing a portion of the 3'untranslated sequence (See Seq. ID No. 18, where the pro region is defined essentially by residues 30-291.) Similar approaches may be used, for example, with hOP-1 (Seq. ID No. 16) or human or mouse OP-2 (Seq. ID Nos. 20 and 22.)

Using these morphogen-specific probes, which may be synthetically engineered or obtained from cloned sequences, morphogen transcripts can be identified in mammalian tissue, using standard methodologies well known to those having ordinary skill in the art. Briefly, total RNA is prepared from various adult murine tissues (e.g., liver, kidney, testis, heart, brain, thymus and stomach) by a standard methodology such as by the method of Chomczyaski et al. ((1987) Anal. Biochem 162:156-159) and described below. Poly (A)+ RNA is prepared by using oligo (dT)-cellulose chromatography (e.g., Type 7, from Pharmacia LKB Biotechnology, Inc.). Poly (A)+ RNA (generally 15 µg) from each tissue is fractionated on a 1% agarose/formaldehyde gel and transferred onto a Nytran membrane (Schleicher & Schuell). Following the transfer, the membrane is baked at 80°C and the RNA is cross-linked under UV light (generally 30 seconds at 1 mW/cm²). Prior to hybridization, the appropriate probe is denatured by heating. The hybridization is carried out in a lucite cylinder rotating in a roller bottle apparatus at approximately 1 rev/min for approximately 15 hours at 37°C using a hybridization mix of 40% formamide, 5 x Denhardts, 5 x SSPE, and 0.1% SDS. Following hybridization, the non-specific counts are washed off the filters in 0.1 x SSPE, 0.1% SDS at 50°C.

Examples demonstrating the tissue distribution of various morphogens, including Vgr-1, OP-1, BMP2, BMP3, BMP4, BMP5, GDF-1, and OP-2 in developing and adult tissue are disclosed in co-pending USSN 752, 764, and in Ozkaynak, et al., (1991) Biochem. Biophys. Res. Commn. 179:116-123, and Ozkaynak, et al. (1992) (JBC, in press). Using the general probing methodology described herein, northern blot hybridizations using probes specific for these morphogens to probe brain, spleen, lung, heart, liver and kidney tissue indicate that kidney-related tissue appears to be the primary expression source for OP-1, with brain, heart and lung tissues being secondary sources. OP-1 mRNA also was identified in salivary glands, specifically rat parotid glands, using this probing methodology. Lung tissue appears to be the primary tissue expression source for Vgr-1, BMP5, BMP4 and BMP3. Lower levels of Vgr-1 also are seen in kidney and heart tissue, while the liver appears to be a secondary expression source for BMP5, and the spleen appears to be a secondary expression source for BMP4. GDF-1 appears to be expressed primarily in brain tissue. To date, OP-2 appears to be expressed primarily in early embryonic tissue. Specifically, northern blots of murine embryos and 6-day post-natal animals shows abundant OP2 expression in 8-day embryos. Expression is reduced significantly in 17-day embryos and is not detected in post-natal animals.

### Example 2. Active Morphogens in Body Fluids

OP-1 expression has been identified in saliva (specifically, the rat parotid gland, see Example 1), human blood serum, and various milk forms, including mammary gland extract, colostrum, and 57-day bovine milk. Moreover, and as described in USSN 923,780, the body fluid-extracted protein is morphogenically active. The discovery that the morphogen naturally is present in milk and saliva, together with the known observation that mature, active OP-1 is acid-stable and protease-resistant, indicate that oral administration is a useful route for therapeutic administration of morphogen to a mammal. Oral administration typically is the preferred mode of delivery for extended or prophylactic therapies. In addition, the identification of morphogen in all milk forms, including colostrum, suggests that the protein may play a significant role in tissue development, including skeletal development, of juveniles.

### 2.1 Morphogen Detection in Milk

OP-1 was partially purified from rat mammary gland extract and bovine colostrum and 57 day milk by passing these fluids over a series of chromatography columns: (e.g., cation-exchange, affinity and reverse phase). At each step the eluant was collected in fractions and these were tested for the presence of OP-1 by standard immunoblot. Immunoreactive fractions then were combined and purified further. The final, partially purified product then was examined for the presence of OP-1 by Western blot analysis using OP-1-specific antisera, and tested for in vivo and in vitro activity.

OP-1 purified from the different milk sources were characterized by Western blotting using antibodies raised against OP-1 and BMP2. Antibodies were prepared using standard immunology protocols well known in the art, and as described generally in Example 15, below, using full-length E. coli-produced OP-1 and BMP2 as the immunogens. In all cases, the purified OP-1 reacted only with the anti-OP-1 antibody, and not with anti-BMP2 antibody.

The morphogenic activity of OP-1 purified from mammary gland extract was evaluated in vivo essentially following the rat model assay described in U.S. Pat. No. 4,968,590. Briefly, a sample was prepared from each OP-1 immunoreactive fraction of the mammary gland extract-derived OP-1 final product by lyophilizing a portion (33%) of the fraction and resuspending the protein in 220µl of 50% acetonitrile/0.1% TFA. After vortexing, 25 mg of collagen matrix was added. The samples were lyophilized overnight, and implanted in Long Evans rats (Charles River Laboratories, Wilmington, MA, 28-35 days old). Each fraction was implanted in duplicate. For details of the collagen matrix implantation procedure, see, for example, U.S. Pat. No. 4,968,590. After 12 days, the implants were removed and evaluated for new bone formation by histological observation as described in U.S. Patent No. 4,968,590. In all cases, the immunoreactive fractions were osteogenically active.

### 2.2 Morphogen Detection in Serum

Morphogen may be detected in serum using morphogen-specific antibodies. The assay may be performed using any standard immunoassay, such as Western blot (immunoblot) and the like. Preferably, the assay is performed using an affinity column to which the morphogen-specific antibody is bound and through which the sample serum then is poured, to selectively extract the morphogen of interest. The morphogen then is eluted. A suitable elution buffer may be determined empirically by determining appropriate binding and elution conditions first with a control (e.g., purified, recombinantly-produced morphogen.) Fractions then are tested for the presence of the morphogen by standard immunoblot, and the results confirmed by N-terminal sequencing. Preferably, the affinity column is prepared using monoclonal antibodies. Morphogen concentrations in serum or other fluid samples then may be determined using standard protein quantification techniques, including by spectrophotometric absorbance or by quantitation of conjugated antibody.

Presented below is a sample protocol for identifying OP-1 in serum. Following this general methodology other morphogens may be detected in body fluids, including serum. The identification of morphogen in serum further indicates that systemic administration is a suitable means for providing therapeutic concentrations of a morphogen to an individual, and that morphogens likely behave systemically as endocrine-like factors. Finally, using this protocol, fluctuations in endogenous morphogen levels can be detected, and these altered levels may be used as an indicator of tissue dysfunction. Alternatively, fluctuations in morphogen levels may be assessed by monitoring morphogen transcription levels, either by standard northern blot analysis as described in Example 1, or by in situ hybridization, using a labelled probe capable of hybridizing specifically to morphogen mRNA, and standard RNA hybridization protocols well described in the art and described generally in Example 1.

OP-1 was detected in human serum using the following assay. A monoclonal antibody raised against mammalian, recombinantly produced OP-1 using standard immunology techniques well described in the art and described generally in Example 15, was immobilized by passing the antibody over an agarose-activated gel (e.g., Affi-Gel™, from Bio-Rad Laboratories, Richmond, CA, prepared following manufacturer's instructions) and used to purify OP-1 from serum. Human serum then was passed over the column and eluted with 3M K-thiocyanate. K-thiocyanante fractions then were dialyzed in 6M urea, 20mM PO₄, pH 7.0, applied to a C8 HPLC column, and eluted with a 20 minute, 25-50% acetonitrile/0.1 % TFA gradient. Mature, recombinantly produced OP-1 homodimers elute between 20-22 minutes. Fractions then were collected and tested for the presence of OP-1 by standard immunoblot using an OP-1 specific antibody as for Example 2.A.

Administered or endogenous morphogen levels may be monitored in the therapies described herein by comparing the quantity of morphogen present in a body fluid sample with a predetermined reference value, for example, to evaluate the efficiency of a therapeutic protocol, and the like. In addition, fluctuations in the level of endogenous morphogen antibodies may be detected by this method, most likely in serum, using an antibody or other binding protein capable of interacting specifically with the endogenous morphogen antibody. Detected fluctuations in the levels of the morphogen or endogenous antibody may be used, for example, as indicators of a change in tissue status. For example, as damaged tissue is regenerated and the tissue or organ's function returns to "normal" and, in the absence of additional tissue damage, lower doses of morphogen may be required, and a higher level of circulating morphogen antibody may be measured.

### Example 3. Effect of Morphogen after the Onset of the Ischemic Process

The cardioprotective effect of morphogens following ischemic-reperfusion injury in a mammal can readily be assessed in a rat model. In this example, morphogen (e.g., OP-1) is administered just prior to the onset of the ischemic process in experimentally-induced myocardial infracted rats, essentially following the method of Lefer, et al. (1990) Science 249:61-64 and (1992) J. Mol. Cell. Cardiol. 24: 385-393. Briefly, loss of myocardial tissue function following ischemia and reperfusion is assayed by measuring loss of myocardial creatine kinease activity (CK) and loss of endothelium-dependent vasorelaxation function (see Example 4, below).

In a first group of ether-anesthetized rats, the left coronary artery was occluded just proximal to the first main branch with a silk ligature to induce a myocardial infarction (Ml). The ligature was removed 10 minutes after occlusion to allow for coronary reperfusion. This first group is referred to herein as the "myocardial infarcted" (Ml) group. A second group of rats underwent the same procedure except that the coronary artery was not occluded, and thus no myocardial infarction occurred. The second group of rats is referred to herein as the "sham myocardial infarcted group" (SHAM MI).

The first group of rats, the Ml group of rats, further was divided into three sup-groups. 2µg of morphogen (OP-1) were injected intravenously into the first sub-group of Ml rats 10 minutes after ligature, immediately before reperfusion; into the second sub-group of Ml rats 20 µg of OP-1 were injected intravenously 10 minutes after ligature and immediately before reperfusion; and into the third sub-group of Ml rats (control) was injected vehicle only, e.g., 0.9% NaCl, as for the OP-1 treated rats.

Twenty-four hours later, the hearts were removed from all of the rats and the levels of creatine kinase (CK) from the left ventricle (the infarcted region) and from the interventricular septum (the control nonischemic region) were determined by standard means. By comparing the difference in CK activities in both regions, the amount of CK activity lost from the infarcted region was used as an index of cardiac cellular injury to the infarcted region.

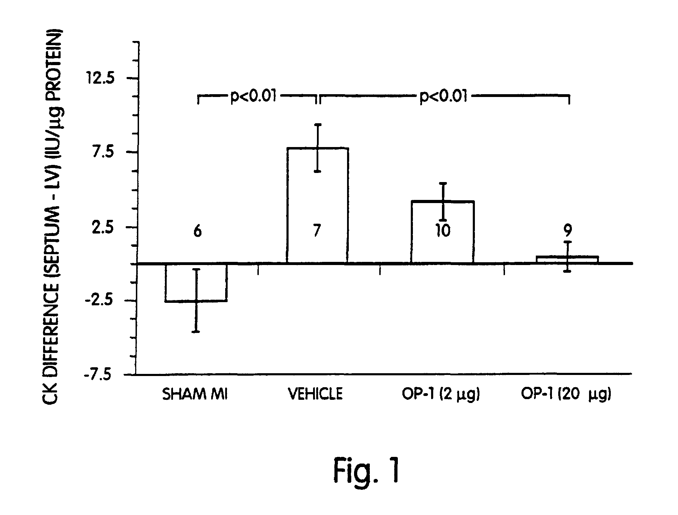

As shown in Figure 1, the data indicate that morphogens (e.g., OP-1) can provide significant cardioprotective effect when provided to ischemic tissue. In the figure, CK loss is graphed as the difference in specific CK activity between the interventricular septum and the left ventricle.

The loss of CK activity by the subgroup of Ml rats which received 2 µg of OP-1 just before reperfusion showed some protection as compared with the control Ml rats which received injections of vehicle alone, when the levels from both subgroups are measured against, and compared to, the levels obtained for the SHAM Ml control. Significant cardioprotection was observed in the subgroup of Ml rats which received 20 µg of OP-1 immediately before reperfusion as compared with the control Ml rats which received injections of vehicle alone, when the levels from both subgroups are measured against, and compared to, the levels contained within the SHAM Ml control.

These data indicate that OP-1 offers significant cardiac protection when administered after ischemia and before reperfusion.

A variation of this example also may be performed providing morphogen to the animal prior to induction of ischemia. The experiments may be performed both in normal and immune-compromised rats to assess the cardioprotective effects of morphogen administered prior to ischemia.

### Example 4. Vasodilation of Myocardial Infarcted Cardiac Tissue Treated with Morphogen

Certain vasodilators like acetylcholine (ACh) and adenosine diphosphate (ADP, an immune mediator) exert their vasodilation activity only in the presence of intact endothelium, which is stimulated to release a substance termed endothelium-derived relaxing factor (EDRF). If the endothelium is injured so that EDRF is not released, no vasodilation occurs in response to these endothelium-dependent agents. In contrast, several other vasodilators including nitroglycerine (NTG) and nitroprusside, are endothelium-independent dilators, as they dilate blood vessels directly.

The present example demonstrates the ability of OP-1 to prevent the loss of cardioendothelium-dependent relaxation (EDR) activity in the coronary microvasculature following reperfusion of ischemic myocardium, and their ability to reduce myocardial injury 24 hours after morphogen treatment. Briefly, 2 or 24 hours after morphogen treatment ischemia-reperfusion injury is induced in isolated rat hearts, the reperfused hearts are are vasodilated with either ACh or NTG. In the absence of morphogen treatment, injured tissue should inhibit ACh-induced vasodilation, but not NTG-induced vasodilation. Morphogen treatment in expected to enhance ACh-induced vasodilation in the reperfused hearts.

Accordingly, 48 adult male Sprague-Dawley rats (250-330 g) were divided into eight groups of 6 rats each. Twelve rats were subjected to sham myocardial infarcts (SHAM MI) as described in Example 3. The hearts of the remaining 36 rats were isolated as follows: one set of twelve rats was injected intravenously with OP-1 24 hours prior to isolation of the heart; another set of rats was injected intravenously with 20µg of OP-1 2 hours prior to isolation of the heart; the final group of rats was injected with vehicle only (e.g., 0.9% NaCl.). The rats then were anesthetized with pentobarbital sodium (35 mg/kg, intraperitonial); their hearts were isolated and perfused by the Langendorff method at a constant flow (15 ml/min) with oxygenated Krebs-Henseleit solution (Aoki et al. (1988) J. Pharmacol. **95:**35).

Each group of rats then were divided into two subgroups of six rats each. Twenty minutes before reperfusion, coronary vasodilator response was measured by inducing constriction with 0.05 µmol U-44619 (9,11-methanoepoxy-prostaglandin H₂) followed by a vasodilating agent 3 minutes later: subgroup one - 15 nmol ACh; subgroup 2 - 15 nmol NTG and the increase in coronary perfusion pressure (CPP) level measured as an indication of vasodilation. When CPP levels returned to normal, the hearts were subjected to ischemia by reducing coronary infusion to 15% of control flow for 30 minutes, then reestablishing normal flow, i.e., reperfusion, for an additional 20 minutes.

The vasodilator reponse then was remeasured by constriction and administration of vasodilating agent as described above.

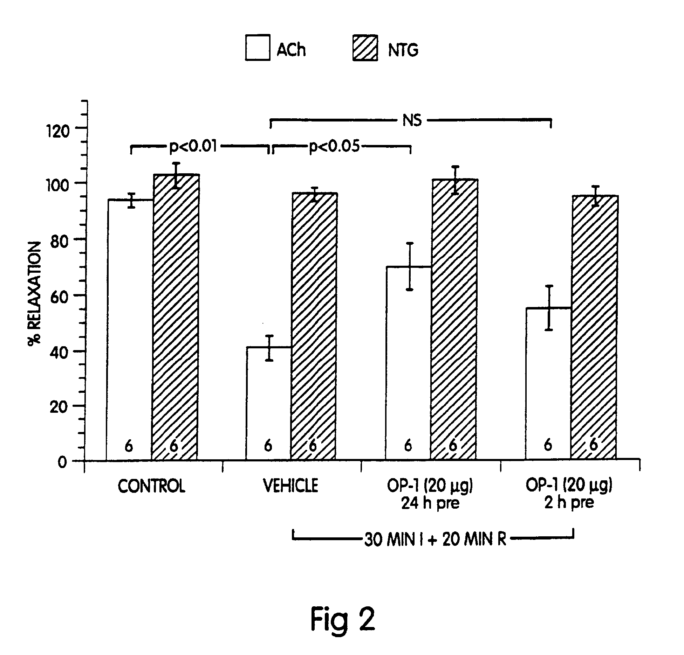

The results of these experiments are shown in FIG 2. Before the ischemic event, both Ach and NTG gave normal vasorelaxant results in all events. The hearts which received OP-1 24 hours prior to ischemia showed an approximately 70% response to ACh while the hearts which received OP-1 2 hours prior to ischemia showed a 55% response to ACh. The group which received vehicle alone showed a 40% response to ACh. Finally, the control group which was not subjected to ischemia showed an ACh response of approximately 95%. This shows that endothelium-dependent vasodilators exert a reduced vasodilator response following ischemia and reperfusion in the rat heart. Moreover, OP-1 significantly preserved endothelium-dependent dilation when provided 24 hours prior to induction of myocardial ischemia. No defect in vasodilation occurred in response to the direct vasodilator (NTG); NTG-induced vasodilation activities were 95% of initial in hearts subject to ischemia and 100% of initial nonischemic hearts.

### Example 5. Effect of Morphogen on Neutrophil Adherence

The role of neutrophil adherence in endothelium dysfunction and the cardioprotective effects of morphogens in modulating this activity can be assessed using a standard polymorphonuclear neutrophil (PMN) adherence assay such as described in Lefer et al., (1992) J. Mol. Cell. Cardiol. 24: 385-393. Briefly, segments of superior mesenteric artery were isolated from rats which had either been treated with morphogen (OP-1, 20 µg) or 0.9% NaCl, 24 h prior to isolation of the artery. The segments were cleaned, cut into transverse rings of 1-2mm in length, and these were subsequently cut open and incubated in K-H solution at 37°C, pH 7.4. Neutrophils were prepared and fluorescently labelled using standard procedures (e.g., leukocytes were isolated from rats essentially following the procedure of Pertroft et. al. (1968) Exp Cell Res 50: 355-368, washed in phosphate buffered saline (PBS), purified by gradient centrifugation; and labelled by the method of Yuan et. al. (1990) Microvasc Res 40: 218-229..

Labelled neutrophils then were added to open ring baths and activated with 100nM leukotriene B₄ (LTB₄). Rings were incubated for 20 minutes and the number of neutrophils adhering to the endothelial surface then determined visually by fluorescent microscopy.

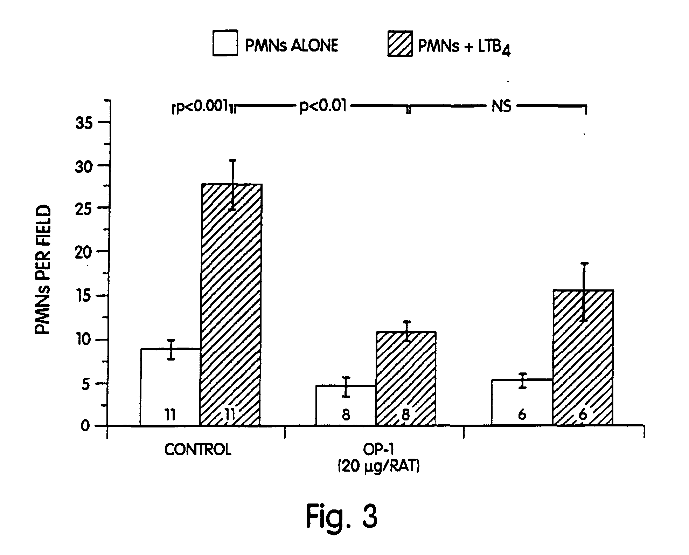

As shown in Figure 3, unstimulated PMNs (i.e., PMNS alone) added to the baths did not significantly adhere to the vascular endothelium. In rings taken from rats injected with 0.9% NaCl, activation of neutrophils with LTB₄ (100 nM) greatly increased the number of PMNs adherent to the endothelium (P<0.001). OP-1 (20 µg administered 24 h prior) significantly inhibited adherence of PMNs activated by LTB₄ (P<0.01 from control).

### Example 6. In Vivo Models for Ischemic-Reperfusion Protection in Lung, Nerve and Renal Tissue.

Other tissues seriously affected by ischemic-reperfusion injury include neural tissue, renal tissue and lung tissue. The effect of morphogens on alleviating the ischemic-reperfusion injury in these tissues may be assessed using methodologies and models known to those skilled in the art, and disclosed below. Similarly, a methodology also is provided for assessing the tissue-protective effects of a morphogen on damaged lung tissue following hyperoxia injury.

For example, the rabbit embolic stroke model provides a useful method for assessing the effect of morphogens on tissue injury following cerebral ischemia-reperfusion. The protocol disclosed below is essentially that of Phillips et al. (1989) Annals of Neurology 25:281-285. Briefly, white New England rabbits (2-3kg) are anesthesized and placed on a respirator. The intracranial circulation then is selectively catheterized by the Seldinger technique. Baseline cerebral angiography then is performed, employing a digital substration unit. The distal internal carotid artery or its branches then is selectively embolized with 0.035 ml of 18-hour-aged autologous thrombus. Arterial occlusion is documented by repeat angiography immediately after embolization. After a time sufficient to induce cerebral infarcts (15 minutes or 90 minutes), reperfusion is induced by administering a bolus of a reperfusion agent such as the TPA analogue Fb-FB-CF (e.g., 0.8 mg/kg over 2 minutes).

The effect of morphogen on cerebral infarcts can be assessed by administering varying concentrations of morphogens, e.g., OP1, at different times preceding or following embolization and/or reperfusion. The rabbits are sacrificed 3-14 days post embolization and their brains prepared for neuropathological examination by fixing by immersion in 10% neutral buffered formalin for at least 2 weeks. The brains then are sectioned in a coronal plane at 2-3 mm intervals, numbered and submitted for standard histological processing in paraffin, and the degree of neutral tissue necrosis determined visually.

The renal-protective effects of morphogens on renal ischemia-reperfusion injury readily can be assessed using the mouse model disclosed by Oueliette, et al. (1990), J. Clin. Invest. 85:766-771. Briefly, renal ischemia is induced surgically in 35-45 days old out-bred Swiss male mice by performing a standard right nephrectomy, and occluding the artery to the left kidney with a microaneurism clamp for 10-30 minutes. Morphogen then may be provided parentally, at various times prior to or following occulsion and/or reperfusion. The effects of morphogen then may be assessed by biological evaluation and histological evaluation using standard techniques well known in the art.

The tissue protective effects of morphogen on tissue exposed to lethally high oxygen concentrations may be assessed by the following procedure. Adult rats (275-300 gms) first are provided with morphogen (e.g., hOP1) or vehicle only, and then are exposed to 96-98% oxygen essentially as described by Rinaldo et al (1983) Am. Rev. Respir. Dis. 130:1065, to induce hyperoxia. Animals are housed in plastic cages (38 cm x 48 xm x 21 cm). A cage containing 4-5 animals is placed in a 75 liter water-sealed plexiglass chamber. An atmosphere of 96-98% oxygen then is maintained by delivery of O₂ gas (liquid O₂). Gas flow through the chamber is adjusted to maintain at least 10 air changes/hr., temperature at 22 ± 1°C, minimal levels of condensation within the cage, and carbon dioxide concentration of < 0.5% as measured with a mass spetrophotometric medical gas analyzer.

At the end of 72 hours all survivors are observed at room air for 1.5 hours and at longer time periods to assess degree of respiratory distress and cyanosis induced by the initial insult and subsequent immune cell-mediated damage. The number of survivors at the end of the challenge is recorded and the treated groups compared with the untreated control group by chi-square test of proportions. Several of the surviving animals for each group are randomly chosen for histological processing of lung tissue.

Lung tissue for histological processing is fixed by infusion of 10% buffered formalin through a tracheal cannula at a constant pressure of 20 cm H₂O. After fixation for 24-48 hours, sections from each lobe are cut and subsequently stained with hematoxylin and eosin. Coded slides then are examined, preferably in a double-blind fashion for evidence of pathological changes such as edema, interstitial cellularity, and inflammatory response.

### Example 7. Morphogen Inhibition of Cellular and Humoral Inflammatory Response

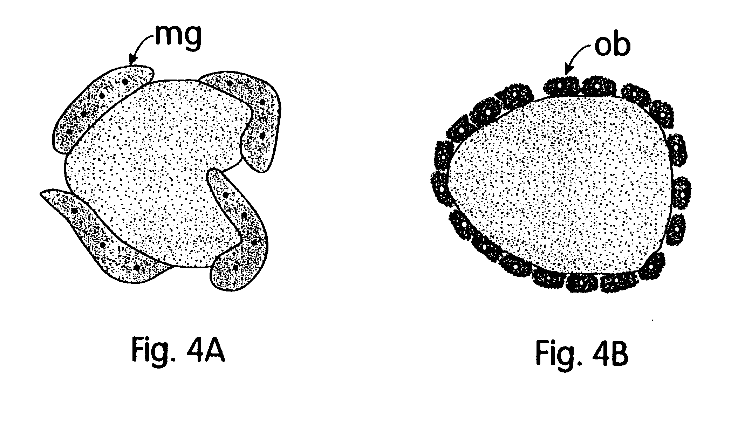

Morphogens described herein inhibit multinucleation of mononuclear phagocytic cells under conditions where these cells normally would be activated, e.g., in response to a tissue injury or the presence of a foreign substance. For example, in the absence of morphogen, an implanted substrate material (e.g., implanted subcutaneously) composed of, for example, mineralized bone, a ceramic such as titanium oxide or any other substrate that provokes multinucleated giant cell formation, rapidly becomes surrounded by multinucleated giant cells, e.g., activated phagocytes stimulated to respond and destroy the foreign object. In the presence of morphogen however, the recruited cells remain in their mononuclear precursor form and the matrix material is undisturbed. Figure 4 illustrates this effect of morphogens, in a schematic representation of histology results of a titanium oxide substrate implanted subcutaneously. In the figure, "mg" means mononuclear giant cells and "ob" means osteoblasts. The substrate represented in Fig. 4B was implanted together with morphogen (OP-1) and newly formed osteoblasts are evident surrounding the substrate. By contrast, the substrate represented in Fig. 4A was implanted without morphogen and extensive multinucleated giant cell formation is evident surrounding the substrate. Accordingly, the morphogens' effect in inhibiting excessive bone mass loss in a mammal also may include inhibiting activation of these cells.

In addition, the morphogens described herein also suppress antibody production stimulated in response to a foreign antigen in a mammal. Specifically, when bovine bone collagen matrix alone was implanted in a bony site in a rat, a standard antibody response to the collagen is stimulated in the rat as determined by standard anti-bovine collagen ELISA experiments performed on blood samples taken at four week intervals following implantation (e.g., between 12 and 20 weeks.) Serum anti-collagen antibody titers, measured by ELISA essentially following the procedure described by Nagler-Anderson et al, (1986) PNAS 83:7443-7446, increased consistently throughout the experiment. However, when the matrix was implanted together with a morphogen (e.g., OP-1, dispersed in the matrix and adsorbed thereto, essentially as described in U.S. Pat. No. 4,968,590) anti-bovine collagen antibody production was suppressed significantly. This ability of morphogen to suppress the humoral response is further evidence of morphogen utility in alleviating tissue damage associated with autoimmune diseases, including autoantibody diseases, such as rheumatoid arthritis.

### Example 8. Morphogen protection of Gastrointestinal Tract Mucosa from Ulceration and Inflammation

Oral mucositis is a gastrointestinal tract inflammatory disease which involves ulcerations of the mouth mucosa as a consequence of, e.g., radiation therapy or chemotherapy. While not typically a chronic disease, the tissue destructive effects of oral mucositis mirror those of chronic inflammatory diseases such as IBD. The example below demonstrates morphogen efficacy in protecting the oral mucosa from oral mucositis in a hamster model, including both inhibiting inflammatory ulceration and enhancing regeneration of ulcerated tissue. Details of the protocol can be found in Sonis, et al., (1990) Oral Surg. Oral Med. Oral Pathol 69: 437-443. Based on these data, the morphogens described herein should be efficacious in treating chronic inflammatory diseases including IBD, arthritis, psoriasis and psoriatic arthritis, multiple sclerosis, and the like.

Golden syrian hamsters (6-8 wks old, Charles River Laboratories, Wilmington, MA) were divided into 3 test groups: Group 1, a placebo (e.g., saline) control, and a morphogen low dose group (100 ng) and a morphogen high dose group (1 µg), Groups 2 and 3, respectively. Morphogen dosages were provided in 30% ethanol. Each group contained 12 animals.

Beginning on day 0 and continuing through day 5, Groups 2 and 3 received twice daily morphogen applications. On day 3, all groups began the mucositis-induction procedure. 5-fluorouracil (60 mg/kg) was injected intraperitoneally on days 3 and 5. On day 7, the right buccal pouch mucosa was superficially irritated with a calibrated 18 gauge needle. In untreated animals, severe ulcerative mucositis was induced in at least 80% of the animals by day 10.

For each administration of the vehicle control (placebo) or morphogen, administration was performed by first gently drying the cheek pouch mucosa, then providing an even application over the mucosal surface of the vehicle or morphogen material. A hydroxypropylcellulose-based coating was used to maintain contact of the morphogen with the mucosa. This coating provided at least 4 hours of contact time.

On day 12, two animals in each group were sacrificed for histological studies. The right buccal pouch mucosa and underlying connective tissue were dissected and fixed in 10% formalin using standard dissection and histology procedures. The specimens were mounted in paraffin and prepared for histologic examination. Sections then were stained with hematoxylin and eosin and were examined blindly by three oral pathologists with expertise in hamster histology and scored blind against a standard mucositis panel. The extent of atrophy, cellular infiltration, connective tissue breakdown, degree of ulceration and epithelialization were assessed.

The mean mucositis score for each group was determined daily for each experimental group for a period of 21 days by photography and visual examination of the right buccal cheek pouch. Differences between groups were determined using a standard 't' test, e.g., the Students' 't' test. In addition, data was evaluated between groups by comparing the numbers of animals with severe mucositis using Chi Square statistical analysis. The significance of differences in mean daily weights also was determined.

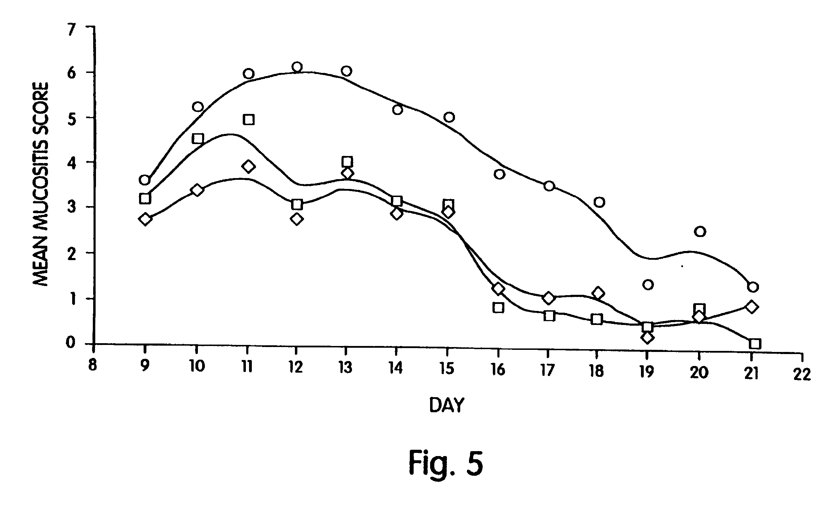

The experimental results are presented in Fig. 5, which graphs the effect of morphogen (high dose, squares; low dose, diamonds) and placebo (circles) on mean mucositis scores. Both low and high morphogen doses inhibit lesion formation significantly in a dose-dependent manner. In addition, histology results consistently showed significantly reduced amounts of tissue atrophy, cellular debris, and immune effector cells, including macrophages and activated neutrophils, in the morphogen-treated animals, as compared with the untreated, control animals.

### Example 9. Morphogen Effect on Fibrogenesis and Scar Tissue Formation

The morphogens described herein induce tissue morphogenesis of damaged or lost tissue. The ability of these proteins to regenerate new tissue enhances the anti-inflammatory effect of these proteins. Provided below are a series of in vitro experiments demonstrating the ability of morphogens to induce migration and accumulation of mesenchymal cells. In addition, the experiments demonstrate that morphogens, unlike TGF-β, do not stimulate fibrogenesis or scar tissue formation. Specifically, morphogens do not stimulate production of collagen, hyaluronic acid (HA) or metalloproteinases in primary fibroblasts, all of which are required for fibrogenesis or scar tissue formation. By contrast, TGF-β, a known inducer of fibrosis, but not of tissue morphogenesis, does stimulate production of these fibrosis markers.

Chemotaxis and migration of mesenchymal progenitor cells were measured in modified Boyden chambers essentially as described by Fava, R.A. et al (1991) J. Exp. Med. 173: 1121-1132, using polycarbonate filters of 2, 3 and 8 micron ports to measure migration of progenitor neutrophils, monocytes and fibroblasts. Chemotaxis was measured over a range of morphogen concentrations, e.g., 10⁻²⁰M to 10⁻¹²M OP-1. For progenitor neutrophils and monocytes, 10⁻¹⁸-10⁻¹⁷M OP-1 consistently induced maximal migration, and 10⁻¹⁴ to 10⁻¹³M OP-1 maximally induced migration of progenitor fibroblasts. In all cases the chemotactic activity could be inhibited with anti-OP-1 antibody. Similar migration activities also were measured and observed with TGF-β.

The effect of morphogen on fibrogenesis was determined by evaluating fibroblast production of hyaluronic acid (HA), collagen, collagenese and tissue inhibitor of metalloproteinases (TIMP).

Human fibroblasts were established from explants of infant foreskins and maintained in monolayer culture using standard culturing procedures. (See, for example, (1976) J. Exp. Med. 144:1188-1203.) Briefly, fibroblasts were grown in maintenance medium consisting of Eagle's MEM, supplemented with nonessential amino acids, ascorbic acid (50 µg/ml), NaHCO₃ and HEPES buffers (pH 7.2), penicillin (100 U/ml), streptomycin (100 µg/ml), amphotericin B (1 µg/ml) and 9% heat inactivated FCS. Fibroblasts used as target cells to measure chemotaxis were maintained in 150 mm diameter glass petri dishes. Fibroblasts used in assays to measure synthesis of collagen, hyaluronic acid, collagenase and tissue inhibitors of metalloproteinases (TIMP) were grown in 100 mm diameter plastic tissue culture petri dishes.

The effects of morphogen on fibroblast production of hyaluronic acid, collagens, collagenase and TIMP were determined by standard assays (See, for example, Posttethwaite et al. (1989) J. Clin. Invest. 83: 629-636, Postteth-waithe (1988) J./Cell Biol. 106:311-318 and Clark et al (1985) Arch. Bio-chem Biophys. 241:36-44). For these assays, fibroblasts were transferred to 24-well tissue culture plates at a density of 8 x 10⁴ cells per well. Fibroblasts were grown confluency in maintenance medium containing 9% FCS for 72 h and then grown in serum-free maintenance medium for 24 h. Medium was then removed from each well and various concentrations of OP-1 (recombinantly produced mature or soluble form) or TGF-β-1 (R&D Systems, Minneapolis) in 50 µl PBS were added to triplicate wells containing the confluent fibroblast monolayers. For experiments that measured production of collagenase and TIMP, maintenance medium (450 µl) containing 5% FCS was added to each well, and culture supernatants were harvested from each well 48 h later and stored at -70°C until assayed. For experiments that assessed HA production, maintenance medium (450 µl) containing 2.5% FCS was added to each well, and cultures grown for 48 h. For experiments that measured fibroblast production of collagens, serum-free maintenance medium (450 µl) without non-essential amino acids was added to each well and cultures grown for 72 h. Fibroblast production of HA was measured by labeling newly synthesized glycosaminoglycans (GAG) with [³H]-acetate the last 24 h of culture and quantitating released radioactivity after incubation with hyaluronidase from Streptomyces hyalurolyticus (ICN Biochemicals, Cleveland, OH) which specifically degrades hyaluronic acid. Production of total collagen by fibroblasts was measured using a collagenase-sensitive protein assay that reflects [³H]-proline incorporation the last 24 h of culture into newly synthesized collagens. Collagenase and TIMP protein levels in fibroblast cultures supernatants was measured by specific ELISAs.

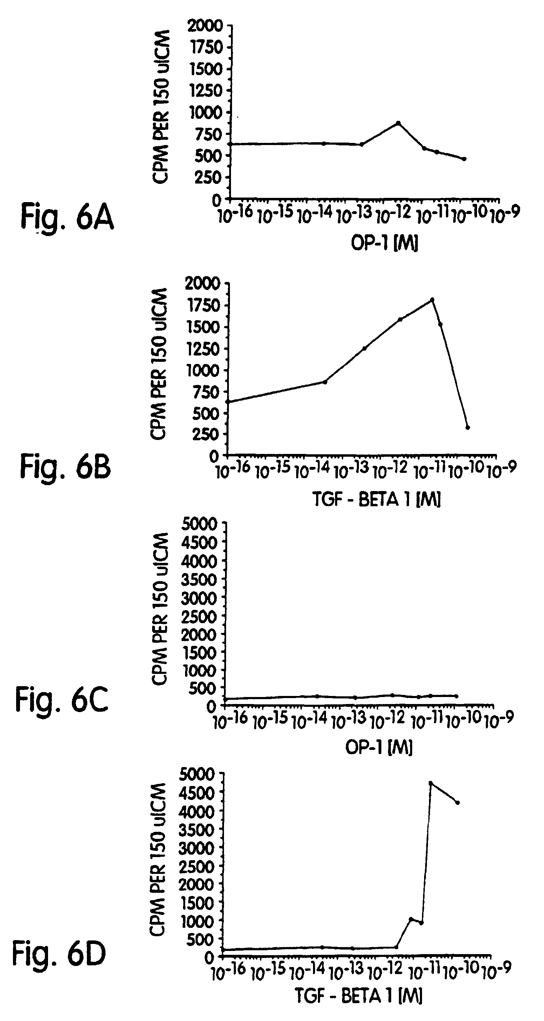

As shown in Fig. 6, OP1 does not stimulate significant collagen or HA production, as compared with TGF-β. In the figure, panel A shows OP-1 efect on collagen production, panel B shows TGF-β effect on collagen production, and panels C and D show OP-1 (panel C) and TGF-β (panel D) effect on HA production. The morphogen results were the same whether the soluble or mature form of OP1 was used. By contrast, the latent form of TGF-β (e.g., pro domain-associated form of TGF-β) was not active.

### Example 10. Morphogen Inhibition of Epithelial Cell Proliferation

This example demonstrates the ability of morphogens to inhibit epithelial cell proliferation in vitro, as determined by ³H-thymidine uptake using culture cells from a mink lung epithelial cell line (ATCC No. CCL 64), and standard mammalian cell culturing procedures. Briefly, cells were grown to confluency in Eagle's minimum essential medium (EMEM) supplemented with 10% fetal bovine serum (FBS), 200 units/ml penicillin, and 200 µg/ml streptomycin, and used to seed a 48-well cell culture plate at a cell density of 200,000 cells per well. When this culture became confluent, the media was replaced with 0.5 ml of EMEM containing 1% FBS and penicillin/streptomycin and the culture incubated for 24 hours at 37 C. Morphogen test samples in EMEM containing 5% FBS then were added to the wells, and the cells incubated for another 18 hours. After incubation, 1.0 µCi of ³H-thymidine in 10 µl was added to each well, and the cells incubated for four hours at 37 C. The media then was removed and the cells washed once with ice-cold phosphate-buffer saline and DNA precipitated by adding 0.5 ml of 10% TCA to each well and incubating at room temperature of 15 minutes. The cells then were washed three times with ice-cold distilled water, lysed with 0.5 ml 0.4 M NaOH, and the lysate from each well then transferred to a scintillation vial and the radioactivity recorded using a scintillation counter (Smith-Kline Beckman).

The results are presented in Table III, below. The anti-proliferative effect of the various morphogens tested was expressed as the counts of 3H-thymidine (x 1000) integrated into DNA, and were compared with untreated cells (negative control) and TGF-β (1 ng), a local-acting factor also known to inhibit epithelial cell proliferation. COP-5 and COP-7 are biosynthetic constructs that previously have been shown to have osteogenic activity, capable of inducing the complete cascade resulting in endochondral bone formation in a standard rat bone assay (see U.S. Pat. No. 5,011,691.) The morphogens significantly inhibit epithelial cell proliferation. Similar experiments, performed with the morphogens COP-16, bOP (bone-purified osteogenic protein, a dimeric protein comprising CBMP2 and OP-1), and recombinant OP-1, also inhibit cell proliferation. bOP and COP-16 also induce endochondral bone formation (see US Pat. No. 4,968,590 and 5,011,691.)

**TABLE III**

| | Thymidine uptake (x 1000) |
|---|---|
| control | 50.048, 53.692 |
| COP-7-1 (10 ng) | 11.874 |
| COP-7-2 (3 ng) | 11.136 |
| COP-5-1 (66 ng) | 16.094 |
| COP-5-2 (164 ng) | 14.43 |
| TGF-β (1 ng) | 1.86, 1.478 |

### Example 11. Morphogen Treatment of a Systemic Inflammatory Disease

The following example provides a rat adjuvant-induced arthritis model for demonstrating morphogen efficacy in treating arthritis and other systemic inflammatory diseases. Rat adjuvant-induced arthritis induces a systemic inflammatory disease with bone and cartilage changes similar to those observed in rhematoid arthritis, but in an accelerated time span (see, for example, Pearson (1964) Arth. Rheum. 7:80). A detailed description of the protocol is provided in Walz, et al., (1971) J. Pharmac. Exp. Ther. 178:223-231.

Briefly, Sprague-Dawley female rats (e.g., Charles River Laboratories, Wilmington, MA) are randomized into 3 groups: control; morphogen, low dose (e.g., 1-10 µg/kg weight per day) and morphogen, high dose (e.g., 10-20 µg/kg weight per day), referred to as Groups 1, 2, and 3, respectively.

Adjuvant arthritis is induced in all three groups by injection of 0.05 ml of a suspension of 1.5% dead Mycobacterium butyricum in mineral oil into the subplantar surface of the right hand paw. On Day 18 after adjuvant injection, the limb volumes of both hind limb are determined. In the absence of morphogen treatment, a systemic arthritic condition is induced in adjuvant-injected rats by this time, as determined by significant swelling of the uninjected hind limbs (<2.3 ml, volume measured by mercury displacement). Subsequent determinations of paw edema and x-ray scores are made on the uninjected hind limb. Rats in Group 2 and 3 also are dosed orally daily, beginning on Day 1, with morphogen. Limb volumes are recorded on Days 29 and 50 after adjuvant injection and edema determined by volume difference compared to Day 18. The uninjected hind limb on each rat is x-rayed on Day 50 and the joint damage assayed on an arbitrary scale of 1 to 10 (1=no damage, 10=maximum damage). Data on differences between control and treated groups (Day 29 edema, Day 50 edema and Day 50 x-ray scores) are analyzed by using a standard "t-test. Morphogen-treated rats show consistently reduced joint damage (e.g., decreased in edema and in x-ray scores) as compared with untreated control rats.

As another, alternative example, Groups 2 and 3 are dosed daily with morphogen beginning on Day 18 and continuing through Day 50 to demonstrate the efficacy of morphogens in arthritic animals.

### Example 12. Morphogen Inhibition of Localized Edema

The following example demonstrates morphogen efficacy in inhibiting a localized inflammatory response in a standard rat edema model. Experimental rats (e.g., Long-Evans from Charles River Laboratories, Wilmington, MA) are divided into three groups: Group 1, a negative control, which receives vehicle alone; Group 2, a positive control, to which is administered a well-known characterized anti-inflammatory agent (e.g., indomethacin), and Group 3, to which morphogen is provided.

Groups 2 and 3 may be further subdivided to test low, medium and high doses (e.g., Group 2: 1.0 mg/kg, 3.0 mg/kg and 9.0 mg/kg indomethacin; Group 3: 0.1-5µg; 5-20µg, and 20-50µg of morphogen). Sixty minutes after indomethacin or morphogen is provided to the rats of Group 2 or 3 (e.g., as by injection into the tail vein, or by oral gavage) inflammation is induced in all rats by a sub-plantar injection of a 1% carrageenin solution (50µl) into the right hind paw. Three hours after carrageenin administration paw thickness is measured as an indication of edema (e.g., swelling) and induced inflammatory response to the injected carrageenin solution.

Significant swelling is evident in untreated rats by three hours after carrageenin injection. Inflammation also is measured by histology by standard means, following euthanasia e.g.: the right hind paw from each animal is removed at the ankle joint and weighed and foot pad tissue is fixed in 10% neutral buffered formalin, and slides prepared for visual examination by staining the prepared tissue with hematoxylin and eosin.

The morphogen-treated rats show substantially reduced edema induction following carrageenin injection as compared with the untreated rats.

### Example 13. Morphogen Treatment of Allergic Encephalomyelitis

The following example demonstrates morphogen efficacy in treating experimental allergic encephalomyelitis (EAE) in a rat. EAE is a well-characterized animal model for multiple sclerosis, an autoimmune disease. A detailed description of the protocol is disclosed in Kuruvilla, et al., (1991) PNAS 88:2918-2921.

Briefly, EAE is induced in rats (e.g., Long-Evans, Charles River Laboratories, Wilmington, MA) by injection of a CNS tissue (e.g., spinal cord) homogenate in complete Freund's adjuvant (CFA) on days -44, -30 and 0 (last day of immunization), by subcutaneous injection to three sites on the animal's back. Morphogen is administered daily by interperitoneal injection beginning on day -31. Preferably, a series of morphogen dose ranges is evaluated (e.g., low, medium and high) as for Example 12, above.) Control rats receive morphogen vehicle only (e.g. 0.9% NaCl or buffered saline). Rats are examined daily for signs of disease and graded on an increasing severity scale of 0-4.

In the absence of morphogen treatment, significant neurological dysfunction (e.g., hind and fore limb weakness, progressing to total hind limb paralysis) is evident by day +7 to +10. Hematology, serum chemistry profiles and histology are performed to evaluate the degree of tissue necropsy using standard procedures. Morphogen treatment significantly inhibits the neurological dysfunction normally evident in an EAE animal. In addition, the histopathological markers typically associated with EAE are absent in the morphogen-treated animals

### Example 14. Morphogen Treatment of Collagen-Induced Arthritis

The following example demonstrates the efficacy of morphogens in inhibiting the inflammatory response in a collagen-induced arthritis (CIA) in a rat. CIA is a well-characterized animal model for rheumatoid arthritis, an autoimmune disease. The protocol disclosed is essentially that disclosed in Kuruvilla et al., (1991) PNAS 88:2918-2921. Briefly, CIA is induced in experimental rats (e.g., Long-Evans, Charles River Laboratories, Wilmington), by multiple intradermal injection of bovine Type II collagen (e.g., 100µg) in CFA (0.2 ml) on Day 1. Animals are divided into two groups: Group 1, control animals, which receive vehicle alone, and Group 2: morphogen-treated animals, which, preferably, are subdivided into low, medium and high dose ranges, as described for Example 13, above. Morphogen is administered daily (e.g., by tail vein injection) beginning at different times following collagen injection, e.g., beginning on day 7, 14, 28, 35 and 42. Animals are evaluated visually and paw thickness and body weight is monitored throughout the experiment. Animals are sacrificed on day 60 and the proximal and distal limb joints, and ear, tail and spinal cord prepared for histological evaluation as described for Examples 12 and 13, above. In a variation of the experiment, morphogen may be administered for prescribed periods, e.g., five day periods, beginning at different times following collagen injection (e.g., on days 0-4, 7-11, 14-18, 28-32.)

In the absence of morphogen treatment, an arthritic condition typically is induced by 30 days post collagen injection. In morphogen-treated animals, CIA is suppressed and the histopathological changes typically evidenced in control CIA-induced animals are absent: e.g., accumulations of activated mononuclear inflammatory cells and fibrous connective tissue. In addition, consistent with the results in Example 7, above, serum anti-collagen antibody titers are suppressed significantly in the morphogen-treated animals.

### Example 15. Screening Assay for Candidate Compounds which Alter Endogenous Morphogen Levels

Candidate compound(s) which may be administered to affect the level of a given morphogen may be found using the following screening assay, in which the level of morphogen production by a cell type which produces measurable levels of the morphogen is determined with and without incubating the cell in culture with the compound, in order to assess the effects of the compound on the cell. This can be accomplished by detection of the morphogen either at the protein or RNA level. A more detailed description also may be found in USSN 752,861.

### 15.1 Growth of Cells in Culture

Cell cultures of kidney, adrenals, urinary bladder, brain, or other organs, may be prepared as described widely in the literature. For example, kidneys may be explanted from neonatal or new born or young or adult rodents (mouse or rat) and used in organ culture as whole or sliced (1-4 mm) tissues. Primary tissue cultures and established cell lines, also derived from kidney, adrenals, urinary, bladder, brain, mammary, or other tissues may be established in multiwell plates (6 well or 24 well) according to conventional cell culture techniques, and are cultured in the absence or presence of serum for a period of time (1-7 days). Cells may be cultured, for example, in Dulbecco's Modified Eagle medium (Gibco, Long Island, NY) containing serum (e.g., fetal calf serum at 1%-10%, Gibco) or in serum-deprived medium, as desired, or in defined medium (e.g., containing insulin, transferrin, glucose, albumin, or other growth factors).

Samples for testing the level of morphogen production includes culture supernatants or cell lysates, collected periodically and evaluated for OP-1 production by immunoblot analysis (Sambrook et al., eds., 1989, Molecular Cloning, Cold Spring Harbor Press, Cold Spring Harbor, NY), or a portion of the cell culture itself, collected periodically and used to prepare polyA+ RNA for RNA analysis. To monitor de novo OP-1 synthesis, some cultures are labeled according to conventional procedures with an ³⁵S-methionine/³⁵S-cysteine mixture for 6-24 hours and then evaluated to OP-1 synthesis by conventional immunoprecipitation methods.

### 15.2 Determination of Level of Morphogenic Protein

In order to quantitate the production of a morphogenic protein by a cell type, an immunoassay may be performed to detect the morphogen using a polyclonal or monoclonal antibody specific for that protein. For example, OP-1 may be detected using a polyclonal antibody specific for OP-1 in an ELISA, as follows.

1 µg/100 µl of affinity-purified polyclonal rabbit IgG specific for OP-1 is added to each well of a 96-well plate and incubated at 37°C for an hour. The wells are washed four times with 0.167M sodium borate buffer with 0.15 M NaCl (BSB), pH 8.2, containing 0.1% Tween 20™. To minimize non-specific binding, the wells are blocked by filling completely with 1% bovine serum albumin (BSA) in BSB and incubating for 1 hour at 37°C. The wells are then washed four times with BSB containing 0.1% Tween 20. A 100 µl aliquot of an appropriate dilution of each of the test samples of cell culture supernatant is added to each well in triplicate and incubated at 37°C for 30 min. After incubation, 100 µl biotinylated rabbit anti-OP-1 serum (stock solution is about 1 mg/ml and diluted 1:400 in BSB containing 1% BSA before use) is added to each well and incubated at 37°C for 30 min. The wells are then washed four times with BSB containing 0.1% Tween 20. 100 µl strepavidin-alkaline (Southern Biotechnology Associates, Inc. Birmingham, Alabama, diluted 1:2000 in BSB containing 0.1% Tween 20 before use) is added to each well and incubated at 37°C for 30 min. The plates are washed four times with 0.5M Tris buffered Saline (TBS), pH 7.2. 50µl substrate (ELISA Amplification System Kit, Life Technologies, Inc., Bethesda, MD) is added to each well incubated at room temperature for 15 min. Then, 50 µl amplifier (from the same amplification system kit) is added and incubated for another 15 min at room temperature. The reaction is stopped by the addition of 50 µl 0.3 M sulphuric acid. The OD at 490 nm of the solution in each well is recorded. To quantitate OP-1 in culture media, a OP-1 standard curve is performed in parallel with the test samples.

Polyclonal antibody may be prepared as follows. Each rabbit is given a primary immunization of 100 ug/500 µl E. coli produced OP-1 monomer (amino acids 328-431 in SEQ ID NO:5) in 0.1% SDS mixed with 500 µl Complete Freund's Adjuvant. The antigen is injected subcutaneously at multiple sites on the back and flanks of the animal. The rabbit is boosted after a month in the same manner using incomplete Freund's Adjuvant. Test bleeds are taken from the ear vein seven days later. Two additional boosts and test bleeds are performed at monthly intervals until antibody against OP-1 is detected in the serum using an ELISA assay. Then, the rabbit is boosted monthly with 100 µg of antigen and bled (15 ml per bleed) at days seven and ten after boosting.

Monoclonal antibody specific for a given morphogen may be prepared as follows. A mouse is given two injections of E. coli produced OP-1 monomer. The first injection contains 100µg of OP-1 in complete Freund's adjuvant and is given subcutaneously. The second injection contains 50 µg of OP-1 in incomplete adjuvant and is given intraperitoneally. The mouse then receives a total of 230 µg of OP-1 (amino acids 307-431 in SEQ ID NO:5) in four intraperitoneal injections at various times over an eight month period. One week prior to fusion, both mice are boosted intraperitoneally with 100 µg of OP-1 (307-431) and 30 µg of the N-terminal peptide (Ser₂₉₃-Asn₃₀₉-Cys) conjugated through the added cysteine to bovine serum albumin with SMCC crosslinking agent. This boost was repeated five days (IP), four days (IP), three days (IP) and one day (IV) prior to fusion. The mouse spleen cells are then fused to myeloma (e.g., 653) cells at a ratio of 1:1 using PEG 1500 (Boeringer Mannheim), and the cell fusion is plated and screened for OP-1-specific antibodies using OP-1 (307-431) as antigen. The cell fusion and monoclonal screening then are according to standard procedures well described in standard texts widely available in the art.

The invention may be embodied in other specific forms and the present embodiments are therefore to be considered in all respects as illustrative and not restrictive, the scope of the invention being indicated by the appended claims.

## Claims

1. Use of a morphogen selected from the group consisting of OP-1, OP-2, BMP-5, BMP-6, BMP-2, BMP-4, Dpp, Vgl, Vgr-1, GDF-1, 60A, BMP-3, COP-1, COP-3, COP-4, COP-5, COP-7, COP-16; or a morphogen comprising a dimeric protein that induces tissue-specific morphogenesis in a mammal (eg a human) and comprises a pair of folded polypeptides, the amino acid sequence of each of which comprises
(a) the sequence of the seven cysteine domain of human OP-1, mouse OP-1, human OP-2, mouse OP-2 or 60A; or
(b) an amino acid sequence selected from Seq. ID Nos 9, 10, 11, 12, 13, 14, 26, 27, 28 or 29 (CBMP2A(fx), CBMP2B(fx), DPP(fx), Vgl(fx), Vgr-1(fx), GDF-1(fx), BMP3, BMP5, BMP6 or OPX), or
(c) an amino acid sequence of residues 38-139 of Seq. ID Nos. 5, 6, 7 or 8 or residues 354-455 of Seq ID No. 24.
for the manufacture of a medicament for:
(a) modulating an inflammatory response; or
(b) alleviating the tissue destructive effects associated with the inflammatory response to tissue injury; or

2. Use according to claim 1 for modulating an inflammatory response in mammalian tissue, wherein the modulation of said inflammatory response comprises:
(a) alleviation of fibrosis or scar tissue formation; and/or
(b) inhibition of adherence of immune effector cells to vascular endothelium; and/or
(c) alleviation of interstitial inflammation; and/or
(d) alleviation of edema or erythema; and/or
(e) alleviation of inflammatory tissue necrosis.

3. Use according to claim 1 or claim 2 wherein the inflammatory response is associated with tissue injury (the injury for example being chemically, mechanically, biologically or immunologically related).

4. Use according to any one of the preceding claims wherein said medicament is administered: (a) for the treatment or prophylaxis of said tissue injury; and/or (b) in an amount effective to inhibit fibrogenesis or stimulate tissue-specific regeneration of said injured or damaged tissue; and/or (c) to inhibit loss or reduction of function of said tissue; and/or (d) orally, parenterally or topically; and/or (e) dispersed in a liquid tissue adhesive; and/or (f) in an aerosol.

5. Use according to claim 3 or 4 wherein said tissue injury includes:
(a) ischaemic-repurfusion injury; or
(b) hyperoxia injury.

6. Use according to claim 5(a) wherein said ischaemic-repurfusion injury is:
(a) associated with cardiac arrest, pulmonary occlusion, arterial (eg renal artery) occlusion, coronary occlusion or occlusive stroke; or
(b) associated with surgery or the reduction or interruption of blood flow to an organ in a clinical procedure (eg carotoid enterectomy, coronary artery bypass, a tissue grafting procedure, an organ transplant or fibronolytic therapy); or
(c) associated with cerebral infarction, myocardial infarction, asphyxia or cardiopulmonary arrest.

7. Use according to claim 5(b) wherein the hyperoxia injury is associated with the treatment of:
(a) a prematurely newborn baby; or
(b) emphysema; or
(c) asphyxia.

8. Use according to any one of the preceding claims wherein the tissue is lung, cardiac, hepatic, neural, pancreatic, synovial, skin or renal tissue, for example wherein:
(a) the tissue is renal tissue and the mammal is afflicted with glomerular nephritis, atherosclerosis or diabetes, or
(b) the tissue is cardiac tissue and the mammal is afflicted with atherosclerosis or vascular disease, or
(c) the tissue is pancreatic tissue and the mammal is afflicted with diabetes, or
(d) the tissue is synovial tissue and the mammal is afflicted with arthritis,
(e) the tissue is skin tissue and the mammal is afflicted with dermatitis or psoriasis,
(f) the tissue is lung tissue and the mammal is afflicted with bronchitis, emphysema, idiopathic pulmonary fibrosis, asthma, asphyxia, or adult respiratory distress syndrome.

9. Use according to claim 3 or 4 wherein the tissue injury includes:
(a) an immune-cell mediated inflammatory response; or
(b) an inflammatory disease (eg a chronic inflammatory disease); or
(c) an abnormal immune response; or
(d) edema or erythema.

10. Use according to claim 9(a), 9(c) or 9(d) wherein the tissue injury includes:
(a) a generalized acute inflammatory response; or
(b) airway inflammation.

11. Use according to claim 9(b) wherein the inflammatory disease includes:
(a) an autoimmune disease; or
(b) arthritis, psoriasis, dermatitis, inflammatory bowel disease or diabetes.

12. Use according to claim 10 wherein:
(a) in claim 10(a) the acute inflammatory response is associated with asphyxia or adult respiratory distress syndrome; or
(b) in claim 10(b) the airway inflammation is associated with chronic bronchitis, emphysema, idiopathic pulmonary fibrosis or asthma.

13. Use according to claim 11 wherein:
(a) in claim 11(a) the autoimmune disease is a neurodegenerative disease (eg multiple sclerosis or amyotrophic lateral sclerosis); or
(b) in claim 11(b) the arthritis is rheumatoid, degenerative or psoriatic arthritis.

14. Use according to any one of claims 3-13 wherein the modulation of the inflammatory response is effected by administration of the medicament before or after the injury, for example prior to or after the reduction or interruption of blood flow or after resumption of blood flow when the injury is ischaemic-repurfusion injury.

15. Use according to claim 6(b) wherein said clinical procedure is a tissue grafting procedure (eg of skin, bone marrow or gastrointestinal mucosa tissue) or an organ transplant procedure (eg of lung, heart, kidney, liver or pancreas).

16. Use according to claim 15 wherein the modulation of the inflammatory response is effected by administration of the medicament:
(a) upon or following removal of the organ or tissue from a donor host; or
(b) during storage or transportation of the organ or tissue prior to implantation into a recipient host; or
(c) upon or following implantation into a recipient host.

17. Use according to any one of the preceding claims wherein said medicament comprises the morphogen dispersed in a physiologically acceptable aqueous carrier suitable for parenteral administration to a mammal.

18. A pharmaceutical composition for use in alleviating injury associated with exposure of mammalian tissue to toxic oxygen concentrations, comprising a morphogen dispersed in a physiologically acceptable aqueous carrier to a concentration effective for alleviating extravasation of immune effector cells, interstitial inflammation, edema, necrosis or fibrogenesis in mammalian tissue at risk of, or afflicted with, said injury, said morphogen selected from the group consisting of OP-1, OP-2, BMP-5, BMP-6, BMP-2, BMP-4, Dpp, Vgl, Vgr-1, GDF-1, 60A, BMP-3, COP-1, COP-3, COP-4, COP-5, COP-7, COP-16; or said morphogen comprising a dimeric protein that induces tissue-specific morphogenesis in a mammal, said dimeric protein comprising a pair of folded polypeptides, the amino acid sequence of each of which comprises the C-terminal seven cysteine domain of human OP-1 (residues 38-139 of Seq. ID No. 5), said composition further comprising a free oxygen radical inhibiting agent or an anticoagulant

19. A preservation solution for maintaining ex vivo viability of mammalian cells or tissue or a mammalian organ, comprising a fluid formulation having an osmotic pressure substantially equivalent to the osmotic pressure of living mammalian cells and a morphogen dispersed in said fluid to a concentration effective for alleviating extravasation of immune effector cells, interstitital inflammation, edema, necrosis or fibrogenesis in said cells, tissue or organ, said morphogen selected from the group consisting of OP-1, OP-2, BMP-5, BMP-6, BMP-2, BMP-4, Dpp, Vgl, Vgr-1, GDF-1, 60A, BMP-3, COP-1, COP-3, COP-4, COP-5, COP-7, COP-16; or said morphogen comprising a dimeric protein that induces tissue-specific morphogenesis in a mammal, said dimeric protein comprising a pair of folded polypeptides, the amino acid sequence of each of which comprises the C-terminal seven cysteine domain of human OP-1 (residues 38-139 of Seq. ID No. 5).

20. A solution according to claim 19, further comprising a sugar, an anticoagulant or a free oxygen radical inhibiting agent; or, further formulated to maintain substantially normal ATP levels in said cells, tissue or organ.

21. The composition of claim 18 or the solution of claim 19 or 20 wherein the morphogen is as defined in claim 1.

22. An ex vivo method for protecting a living mammalian tissue or transplanted mammalian organ from injury associated with the inflammatory response of activated immune effector cells comprising the step of providing to said tissue or organ a morphogen selected from the group consisting of OP-1, OP-2, BMP-5, BMP-6, BMP-2, BMP-4, Dpp, Vgl, Vgr-1, GDF-1, 60A, BMP-3, COP-1, COP-3, COP-4, COP-5, COP-7, COP-16; or said morphogen omprising a dimeric protein that induces tissue-specific morphogenesis in a mammal, said dimeric protein comprising a pair of folded polypeptides, the amino acid sequence of each of which comprises the C-terminal seven cysteine domain of human OP-1 (residues 38-139 of Seq. ID No. 5).

23. The method of claim 22 wherein:
(a) the tissue or organ is as defined in claim 15; and/or
(b) the morphogen is as defined in claim 1,
(c) the morphogen is provided in the form of a solution as defined in claim 19 or 20.

## Patentansprüche

1. Verwendung eines Morphogens, das aus der Gruppe ausgewählt ist, die aus OP-1, OP-2, BMP-5, BMP-6, BMP-2, BMP-4, Dpp, Vgl, Vgr-1, GDF-1, 60A, BMP-3, COP-1, COP-3, COP-4, COP-5, COP-7, COP-16 besteht; oder eines Morphogens, das ein dimeres Protein umfaßt, das bei einem Säugetier (beispielsweise einem Menschen) eine gewebsspezifische Morphogenese induziert und zwei gefaltete Polypeptide umfaßt, wobei die Aminosäure-Sequenz von jedem folgendes umfaßt:
(a) die Sequenz der Sieben-Cystein-Domäne von menschlichem OP-1, Maus OP-1, menschlichem OP-2, Maus OP-2 oder 60A; oder
(b) eine Aminosäure-Sequenz, die aus den Sequenz ID Nr. 9, 10, 11, 12, 13, 14, 26, 27, 28 oder 29 (CBMP2A (fx), CBMP2B(fx), DPP(fx), Vgl(fx), Vgr-1(fx), GDF-1(fx), BMP3, BMP5, BMP6 oder OPX) ausgewählt ist; oder
(e) eine Aminosäure-Sequenz der Reste 38-139 von Sequenz ID Nr. 5, 6, 7 oder 8 oder der Reste 354-455 von Sequenz ID Nr. 24; zur Herstellung eines Medikamentes zur:
(a) Modulierung einer Entzündungsreaktion; oder
(b) Verminderung der Gewebs-zerstörenden Wirkungen, die mit der Entzündungsreaktion auf eine Gewebsschädigung verbunden sind.

2. Verwendung nach Anspruch 1 zur Modulierung einer Entzündungsreaktion in Säugetiergewebe, wobei die Modulation der Entzündungsreaktion folgendes umfaßt:
(a) Verminderung einer Fibrose oder Narbengewebsbildung; und/oder
(b) Hemmung der Anhaftung von Immuneffektorzellen an vaskuläres Endothel; und/oder
(c) Verminderung einer interstitiellen Entzündung; und/oder
(d) Verminderung eines Ödems oder Erythems; und/oder
(e) Verminderung einer entzündlichen Gewebsnekrose.

3. Verwendung nach Anspruch 1 oder Anspruch 2, bei der die Entzündungsreaktion mit einer Gewebsschädigung verbunden ist (wobei die Verletzung beispielsweise chemisch, mechanisch, biologisch oder immunologisch verursacht ist).

4. Verwendung nach einem der vorhergehenden Ansprüche, bei der das Medikament:
(a) zur Behandlung oder Prophylaxe der Gewebsschädigung; und/oder
(b) in einer Menge, die zur Hemmung einer Fibrogenese oder zur Stimulierung einer gewebsspezifischen Regeneration des verletzten oder geschädigten Gewebes wirksam ist; und/oder
(c) zur Hemmung eines Verlustes oder einer Verminderung der Funktion des Gewebes; und/oder
(d) oral, parenteral oder topisch; und/oder
(e) in einem flüssigen Gewebsklebstoff dispergiert; und/oder
(f) in einem Aerosol
verabreicht wird.

5. Verwendung nach Anspruch 3 oder Anspruch 4, bei der die Gewebsschädigung:
(a) eine ischämische Reperfusions-Schädigung; oder
(b) Hyperoxie-Schädigung
einschließt.

6. Verwendung gemäß Anspruch 5 (a), bei der die ischämische Reperfusions- Schädigung:
(a) mit Herzstillstand, Lungenarterienverschluß, Arterienverschluß (beispielsweise Nierenarterienverschluß), Koronararberienverschluß oder durch Okklusion verursachten Schlag verbunden ist; oder
(b) mit einem chirurgischen Eingriff oder der Verminderung oder Unterbrechung des Blutstromes zu einem Organ bei einem klinischen Verfahren (beispielsweise einer Carotis-Enterektomie, aorto-koronarem Bypass, einem Gewebstransplantationsverfahren, einer Organtranaplantations- oder fibronolytische Therapie) verbunden ist; oder
(c) mit einem Gehirninfarkt, Herzinfarkt, mit Asphyxie oder Herz-/Lungenstillstand verbunden ist.

7. Verwendung nach Anspruch 5 (b), bei der die Hyperoxie-Schädigung mit der Behandlung:
(a) eines frühgeborenen Säuglings; oder
(b) Emphysems; oder
(c) Asphyxie
verbunden ist.

8. Verwendung nach einem der vorhergehenden Ansprüche, bei der das Gewebe Lungen-, Herz-, Leber-, Nerven-, Pankreas-, Synovial-, Haut- oder Nierengewebe ist, bei dem beispielsweise:
(a) das Gewebe Nierengewebe ist und das Säugetier an glomerulärer Nephritis, Arteriosklerose oder Diabetes leidet, oder
(b) das Gewebe Herzgewebe ist und das Säugetier an Arteriosklerose oder einer Gefäßerkrankung leidet, oder
(c) das Gewebe Pankreasgewebe ist und das Säugetier an Diabetes leidet, oder
(d) das Gewebe Synovialgewebe ist und das Säugetier an Arthritis leidet,
(e) das Gewebe Hautgewebe ist und das Säugetier an Dermatitis oder Psoriasis leidet,
(f) das Gewebe Lungengewebe ist und das Säugetier an Bronchitis, Emphysem, ideopathischer Lungenfibrose, Asthma, Asphyxie oder Schocklunge leidet.

9. Verwendung nach Anspruch 3 oder Anspruch 4, bei der die Gewebsverletzung;
(a) eine Immunzell-vermittelte Entzündungsreaktion; oder
(b) eine entzündliche Erkrankung (beispielsweise eine chronische entzündliche Erkrankung); oder
(c) eine abnormale Immunreaktion; oder
(d) ein Ödem oder Erythem einschließt.

10. Verwendung nach Anspruch 9 (a), 9 (c) oder 9 (d), bei der die Gewebsschädigung:
(a) eine generalisierte akute entzündliche Reaktion; oder
(b) Atemwegsentzündung
einschließt.

11. Verwendung nach Anspruch 9 (b), bei der die entzündliche Erkrankung:
(a) eine Autoimmunerkrankung; oder
(b) Arthritis, Psoriasis, Dermatitis, eine entzündliche Darmerkrankung oder Diabetes
einschließt.

12. Verwendung nach Anspruch 10, bei der:
(a) in Anspruch 10 (a) die akute entzündliche Reaktion mit Asphyxie oder Schocklunge verbunden ist; oder
(b) in Anspruch 10 (b) die Atemwegsentzündung mit chronischer Bronchitis, Emphysem, ideopathischer Lungenfibrose oder Asthma verbunden ist.

13. Verwendung nach Anspruch 11, bei der:
(a) in Anspruch 11 (a) die Autoimmunerkrankung eine neurodegenerative Erkrankung (beispielsweise Multiple Sklerose oder amyotrophe Lateralsklerose) ist; oder
(b) in Anspruch 11 (b) die Arthritis eine rheumatoide, degenerative oder psoriatische Arthritis ist.

14. Verwendung nach einem der Ansprüche 3 bis 13, bei der die Modulation der Entzündungsreaktion durch Verabreichung des Medikaments vor oder nach der Schädigung, beispielsweise vor oder nach der Verringerung oder Unterbrechung des Blutstromes oder nach Wiederaufnahme eines Blutstromes verabreicht wird, wenn die Schädigung eine ischämische Reperfusions-Schädigung ist.

15. Verwendung nach Anspruch 6 (b), bei der das klinische Verfahren ein Gewebs-Transplantations-Verfahren (beispielsweise von Haut-, Knochenmark- oder Gewebe der Gastrointestinalschleimhaut) oder ein Organtransplantations-Verfahren (beispielsweise von Lunge, Herz, Niere, Leber oder Pankreas) ist.

16. Verwendung nach Anspruch 15, bei der die Modulation der Entzündungsreaktion durch Verabreichung des Medikamentes:
(a) bei oder im Anschluß an die Entfernung des Organs oder Gewebes von einem Spender; oder
(b) während der Aufbewahrung oder des Transportes des Organs oder Gewebes vor Implantation in einen Empfänger; oder
(c) bei oder im Anschluß an Implantation in einen Empfänger
bewirkt wird.

17. Verwendung nach einem der vorhergehenden Ansprüche, bei der das Medikament das das Morphogen in einem physiologisch akzeptablen wässrigen Träger, der zur parenteralen Verabreichung an ein Säugetier geeignet ist, dispergiert umfaßt.

18. Pharmazeutische Zusammensetzung zur Verwendung bei der Linderung einer Verletzung, die mit der Exposition von Säugetiergewebe gegenüber toxischen Sauerstoff--Konzentrationen verbunden ist, die ein Morphogen in einem physiologisch verträglichen wässrigen Träger in einer Konzentration dispergiert umfaßt, die zur Verminderung einer Extravasation von Immuneffektorzellen, einer interstitiellen Entzündung, eines Ödems, einer Nekrose oder einer Fibrogenese in säugetiergewebe wirksam ist, das dem Risiko der Schädigung ausgesetzt ist oder darunter leidet, wobei das Morphogen aus der Gruppe ausgewählt ist, die aus OP-1, OP-2, BMP-5, BMP-6, BMP-2, BMP-4, Dpp, Vgl, Vgr-1, GDF-1, 60A, BMP-3, COP-1, COP-3, COP-4, COP-5, COP-7, COP-16 besteht; oder das Morphogen ein dimeres Protein umfaßt, das eine gewebsspezifische Morphogenese bei einem Säugetier induziert, wobei das dimere Protein zwei gefaltete Polypeptide umfaßt, und wobei die Aminosäure-Sequenz jedes der beiden die C-terminale Sieben-Cystein-Domäne von menschlichem OP-1 (Reste 38-139 von Seq. ID Nr. 5) umfaßt, wobei die Zusammensetzung weiterhin ein Mittel zur Hemmung freier Sauerstoff-Radikale oder ein Antikoagulanz umfaßt.

19. Eine Konservierungslösung zur Aufrechterhaltung der ex vivo-Lebensfähigkeit von Säugetierzellen oder -gewebe oder eines Säugetierorgans, das eine flüssige Zubereitung umfaßt, die einen im wesentlichen dem osmotischen Druck lebender Säugetierzellen äquivalenten Druck und ein in der Flüssigkeit in einer Konzentration dispergiertes Morphogen aufweist, die zur Verminderung der Extravasation von Immuneffektorzellen, einer interstitiellen Entzündung, eines Ödems, einer Nekrose oder Fibrogenese bei diesen Zellen, Gewebe oder Organ wirksam ist, wobei das Morphogen aus der Gruppe ausgewählt ist, die aus OP-1, OP-2, BMP-5, BMP-6, BMP-2, BMP-4, Dpp, Vgl, Vgr-1, GDF-1, 60A, BMP-3, COP-1, COP-3, COP-4, COP-5, COP-7, COP-16 besteht; oder das Morphogen ein dimeres Protein umfaßt, das eine gewebsspezifische Morphogenese bei einem Säugetier induziert, wobei das dimere Protein zwei gefaltete Polypeptide umfaßt, und wobei die Aminosäure-Sequenz von jedem die C-terminale Sieben-Cystein-Domäne von menschlichem OP-1 (Reste 38-139 von Seq. ID Nr. 5) umfaßt.

20. Lösung nach Anspruch 19, die weiterhin einen Zucker, ein Antikoagulanz oder ein Mittel zur Hemmung freier Sauerstoff-Radikale umfaßt; oder weiterhin zur Aufrechterhaltung von im wesentlichen normalen ATP-Spiegeln in diesen Zellen, Gewebe oder Organ formuliert ist.

21. Zusammensetzung nach Anspruch 18 oder die Lösung nach Anspruch 19 oder 20, bei der das Morphogen wie in Anspruch 1 definiert ist.

22. Ex-vivo-Verfahren zum Schutz eines lebenden Säugetiergewebes oder transplantierten Säugetierorganes vor einer Schädigung, die mit der Entzündungareaktion von aktivierten Immuneffektorzellen verbunden ist, das den Schritt umfaßt, dem Gewebe oder Organ ein Morphogen, das aus der Gruppe ausgewählt ist, die aus OP-1, OP-2, BMP-5, BMP-6, BMP-2, BMP-4, Dpp, Vgl, Vgr-1, GDF-1, 60A, BMP-3, COP-1, COP-3, COP-4, COP-5, COP-7, COP-16 besteht; oder ein Morphogen zu verabreichen, das ein dimeres Protein umfaßt, das eine gewebsspezifische Morphogenese bei einem Säugetier induziert, wobei das dimere Protein zwei gefaltete Polypeptide umfaßt, und wobei die Aminosäure-Sequenz von jedem die C-terminale Sieben-Cystein-Domäne von menschlichem OP-1 (Reste 38-139 von Seq. ID Nr. 5) umfaßt.

23. Verfahren nach Anspruch 24, bei dem:
(a) das Gewebe oder Organ wie in Anspruch 15 definiert ist; und/oder
(b) das Morphogen wie in Anspruch 1 definiert ist; und/oder
(c) das Morphogen in der Form einer Lösung wie in Anspruch 19 oder 20 definiert verabreicht wird.

## Revendications

1. Utilisation d'un morphogène choisi dans le groupe consistant en OP-1, OP-2, BMP-5, BMP-6, BMP-2, BMP-4, Dpp, Vgl, Vgr-1, GDF-1, 60A, BMP-3, COP-1, COP-3, COP-4, COP-5, COP-7, COP-16 ou un morphogène comprenant une protéine dimère qui induit la morphogenèse tissu-spécifique chez un mammifère (par exemple un humain) et comprend une paire de polypeptides repliés, la séquence des acides aminés de chacun de ceux-ci comprenant:
(a) la séquence du domaine à sept cystéines de l'OP-1 humaine, OP-1 de souris, OP-2 humaine, OP-2 ou 60A de souris.
(b) une séquence d'acides aminés choisie parmi la séquence ID Nos. 9, 10, 11, 12, 13, 14, 26, 27, 28 ou 29 (CBMP2A(fx), CBMP2B(fx), DPP(fx), Vgl(fx), Vgr-1(fx), GDF-1(fx), BMP3, BMP5, BMP6 ou OPX); ou
(c) une séquence des acides aminés des résidus 38-139 de la séquence ID No. 5, 6, 7 ou 8 ou les résidus 354-455 de la séquence ID No. 24,
pour la fabrication d'un médicament pour:
(a) moduler une réponse inflammatoire; ou
(b) ralentir les effets de détérioration tissulaire associés à la réponse inflammatoire provoquée par une lésion tissulaire; ou
(c) accroître la viabilité des tissus lésés ou endommagés de mammifères (par exemple un tissu placé in vivo dans un mammifère).

2. Utilisation selon la revendication 1 pour moduler une réponse inflammatoire dans un tissu de mammifère, dans laquelle la modulation de cette réponse inflammatoire comprend:
(a) Le ralentissement de la formation des tissus fibrosés ou cicatriciels; et/ou
(b) l'inhibition de l'adhérence des cellules effectrices immunes sur l'endothélium vasculaire; et/ou
(c) le ralentissement d'une inflammation interstitielle; et/ou
(d) le ralentissement d'un oedème ou érythème; et/ou
(e) le ralentissement d'une nécrose des tissus inflammatoires.

3. Utilisation selon la revendication 1 ou la revendication 2 d'après laquelle la réponse inflammatoire est associée à une lésion tissulaire (la lésion étant par exemple liée à des causes chimiques, mécaniques, biologiques ou immunologiques).

4. Utilisation selon l'une quelconque des revendications précédentes dans laquelle ledit médicament est administré : (a) pour le traitement ou la prophylaxie de ladite lésion tissulaire; et/ou (b) en une quantité efficace pour inhiber la fibrogénèse ou stimuler la régénération tissu-spécifique de ce tissu lésé ou endommagé; et/ou (c) pour inhiber la perte ou la diminution de la fonction dudit tissu ;et/ou (d) par voie orale, parentérale ou topique; et/ou (e) dispersé dans un adhésif liquide tissulaire; et/ou (f) dans un aérosol.

5. Utilisation selon la revendication 3 ou 4 dans laquelle la dite lésion tissulaire comporte:
(a) une lésion ischémique par reperfusion; ou
(b) une lésion par hyperoxie.

6. Utilisation selon la revendication 5(a) dans laquelle la dite lésion ischémique par reperfusion est:
(a) associée à un arrêt cardiaque, une occlusion pulmonaire, une occlusion artérielle (par exemple d'une artère rénale), une occlusion d'une artère coronaire ou une attaque à caractère occlusif; ou
(b) associée à la chirurgie ou à la diminution ou à l'interruption de la circulation du sang vers un organe dans un traitement clinique (par exemple entérectomie de la carotoïde, un pontage d'une artère coronaire, un procédé de greffage d'un tissu, une transplantation d'organe ou une thérapie fibrinolytique); ou
(c) associée à un infarctus cérébral, un infarctus du myocarde, une asphyxie ou un arrêt cardio-pulmonaire.

7. Utilisation selon la revendication 5(b) dans laquelle la lésion à caractère hyperoxie est associée au traitement:
(a) d'un nouveau-né prématuré; ou
(b) d'un emphysème; ou
(c) d'une asphyxie.

8. Utilisation selon l'une quelconque des revendications précédentes d'après laquelle le tissu est un tissu pulmonaire, cardiaque, hépatique, neural, pancréatique, synovial, de la peau ou rénal, par exemple dans lequel:
(a) le tissu est un tissu rénal et le mammifère souffre d'une néphrite glomérulaire, d'athérosclérose ou d'un diabète, ou
(b) le tissu est un tissu cardiaque et le mammifère souffre d'une athérosclérose ou d'une maladie vasculaire, ou
(c) le tissu est un tissu pancréatique et le mammifère souffre du diabète, ou
(d) le tissu est un tissu synovial et le mammifère souffre d'arthrite,
(e) le tissu est un tissu de la peau et le mammifère souffre d'une dermatite ou d'un psoriasis,
(f) le tissu est un tissu du poumon et le mammifère souffre d'une bronchite, d'emphysème, de fibrose pulmonaire protopathique, d'asthme, d'asphyxie, ou du syndrome d'une douleur respiratoire de l'adulte.

9. Utilisation selon la revendication 3 ou 4 dans laquelle la lésion tissulaire comporte :
(a) une réponse inflammatoire à médiation de cellules immunitaires;
(b) une maladie inflammatoire ( par exemple une maladie inflammatoire chronique); ou
(c) une réponse immunitaire anormale; ou
(d) un oedème ou un érythème.

10. Utilisation selon la revendication 9(a), 9(c), ou 9(d) dans laquelle la lésion tissulaire comporte:
(a) une réponse inflammatoire aiguë généralisée; ou
(b) une inflammation des voies aériennes.

11. Utilisation selon la revendication 9(b) d'après laquelle la maladie inflammatoire comporte :
(a) une maladie auto-immune; ou
(b) l'arthrite, un psoriasis, une dermatite, une maladie d'inflammation des intestins ou un diabète.

12. Utilisation selon la revendication 10 dans laquelle:
(a) dans la revendication 10(a) la réponse inflammatoire aiguë est associée à une asphyxie ou à un syndrome d'une douleur respiratoire de l'adulte; ou
(b) dans la revendication 10(b) l'inflammation de la voie aérienne est associée à une bronchite chronique, un emphysème, une fibrose pulmonaire protopathique ou à de l'asthme.

13. Utilisation selon la revendication 11 dans laquelle:
(a) dans la revendication 11(a) la maladie auto-immune est une maladie neuro-dégénérative (par exemple une sclérose multiple ou une sclérose latérale amyotrophique); ou
(b) dans la revendication 11(b) l'arthrite est à caractère rhumatismal, dégénératif ou arthrite psoriasique.

14. Utilisation selon l'une quelconque des revendications 3-13 dans laquelle la modulation de la réponse inflammatoire est effectuée par l'administration d'un médicament avant ou après la lésion, par exemple avant ou après la réduction ou l'interruption de la circulation du sang ou après la reprise de la circulation du sang lorsque la lésion est une lésion ischémique par reperfusion.

15. Utilisation selon la revendication 6(b) dans laquelle la dite procédure clinique est un procédé de greffage d'un tissu (par exemple de peau, de moelle osseuse ou d'un tissu de la muqueuse gastro-intestinale) ou un procédé de transplantation d'un organe (par exemple d'un poumon, d'un coeur, d'un rein, d'un foie ou d'un pancréas).

16. Utilisation selon la revendication 15 d'après laquelle la modulation de la réponse inflammatoire est effectuée par l'administration du médicament:
(a) dès ou après l'élimination de l'organe ou du tissu provenant d'un hôte donneur; ou
(b) pendant la conservation ou le transport de l'organe ou du tissu avant l'implantation chez un hôte receveur; ou
(c) dès ou après l'implantation chez l'hôte receveur.

17. Utilisation selon l'une quelconque des revendications précédentes d'après laquelle ledit médicament comprend le morphogène dispersé dans un véhicule aqueux approprié physiologiquement acceptable pour une administration par voie parentérale à un mammifère.

18. Une composition pharmaceutique pour l'utilisation dans le ralentissement des lésions associées à une exposition d'un tissu d'un mammifère à des concentrations toxiques d'oxygène, comprenant un morphogène dispersé dans un véhicule aqueux physiologiquement acceptable à une concentration efficace pour ralentir l'extravasation des cellules effectrices immunes, un inflammation interstitielle, un oedème, une nécrose ou fibrogenèses dans un tissu de mammifère à risque, ou ayant souffert de, cette lésion, ledit morphogène choisi dans le groupe consistant en OP-1, OP-2, BMP-5, BMP-6, BMP-2, BMP-4, Dpp, Vgl, Vgr-1, GDF-1, 60A, BMP-3, COP-1, COP-3, COP4, COP-5, COP-7, COP-16 ou ce morphogène comprenant une protéine dimère qui induit une morphogenèse, tissu-spécifique chez un mammifère, cette protéine dimère comprenant une paire de polypeptides repliés, la séquence des acides aminés de chacune de celles-ci comprenant le domaine à sept cystéines du C-terminal de l'OP-1 humaine (résidus 38-139 de la séquence ID No.5), la dite composition comprenant en outre un agent inhibant ou un anticoagulant à radical oxygène libre.

19. Une solution de conservation pour maintenir la viabilité ex vivo de cellules ou d'un tissu d'un mammifère ou d'un organe d'un mammifère, comprenant une formulation liquide possédant une pression osmotique essentiellement équivalente à la pression osmotique des cellules vivantes de mammifère et un morphogène dispersé dans ce liquide à une concentration efficace pour ralentir l'extravasation des cellules effectrices immunes, une inflammation interstitielle, un oedème, une nécrose ou fibrogenèse dans ces cellules , tissu ou organe, ce morphogène comprenant choisi dans le groupe consistant en OP-1, OP-2, BMP-5, BMP-6, BMP-2, BMP-4, Dpp, Vgl, Vgr-1, GDF-1, 60A, BMP-3, COP-1, COP-3, COP-4, COP-5, COP-7, COP-16 ou ce morphogène une protéine dimère qui induit une morphogenèse tissu-spécifique chez un mammifère, ladite protéine dimère comprenant une paire de polypeptides repliés, la séquence des acides aminés de chacune de celles-ci comprend la séquence des acides aminés avec le C-terminal du domaine à sept cystéine de l'OP-1 humaine (résidus 38-139 de la séquence ID No.5).

20. Une solution selon la revendication 19, comprenant en outre un sucre, un anticoagulant ou un agent inhibant à radical oxygène libre; ou, en outre formulé pour essentiellement maintenir les niveaux normaux d'ATP dans lesdites cellules, tissu ou organe.

21. Une composition selon la revendication 18 ou la solution selon la revendication 19 ou 20 dans laquelle le morphogène est tel que défini dans la revendication 1.

22. Une méthode ex vivo pour protéger un tissu vivant de mammifère ou un organe transplanté de mammifère contre des lésions associées à une réponse inflammatoire de cellules effectrices immunes activées comprenant l'étape de fournir au dit tissus ou organe un morphogène choisi dans le groupe consistant en OP-1, OP-2, BMP-5, BMP-6, BMP-2, BMP-4, Dpp, Vgl, Vgr-1, GDF-1, 60A, BMP-3, COP-1, COP-3, COP-4, COP-5, COP-7, COP-16 ou ce morphogène comprenant une protéine dimère qui induit une morphogenèse tissu-spécifique chez un mammifère, la dite protéine dimère comprenant une paire de polypeptides repliés, la séquence des acides aminés de chacune de celle-ci comprend la séquence des acides aminés avec le domaine à sept cystéines du C-terminal de l'OP-1 humaine (résidus 38-139 de la séquence ID No.5).

23. La méthode selon la revendication 22 dans laquelle:
(a) le tissu ou organe est tel que défini dans la revendication 15; et/ou
(b) le morphogène est tel que défini dans la revendication 1;
(c) le morphogène est fourni sous forme d'une solution telle que définie dans la revendication 19 ou 20.


### SEQUENCE LISTING

(1) GENERAL INFORMATION:
   (i)APPLICANT:
      KUBERASAMPATH, THANGAVEL
      PANG, ROY H.L.
      OPPERMANN, HERMANN
      RUEGER, DAVID C.
      COHEN, CHARLES M.
      OZKAYNAK, ENGIN
      SMART, JOHN
   (ii) TITLE OF INVENTION: MORPHOGEN-INDUCED MODULATION OF INFLAMMATORY RESPONSE
   (iii) NUMBER OF SEQUENCES: 33
   (iv) CORRESPONDENCE ADDRESS:
      (A) ADDRESSEE: CREATIVE BIOMOLECULES
      (B) STREET: 35 SOUTH STREET
      (C) CITY: HOPKINTON
      (D) STATE: MASSACHUSETTS
      (E) COUNTRY: U.S.A.
      (F) ZIP:
   (v) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: Patent In Release #1.0, Version #1.25
   (vii) PRIOR APPLICATION DATA:
      (A) APPLICATION NUMBER: US 667,274
      (B) FILING DATE: 11-MAR-1991
   (vii) PRIOR APPLICATION DATA:
      (A) APPLICATION NUMBER: US 753,059
      (B) FILING DATE: 30-AUG-1991
   (vii) PRIOR APPLICATION DATA:
      (A) APPLICATION NUMBER: US 752.764
      (B) FILING DATE: 30-AUG-1991
(2) INFORMATION FOR SEQ ID NO:1:
   (i) SEQUENCE CHARACTERISTICS:
      (A) LENGTH: 97 amino acids
      (B) TYPE: amino acids
      (C) TOPOLOGY: linear
   (ii) MOLECULE TYPE: protein
   (ix) FEATURE:
      (A) NAME: Generic Sequence 1
      (D) OTHER INFORMATION: Each Xaa indicates one of the 20 naturally-occurring L-isomer, α-amino acids or a derivative thereof.
   (xi) SEQUENCE DESCRIPTION: SEQ ID NO:1:
   Xaa Xaa Xaa Xaa Xaa Xaa
    1               5
   Xaa Xaa Xaa Xaa Xaa Xaa Xaa Xaa Xaa Xaa Xaa
                10                  15
   Xaa Xaa Xaa Xaa Xaa Xaa Xaa Cys Xaa Xaa Xaa
            20                  25
   Cys Xaa Xaa Xaa Xaa Xaa Xaa Xaa Xaa Xaa Xaa
        30                  35
   Xaa Xaa Xaa Xaa Xaa Xaa Xaa Xaa Xaa Xaa Xaa
   40                  45                  50
   Xaa Xaa Xaa Xaa Xaa Xaa Xaa Xaa Xaa Xaa Cys
                    55                  60
   Cys Xaa Xaa Xaa Xaa Xaa Xaa Xaa Xaa Xaa Xaa
                65                  70
   Xaa Xaa Xaa Xaa Xaa Xaa Xaa Xaa Xaa Xaa Xaa
            75                  80
   Xaa Xaa Xaa Xaa Xaa Xaa Xaa Xaa Xaa Xaa Cys
        85                  90
   Xaa Cys Xaa
    95
(2) INFORMATION FOR SEQ ID NO:2:
   (i) SEQUENCE CHARACTERISTICS:
      (A) LENGTH: 97 amino acids
      (B) TYPE: amino acids
      (C) TOPOLOGY: linear
   (ii) MOLECULE TYPE: protein
   (ix) FEATURE:
      (A) NAME: Generic Sequence 2
      (D) OTHER INFORMATION: Each Xaa indicates one of the 20 naturally-occurring L-isomer, α-amino acids or a derivative thereof.
   (xi) SEQUENCE DESCRIPTION: SEQ ID NO:2:
   Xaa Xaa Xaa Xaa Xaa Xaa
    1               5
   Xaa Xaa Xaa Xaa Xaa Xaa Xaa Xaa Xaa Xaa Xaa
                10                  15
   Xaa Xaa Xaa Xaa Xaa Xaa Xaa Cys Xaa Xaa Xaa
            20                  25
   Cys Xaa Xaa Xaa Xaa Xaa Xaa Cys Xaa Xaa Xaa
        30                  35
   Xaa Xaa Xaa Xaa Xaa Xaa Xaa Xaa Xaa Xaa Xaa
   40                  45                  50
   Xaa Xaa Xaa Xaa Xaa Xaa Xaa Xaa Xaa Xaa Cys
                   55                  60
   Cys Xaa Xaa Xaa Xaa Xaa Xaa Xaa Xaa Xaa Xaa
               65                  70
   Xaa Xaa Xaa Xaa Xaa Xaa Xaa Xaa Xaa Xaa Xaa
           75                  80
   Xaa Xaa Xaa Xaa Xaa Xaa Xaa Xaa Xaa Xaa Cys
       85                  90
   Xaa Cys Xaa
   95
(2) INFORMATION FOR SEQ ID NO:3:
   (i) SEQUENCE CHARACTERISTICS:
      (A) LENGTH: 97 amino acids
      (B) TYPE: amino acids
      (C) TOPOLOGY: linear
   (ii) MOLECULE TYPE: protein
   (ix) FEATURE:
      (A) NAME: Generic Sequence 3
      (D) OTHER INFORMATION: wherein each Xaa is independently selected from a group of one or more specified amino acids as defined in the specification.
   (xi) SEQUENCE DESCRIPTION: SEQ ID NO:3:
   Leu Tyr Val Xaa Phe
   1               5
   Xaa Xaa Xaa Gly Trp Xaa Xaa Trp Xaa
                   10
   Xaa Ala Pro Gly Xaa Xaa Xaa Ala
   15                  20
   Xaa Tyr Cys Xaa Gly Xaa Cys Xaa
           25                  30
   Xaa Pro Xaa Xaa Xaa Xaa Xaa
                   35
   Xaa Xaa Xaa Asn His Ala Xaa Xaa
           40                  45
   Xaa Xaa Leu Xaa Xaa Xaa Xaa Xaa
                   50
   Xaa Xaa Xaa Xaa Xaa Xaa Xaa Cys
       55                  60
   Cys Xaa Pro Xaa Xaa Xaa Xaa Xaa
               65
   Xaa Xaa Xaa Leu Xaa Xaa Xaa
   70                  75
   Xaa Xaa Xaa Xaa Val Xaa Leu Xaa
               80
   Xaa Xaa Xaa Xaa Met Xaa Val Xaa
   85                  90
   Xaa Cys Gly Cys Xaa
           95
(2) INFORMATION FOR SEQ ID NO:4:
   (i) SEQUENCE CHARACTERISTICS:
      (A) LENGTH: 102 amino acids
      (B) TYPE: amino acids
      (C) TOPOLOGY: linear
   (ii) MOLECULE TYPE: protein
   (ix) FEATURE:
      (A) NAME: Generic Sequence 4
      (D) OTHER INFORMATION: wherein each Xaa is independently selected from a group of one or more specified amino acids as defined in the specification.
   (xi) SEQUENCE DESCRIPTION: SEQ ID NO:4:
   Cys Xaa Xaa Xaa Xaa Leu Tyr Val Xaa Phe
    1               5                   10
   Xaa Xaa Xaa Gly Trp Xaa Xaa Trp Xaa
                    15
   Xaa Ala Pro Xaa Gly Xaa Xaa Ala
    20                  25
   Xaa Tyr Cys Xaa Gly Xaa Cys Xaa
            30                  35
   Xaa Pro Xaa Xaa Xaa Xaa Xaa
                    40
   Asn Xaa Xaa Asn His Ala Xaa Xaa
            45                  50
   Xaa Xaa Leu Xaa Xaa Xaa Xaa Xaa
                    55
   Xaa Xaa Xaa Xaa Xaa Xaa Xaa Cys
        60                  65
   Cys Xaa Pro Xaa Xaa Xaa Xaa Xaa
                70
   Xaa Xaa Xaa Leu Xaa Xaa Xaa
    75                  80
   Xaa Xaa Xaa Xaa Val Xaa Leu Xaa
                85
   Xaa Xaa Xaa Xaa Met Xaa Val Xaa
    90                  95
   Xaa Cys Gly Cys Xaa
           100
(2) INFORMATION FOR SEQ ID NO:5:
   (i) SEQUENCE CHARACTERISTICS:
      (A) LENGTH: 139 amino acids
      (B) TYPE: amino acids
      (C) TOPOLOGY: linear
   (ii) MOLECULE TYPE: protein
   (ix) FEATURE:
      (A) NAME: hOP-1 (mature form)
   (xi) SEQUENCE DESCRIPTION: SEQ ID NO:5:
   Ser Thr Gly Ser Lys Gln Arg Ser Gln
   1               5
   Asn Arg Ser Lys Thr Pro Lys Asn Gln
   10                  15
   Glu Ala Leu Arg Met Ala Asn Val Ala
       20                  25
   Glu Asn Ser Ser Ser Asp Gln Arg Gln
           30                  35
   Ala Cys Lys Lys His Glu Leu Tyr Val
               40                  45
   Ser Phe Arg Asp Leu Gly Trp Gln Asp
                   50
   Trp Ile Ile Ala Pro Glu Gly Tyr Ala
   55                  60
   Ala Tyr Tyr Cys Glu Gly Glu Cys Ala
       65                  70
   Phe Pro Leu Asn Ser Tyr Met Asn Ala
           75                  80
   Thr Asn His Ala Ile Val Gln Thr Leu
               85                  90
   Val His Phe Ile Asn Pro Glu Thr Val
                   95
   Pro Lys Pro Cys Cys Ala Pro Thr Gln
   100                 105
   Leu Asn Ala Ile Ser Val Leu Tyr Phe
       110                 115
   Asp Asp Ser Ser Asn Val Ile Leu Lys
           120                 125
   Lys Tyr Arg Asn Met Val Val Arg Ala
               130                 135
   Cys Gly Cys His
(2) INFORMATION FOR SEQ ID NO:6:
   (i) SEQUENCE CHARACTERISTICS:
      (A) LENGTH: 139 amino acids
      (B) TYPE: amino acids
      (C) TOPOLOGY: linear
   (ii) MOLECULE TYPE: protein
   (ix) FEATURE:
      (A) NAME: mOP-1 (mature form)
   (xi) SEQUENCE DESCRIPTION: SEQ ID NO:6:
   Ser Thr Gly Gly Lys Gln Arg Ser Gln
   1               5
   Asn Arg Ser Lys Thr Pro Lys Asn Gln
   10                  15
   Glu Ala Leu Arg Met Ala Ser Val Ala
       20                  25
   Glu Asn Ser Ser Ser Asp Gln Arg Gln
           30                  35
   Ala Cys Lys Lys His Glu Leu Tyr Val
               40                  45
   Ser Phe Arg Asp Leu Gly Trp Gln Asp
                   50
   Trp Ile Ile Ala Pro Glu Gly Tyr Ala
    55                 60
   Ala Tyr Tyr Cys Glu Gly Glu Cys Ala
        65                  70
   Phe Pro Leu Asn Ser Tyr Met Asn Ala
            75                  80
   Thr Asn His Ala Ile Val Gln Thr Leu
                85                  90
   Val His Phe Ile Asn Pro Asp Thr Val
                    95
   Pro Lys Pro Cys Cys Ala Pro Thr Gln
   100                 105
   Leu Asn Ala Ile Ser Val Leu Tyr Phe
       110                 115
   Asp Asp Ser Ser Asn Val Ile Leu Lys
           120                 125
   Lys Tyr Arg Asn Met Val Val Arg Ala
               130                 135
   Cys Gly Cys His
(2) INFORMATION FOR SEQ ID NO:7:
   (i) SEQUENCE CHARACTERISTICS:
      (A) LENGTH: 139 amino acids
      (B) TYPE: amino acids
      (C) TOPOLOGY: linear
   (ii) MOLECULE TYPE: protein
   (ix) FEATURE:
      (A) NAME: hOP-2 (mature form)
   (xi) SEQUENCE DESCRIPTION: SEQ ID NO:7:
   Ala Val Arg Pro Leu Arg Arg Arg Gln
   1               5
   Pro Lys Lys Ser Asn Glu Leu Pro Gln
   10                  15
   Ala Asn Arg Leu Pro Gly Ile Phe Asp
       20                  25
   Asp Val His Gly Ser His Gly Arg Gln
           30                  35
   Val Cys Arg Arg His Glu Leu Tyr Val
               40                  45
   Ser Phe Gln Asp Leu Gly Trp Leu Asp
                   50
   Trp Val Ile Ala Pro Gln Gly Tyr Ser
   55                  60
   Ala Tyr Tyr Cys Glu Gly Glu Cys Ser
       65                  70
   Phe Pro Leu Asp Ser Cys Met Asn Ala
           75                  80
   Thr Asn His Ala Ile Leu Gln Ser Leu
               85                  90
   Val His Leu Met Lys Pro Asn Ala Val
                   95
   Pro Lys Ala Cys Cys Ala Pro Thr Lys
   100                 105
   Leu Ser Ala Thr Ser Val Leu Tyr Tyr
       110                 115
   Asp Ser Ser Asn Asn Val Ile Leu Arg
           120                 125
   Lys His Arg Asn Met Val Val Lys Ala
               130                 135
   Cys Gly Cys His
(2) INFORMATION FOR SEQ ID NO:8:
   (i) SEQUENCE CHARACTERISTICS:
      (A) LENGTH: 139 amino acids
      (B) TYPE: amino acids
      (C) TOPOLOGY: linear
   (ii) MOLECULE TYPE: protein
   (ix) FEATURE:
      (A) NAME: mOP-2 (mature form)
   (xi) SEQUENCE DESCRIPTION: SEQ ID NO:8:
   Ala Ala Arg Pro Leu Lys Arg Arg Gln
   1               5
   Pro Lys Lys Thr Asn Glu Leu Pro His
   10                  15
   Pro Asn Lys Leu Pro Gly Ile Phe Asp
       20                  25
   Asp Gly His Gly Ser Arg Gly Arg Glu
           30                  35
   Val Cys Arg Arg His Glu Leu Tyr Val
               40                  45
   Ser Phe Arg Asp Leu Gly Trp Leu Asp
                   50
   Trp Val Ile Ala Pro Gln Gly Tyr Ser
   55                  60
   Ala Tyr Tyr Cys Glu Gly Glu Cys Ala
       65                  70
   Phe Pro Leu Asp Ser Cys Met Asn Ala
           75                  80
   Thr Asn His Ala Ile Leu Gln Ser Leu
               85                  90
   Val His Leu Met Lys Pro Asp Val Val
                   95
   Pro Lys Ala Cys Cys Ala Pro Thr Lys
   100                 105
   Leu Ser Ala Thr Ser Val Leu Tyr Tyr
       110                 115
   Asp Ser Ser Asn Asn Val Ile Leu Arg
           120                 125
   Lys His Arg Asn Met Val Val Lys Ala
               130                 135
   Cys Gly Cys His
(2) INFORMATION FOR SEQ ID NO:9:
   (i) SEQUENCE CHARACTERISTICS:
      (A) LENGTH: 96 amino acids
      (B) TYPE: amino acids
      (C) TOPOLOGY: linear
   (ii) MOLECULE TYPE: protein
   (ix) FEATURE:
      (A) NAME: CBMP-2A(fx)
   (xi) SEQUENCE DESCRIPTION: SEQ ID NO:9:
(2) INFORMATION FOR SEQ ID NO:10:
   (i) SEQUENCE CHARACTERISTICS:
      (A) LENGTH: 101 amino acids
      (B) TYPE: amino acids
      (C) TOPOLOGY: linear
   (ii) MOLECULE TYPE: protein
   (ix) FEATURE:
      (A) NAME: CBMP-2B(fx)
   (xi) SEQUENCE DESCRIPTION: SEQ ID NO:10:
   Cys Arg Arg His Ser
   1               5
   Leu Tyr Val Asp Phe Ser Asp Val Gly Trp Asn
                   10                  15
   Asp Trp Ile Val Ala Pro Pro Gly Tyr Gln Ala
               20                  25
   Phe Tyr Cys His Gly Asp Cys Pro Phe Pro Leu
   30 35
   Ala Asp His Leu Asn Ser Thr Asn His Ala Ile
   40 45
   Val Gln Thr Leu Val Asn Ser Val Asn Ser Ser
   50 55 60
   Ile Pro Lys Ala Cys Cys Val Pro Thr Glu Leu
   65 70
   Ser Ala Ile Ser Met Leu Tyr Leu Asp Glu Tyr
   75 80
   Asp Lys Val Val Leu Lys Asn Tyr Gln Glu Met
   85 90
   Val Val Glu Gly Cys Gly Cys Arg
   95 100
(2) INFORMATION FOR SEQ ID NO:11:
   (i) SEQUENCE CHARACTERISTICS:
      (A) LENGTH: 102 amino acids
      (B) TYPE: amino acids
      (C) TOPOLOGY: linear
   (ii) MOLECULE TYPE: protein
   (ix) FEATURE:
      (A) NAME: DPP(fx)
   (xi) SEQUENCE DESCRIPTION: SEQ ID NO:11:
   Cys Arg Arg His Ser Leu Tyr Val Asp Phe Ser
   1 5 10
   Asp Val Gly Trp Asp Asp Trp Ile Val Ala Pro
   15 20
   Leu Gly Tyr Asp Ala Tyr Tyr Cys His Gly Lys
   25 30
   Cys Pro Phe Pro Leu Ala Asp His Phe Asn Ser
   35 40
   Thr Asn His Ala Val Val Gln Thr Leu Val Asn
   45 50 55
   Asn Asn Asn Pro Gly Lys Val Pro Lys Ala Cys
   60 65
   Cys Val Pro Thr Gln Leu Asp Ser Val Ala Met
   70 75
   Leu Tyr Leu Asn Asp Gln Ser Thr Val Val Leu
   80 85
   Lys Asn Tyr Gln Glu Met Thr Val Val Gly Cys
   90 95
   Gly Cys Arg
   100
(2) INFORMATION FOR SEQ ID NO:12:
   (i) SEQUENCE CHARACTERISTICS:
      (A) LENGTH: 102 amino acids
      (B) TYPE: amino acids
      (C) TOPOLOGY: linear
   (ii) MOLECULE TYPE: protein
   (ix) FEATURE:
      (A) NAME: Vgl(fx)
   (xi) SEQUENCE DESCRIPTION: SEQ ID NO:12:
   Cys Lys Lys Arg His Leu Tyr Val Glu Phe Lys
    1               5                  10
   Asp Val Gly Trp Gln Asn Trp Val Ile Ala Pro
                15                 20
   Gln Gly Tyr Met Ala Asn Tyr Cys Tyr Gly Glu
            25                 30
   Cys Pro Tyr Pro Leu Thr Glu Ile Leu Asn Gly
        35                 40
   Ser Asn His Ala Ile Leu Gln Thr Leu Val His
    45                 50                  55
   Ser Ile Glu Pro Glu Asp Ile Pro Leu Pro Cys
                    60                 65
   Cys Val Pro Thr Lys Met Ser Pro Ile Ser Met
                70                 75
   Leu Phe Tyr Asp Asn Asn Asp Asn Val Val Leu
            80                 85
   Arg His Tyr Glu Asn Met Ala Val Asp Glu Cys
        90                 95
   Gly Cys Arg
   100
(2) INFORMATION FOR SEQ ID NO:13:
   (i) SEQUENCE CHARACTERISTICS:
      (A) LENGTH: 102 amino acids
      (B) TYPE: amino acids
      (C) TOPOLOGY: linear
   (ii) MOLECULE TYPE: protein
   (ix) FEATURE:
      (A) NAME: Vgr-1(fx)
   (xi) SEQUENCE DESCRIPTION: SEQ ID NO:13:
   Cys Lys Lys His Glu Leu Tyr Val Ser Phe Gln
    1               5                  10
   Asp Val Gly Trp Gln Asp Trp Ile Ile Ala Pro
                15                  20
   Xaa Gly Tyr Ala Ala Asn Tyr Cys Asp Gly Glu
            25                  30
   Cys Ser Phe Pro Leu Asn Ala His Met Asn Ala
        35                  40
   Thr Asn His Ala Ile Val Gln Thr Leu Val His
   45                   50                  55
   Val Met Asn Pro Glu Tyr Val Pro Lys Pro Cys
                    60                  65
   Cys Ala Pro Thr Lys Val Asn Ala Ile Ser Val
                70                  75
   Leu Tyr Phe Asp Asp Asn Ser Asn Val Ile Leu
            80                  85
   Lys Lys Tyr Arg Asn Met Val Val Arg Ala Cys
        90                  95
   Gly Cys His
   100
(2) INFORMATION FOR SEQ ID NO:14:
   (i) SEQUENCE CHARACTERISTICS:
      (A) LENGTH: 106 amino acids
      (B) TYPE: protein
      (C) STRANDEDNESS: single
      (D) TOPOLOGY: linear
   (ii) MOLECULE TYPE: protein
   (vi) ORIGINAL SOURCE:
      (A) ORGANISM: human
      (F) TISSUE TYPE: BRAIN
   (ix) FEATURE:
      (D) OTHER INFORMATION: /product= "GDF-1 (fx)"
   (xi) SEQUENCE DESCRIPTION: SEQ ID NO:14:
   Cys Arg Ala Arg Arg Leu Tyr Val Ser Phe Arg Glu Val Gly
    1               5                   10
   Trp His Arg Trp Val Ile Ala Pro Arg Gly Phe Leu Ala Asn Tyr
   15                  20                  25
   Cys Gln Gly Gln Cys Ala Leu Pro Val Ala Leu Ser Gly Ser Gly
   30                  35                  40
   Gly Pro Pro Ala Leu Asn His Ala Val Leu Arg Ala Leu Met His
   45                  50                  55
   Ala Ala Ala Pro Gly Ala Ala Asp Leu Pro Cys Cys Val Pro Ala
   60                  65                  70
   Arg Leu Ser Pro Ile Ser Val Leu Phe Phe Asp Asn Ser Asp Asn
   75                  80                  85
   Val Val Leu Arg Gln Tyr Glu Asp Met Val Val Asp Glu Cys Gly
   90                  95                  100
   Cys Arg
   105
(2) INFORMATION FOR SEQ ID NO:15:
   (i) SEQUENCE CHARACTERISTICS:
      (A) LENGTH: 5 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:15:
   Cys Xaa Xaa Xaa Xaa
    1               5
(2) INFORMATION FOR SEQ ID NO:16:
   (i) SEQUENCE CHARACTERISTICS:
      (A) LENGTH: 1822 base pairs
      (B) TYPE: nucleic acid
      (C) STRANDEDNESS: single
      (D) TOPOLOGY: linear
   (ii) MOLECULE TYPE: cDNA
   (vi) ORIGINAL SOURCE:
      (A) ORGANISM: HOMO SAPIENS
      (F) TISSUE TYPE: HIPPOCAMPUS
   (ix) FEATURE:
      (A) NAME/KEY: CDS
      (B) LOCATION: 49..1341
      (D) OTHER INFORMATION:/standard_name= "hOP1"
   (xi) SEQUENCE DESCRIPTION: SEQ ID NO:16:
   GGTGCGGGCC CGGAGCCCGG AGCCCGGGTA GCGCGTAGAG CCGGCGCG ATG CAC GTG      57
                                                        Met His Val
                                                         1
   CGC TCA CTG CGA GCT GCG GCG CCG CAC AGC TTC GTG GCG CTC TGG GCA      105
   Arg Ser Leu Arg Ala Ala Ala Pro His Ser Phe Val Ala Leu Trp Ala
        5                  10                  15
   CCC CTG TTC CTG CTG CGC TCC GCC CTG GCC GAC TTC AGC CTG GAC AAC      153
   Pro Leu Phe Leu Leu Arg Ser Ala Leu Ala Asp Phe Ser Leu Asp Asn
    20                  25                  30                  35
   GAG GTG CAC TCG AGC TTC ATC CAC CGG CGC CTC CGC AGC CAG GAG CGG      201
   Glu Val His Ser Ser Phe Ile His Arg Arg Leu Arg Ser Gln Glu Arg
                    40                  45                  50
   CGG GAG ATG CAG CGC GAG ATC CTC TCC ATT TTG GGC TTG CCC CAC CGC         249
   Arg Glu Met Gln Arg Glu Ile Leu Ser Ile Leu Gly Leu Pro His Arg
               55                  60                  65
   CCG CGC CCG CAC CTC CAG GGC AAG CAC AAC TCG GCA CCC ATG TTC ATG         297
   Pro Arg Pro His Leu Gln Gly Lys His Asn Ser Ala Pro Met Phe Met
           70                  75                  80
   CTG GAC CTG TAC AAC GCC ATG GCG GTG GAG GAG GGC GGC GGG CCC GGC         345
   Leu Asp Leu Tyr Asn Ala Met Ala Val Glu Glu Gly Gly Gly Pro Gly
       85                  90                  95
   GGC CAG GGC TTC TCC TAC CCC TAC AAG GCC GTC TTC AGT ACC CAG GGC         393
   Gly Gln Gly Phe Ser Tyr Pro Tyr Lys Ala Val Phe Ser Thr Gln Gly
   100                 105                 110                 115
   CCC CCT CTG GCC AGC CTG CAA GAT AGC CAT TTC CTC ACC GAC GCC GAC         441
   Pro Pro Leu Ala Ser Leu Gln Asp Ser His Phe Leu Thr Asp Ala Asp
                   120                 125                 130
   ATG GTC ATG AGC TTC GTC AAC CTC GTG GAA CAT GAC AAG GAA TTC TTC         489
   Met Val Met Ser Phe Val Asn Leu Val Glu His Asp Lys Glu Phe Phe
               135                 140                 145
   CAC CCA CGC TAC CAC CAT CGA GAG TTC CGG TTT GAT CTT TCC AAG ATC         537
   His Pro Arg Tyr His His Arg Glu Phe Arg Phe Asp Leu Ser Lys Ile
           150                 155                 160
   CCA GAA GGG GAA GCT GTC ACG GCA GCC GAA TTC CGG ATC TAC AAG GAC         585
   Pro Glu Gly Glu Ala Val Thr Ala Ala Glu Phe Arg Ile Tyr Lys Asp
       165                 170                 175
   TAC ATC CGG GAA CGC TTC GAC AAT GAG ACG TTC CGG ATC AGC GTT TAT         633
   Tyr Ile Arg Glu Arg Phe Asp Asn Glu Thr Phe Arg Ile Ser Val Tyr
   180                 185                 190                 195
   CAG GTG CTC CAG GAG CAC TTG GGC AGG GAA TCG GAT CTC TTC CTG CTC         681
   Gln Val Leu Gln Glu His Leu Gly Arg Glu Ser Asp Leu Phe Leu Leu
                   200                 205                 210
   GAC AGC CGT ACC CTC TGG GCC TCG GAG GAG GGC TGG CTG GTG TTT GAC         729
   Asp Ser Arg Thr Leu Trp Ala Ser Glu Glu Gly Trp Leu Val Phe Asp
               215                 220                 225
   ATC ACA GCC ACC AGC AAC CAC TGG GTG GTC AAT CCG CGG CAC AAC CTG         777
   Ile Thr Ala Thr Ser Asn His Trp Val Val Asn Pro Arg His Asn Leu
           230                 235                 240
   GGC CTG CAG CTC TCG GTG GAG ACG CTG GAT GGG CAG AGC ATC AAC CCC         825
   Gly Leu Gln Leu Ser Val Glu Thr Leu Asp Gly Gln Ser Ile Asn Pro
       245                 250                 255
   AAG TTG GCG GGC CTG ATT GGG CGG CAC GGG CCC CAG AAC AAG CAG CCC      873
   Lys Leu Ala Gly Leu Ile Gly Arg His Gly Pro Gln Asn Lys Gln Pro
   260                 265                 270                 275
   TTC ATG GTG GCT TTC TTC AAG GCC ACG GAG GTC CAC TTC CGC AGC ATC      921
   Phe Met Val Ala Phe Phe Lys Ala Thr Glu Val His Phe Arg Ser Ile
                   280                 285                 290
   CGG TCC ACG GGG AGC AAA CAG CGC AGC CAG AAC CGC TCC AAG ACG CCC      969
   Arg Ser Thr Gly Ser Lys Gln Arg Ser Gln Asn Arg Ser Lys Thr Pro
               295                 300                 305
   AAG AAC CAG GAA GCC CTG CGG ATG GCC AAC GTG GCA GAG AAC AGC AGC     1017
   Lys Asn Gln Glu Ala Leu Arg Met Ala Asn Val Ala Glu Asn Ser Ser
           310                 315                 320
   AGC GAC CAG AGG CAG GCC TGT AAG AAG CAC GAG CTG TAT GTC AGC TTC     1065
   Ser Asp Gln Arg Gln Ala Cys Lys Lys His Glu Leu Tyr Val Ser Phe
       325                 330                 335
   CGA GAC CTG GGC TGG CAG GAC TGG ATC ATC GCG CCT GAA GGC TAC GCC     1113
   Arg Asp Leu Gly Trp Gln Asp Trp Ile Ile Ala Pro Glu Gly Tyr Ala
   340                 345                 350                 355
   GCC TAC TAC TGT GAG GGG GAG TGT GCC TTC CCT CTG AAC TCC TAC ATG     1161
   Ala Tyr Tyr Cys Glu Gly Glu Cys Ala Phe Pro Leu Asn Ser Tyr Met
                   360                 365                 370
   AAC GCC ACC AAC CAC GCC ATC GTG CAG ACG CTG GTC CAC TTC ATC AAC     1209
   Asn Ala Thr Asn His Ala Ile Val Gln Thr Leu Val His Phe Ile Asn
               375                 380                 385
   CCG GAA ACG GTG CCC AAG CCC TGC TGT GCG CCC ACG CAG CTC AAT GCC     1257
   Pro Glu Thr Val Pro Lys Pro Cys Cys Ala Pro Thr Gln Leu Asn Ala
           390                 395                 400
   ATC TCC GTC CTC TAC TTC GAT GAC AGC TCC AAC GTC ATC CTG AAG AAA     1305
   Ile Ser Val Leu Tyr Phe Asp Asp Ser Ser Asn Val Ile Leu Lys Lys
       405                 410                 415
   TAC AGA AAC ATG GTG GTC CGG GCC TGT GGC TGC CAC TAGCTCCTCC          1351
   Tyr Arg Asn Met Val Val Arg Ala Cys Gly Cys His
   420                 425                 430
   GAGAATTCAG ACCCTTTGGG GCCAAGTTTT TCTGGATCCT CCATTGCTCG CCTTGGCCAG   1411
   GAACCAGCAG ACCAACTGCC TTTTGTGAGA CCTTCCCCTC CCTATCCCCA ACTTTAAAGG   1471
   TGTGAGAGTA TTAGGAAACA TGAGCAGCAT ATGGCTTTTG ATCAGTTTTT CAGTGGCAGC   1531
   ATCCAATGAA CAAGATCCTA CAAGCTGTGC AGGCAAAACC TAGCAGGAAA AAAAAACAAC   1591
   GCATAAAGAA AAATGGCCGG GCCAGGTCAT TGGCTGGGAA GTCTCAGCCA TGCACGGACT 1651
   CGTTTCCAGA GGTAATTATG AGCGCCTACC AGCCAGGCCA CCCAGCCGTG GGAGGAAGGG 1711
   GGCGTGGCAA GGGGTGGGCA CATTGGTGTC TGTGCGAAAG GAAAATTGAC CCGGAAGTTC 1771
   CTGTAATAAA TGTCACAATA AAACGAATGA ATGAAAAAAA AAAAAAAAAA A 1822
(2) INFORMATION FOR SEQ ID NO:17:
   (i) SEQUENCE CHARACTERISTICS:
      (A) LENGTH: 431 amino acids
      (B) TYPE: amino acid
      (D) TOPOLOGY; linear
   (ii) MOLECULE TYPE: protein
   (ix) FEATURE:
      (D) OTHER INFORMATION: /Product="OP1-PP"
   (xi) SEQUENCE DESCRIPTION: SEQ ID No:17:
   Met His Val Arg Ser Leu Arg Ala Ala Ala Pro His Ser Phe Val Ala
   1 5 10 15
   Leu Trp Ala Pro Leu Phe Leu Leu Arg Ser Ala Leu Ala Asp Phe Ser
   20 25 30
   Leu Asp Asn Glu Val His Ser Ser Phe Ile His Arg Arg Leu Arg Ser
   35 40 45
   Gln Glu Arg Arg Glu Met Gln Arg Glu Ile Leu Ser Ile Leu Gly Leu
   50 55 60
   Pro His Arg Pro Arg Pro His Leu Gln Gly Lys His Asn Ser Ala Pro
   65 70 75 80
   Met Phe Met Leu Asp Leu Tyr Asn Ala Met Ala Val Glu Glu Gly Gly
   85 90 95
   Gly Pro Gly Gly Gln Gly Phe Ser Tyr Pro Tyr Lys Ala Val Phe Ser
   100 105 110
   Thr Gln Gly Pro Pro Leu Ala Ser Leu Gln Asp Ser His Phe Leu Thr
   115 120 125
   Asp Ala Asp Met Val Met Ser Phe Val Asn Leu Val Glu His Asp Lys
   130 135 140
   Glu Phe Phe His Pro Arg Tyr His His Arg Glu Phe Arg Phe Asp Leu
   145 150 155 160
   Ser Lys Ile Pro Glu Gly Glu Ala Val Thr Ala Ala Glu Phe Arg Ile
                   165                 170                 175
   Tyr Lys Asp Tyr Ile Arg Glu Arg Phe Asp Asn Glu Thr Phe Arg Ile
               180                 185                 190
   Ser Val Tyr Gln Val Leu Gln Glu His Leu Gly Arg Glu Ser Asp Leu
           195                 200                 205
   Phe Leu Leu Asp Ser Arg Thr Leu Trp Ala Ser Glu Glu Gly Trp Leu
       210                 215                 220
   Val Phe Asp Ile Thr Ala Thr Ser Asn His Trp Val Val Asn Pro Arg
   225                 230                 235                 240
   His Asn Leu Gly Leu Gln Leu Ser Val Glu Thr Leu Asp Gly Gln Ser
                   245                 250                 255
   Ile Asn Pro Lys Leu Ala Gly Leu Ile Gly Arg His Gly Pro Gln Asn
               260                 265                 270
   Lys Gln Pro Phe Met Val Ala Phe Phe Lys Ala Thr Glu Val His Phe
           275                 280                 285
   Arg Ser Ile Arg Ser Thr Gly Ser Lys Gln Arg Ser Gln Asn Arg Ser
       290                 295                 300
   Lys Thr Pro Lys Asn Gln Glu Ala Leu Arg Met Ala Asn Val Ala Glu
   305                 310                 315                 320
   Asn Ser Ser Ser Asp Gln Arg Gln Ala Cys Lys Lys His Glu Leu Tyr
                   325                 330                 335
   Val Ser Phe Arg Asp Leu Gly Trp Gln Asp Trp Ile Ile Ala Pro Glu
               340                 345                 350
   Gly Tyr Ala Ala Tyr Tyr Cys Glu Gly Glu Cys Ala Phe Pro Leu Asn
           355                 360                 365
   Ser Tyr Met Asn Ala Thr Asn His Ala Ile Val Gln Thr Leu Val His
       370                 375                 380
   Phe Ile Asn Pro Glu Thr Val Pro Lys Pro Cys Cys Ala Pro Thr Gln
   385                 390                 395                 400
   Leu Asn Ala Ile Ser Val Leu Tyr Phe Asp Asp Ser Ser Asn Val Ile
                   405                 410                 415
   Leu Lys Lys Tyr Arg Asn Met Val Val Arg Ala Cys Gly Cys His
               420                 425                 430
(2) INFORMATION FOR SEQ ID NO:18:
   (i) SEQUENCE CHARACTERISTICS:
      (A) LENGTH: 1873 base pairs
      (B) TYPE: nucleic acid
      (C) STRANDEDNESS: single
      (D) TOPOLOGY: linear
   (ii) MOLECULE TYPE: cDNA
   (vi) ORIGINAL SOURCE:
      (A) ORGANISM: MURIDAE
      (F) TISSUE TYPE: EMBRYO
   (ix) FEATURE:
      (A) NAME/KEY: CDS
      (B) LOCATION: 104..1393
      (D) OTHER INFORMATION: /note= "MOP1 (CDNA)"
   (xi) SEQUENCE DESCRIPTION: SEQ ID NO:18:
   CTGCAGCAAG TGACCTCGGG TCGTGGACCG CTGCCCTGCC CCCTCCGCTG CCACCTGGGG      60
   CGGCGCGGGC CCGGTGCCCC GGATCGCGCG TAGAGCCGGC GCG ATG CAC GTG CGC       115
                                                   Met His Val Arg
                                                     1
   TCG CTG CGC GCT GCG GCG CCA CAC AGC TTC GTG GCG CTC TGG GCG CCT      163
   Ser Leu Arg Ala Ala Ala Pro His Ser Phe Val Ala Leu Trp Ala Pro
     5                  10                  15                  20
   CTG TTC TTG CTG CGC TCC GCC CTG GCC GAT TTC AGC CTG GAC AAC GAG      211
   Leu Phe Leu Leu Arg Ser Ala Leu Ala Asp Phe Ser Leu Asp Asn Glu
                    25                  30                  35
   GTG CAC TCC AGC TTC ATC CAC CGG CGC CTC CGC AGC CAG GAG CGG CGG      259
   Val His Ser Ser Phe Ile His Arg Arg Leu Arg Ser Gln Glu Arg Arg
                40                  45                  50
   GAG ATG CAG CGG GAG ATC CTG TCC ATC TTA GGG TTG CCC CAT CGC CCG      307
   Glu Met Gln Arg Glu Ile Leu Ser Ile Leu Gly Leu Pro His Arg Pro
            55                  60                  65
   CGC CCG CAC CTC CAG GGA AAG CAT AAT TCG GCG CCC ATG TTC ATG TTG      355
   Arg Pro His Leu Gln Gly Lys His Asn Ser Ala Pro Met Phe Met Leu
        70                  75                  80
   GAC CTG TAC AAC GCC ATG GCG GTG GAG GAG AGC GGG CCG GAC GGA CAG      403
   Asp Leu Tyr Asn Ala Met Ala Val Glu Glu Ser Gly Pro Asp Gly Gln
    85                  90                  95                 100
   GGC TTC TCC TAC CCC TAC AAG GCC GTC TTC AGT ACC CAG GGC CCC CCT        451
   Gly Phe Ser Tyr Pro Tyr Lys Ala Val Phe Ser Thr Gln Gly Pro Pro
                   105                 110                 115
   TTA GCC AGC CTG CAG GAC AGC CAT TTC CTC ACT GAC GCC GAC ATG GTC        499
   Leu Ala Ser Leu Gln Asp Ser His Phe Leu Thr Asp Ala Asp Met Val
               120                 125                 130
   ATG AGC TTC GTC AAC CTA GTG GAA CAT GAC AAA GAA TTC TTC CAC CCT        547
   Met Ser Phe Val Asn Leu Val Glu His Asp Lys Glu Phe Phe His Pro
           135                 140                 145
   CGA TAC CAC CAT CGG GAG TTC CGG TTT GAT CTT TCC AAG ATC CCC GAG        595
   Arg Tyr His His Arg Glu Phe Arg Phe Asp Leu Ser Lys Ile Pro Glu
       150                 155                 160
   GGC GAA CGG GTG ACC GCA GCC GAA TTC AGG ATC TAT AAG GAC TAC ATC        643
   Gly Glu Arg Val Thr Ala Ala Glu Phe Arg Ile Tyr Lys Asp Tyr Ile
   165                 170                 175                 180
   CGG GAG CGA TTT GAC AAC GAG ACC TTC CAG ATC ACA GTC TAT CAG GTG        691
   Arg Glu Arg Phe Asp Asn Glu Thr Phe Gln Ile Thr Val Tyr Gln Val
                   185                 190                 195
   CTC CAG GAG CAC TCA GGC AGG GAG TCG GAC CTC TTC TTG CTG GAC AGC        739
   Leu Gln Glu His Ser Gly Arg Glu Ser Asp Leu Phe Leu Leu Asp Ser
               200                 205                 210
   CGC ACC ATC TGG GCT TCT GAG GAG GGC TGG TTG GTG TTT GAT ATC ACA        787
   Arg Thr Ile Trp Ala Ser Glu Glu Gly Trp Leu Val Phe Asp Ile Thr
           215                 220                 225
   GCC ACC AGC AAC CAC TGG GTG GTC AAC CCT CGG CAC AAC CTG GGC TTA        835
   Ala Thr Ser Asn His Trp Val Val Asn Pro Arg His Asn Leu Gly Leu
       230                 235                 240
   CAG CTC TCT GTG GAG ACC CTG GAT GGG CAG AGC ATC AAC CCC AAG TTG        883
   Gln Leu Ser Val Glu Thr Leu Asp Gly Gln Ser Ile Asn Pro Lys Leu
   245                 250                 255                 260
   GCA GGC CTG ATT GGA CGG CAT GGA CCC CAG AAC AAG CAA CCC TTC ATG        931
   Ala Gly Leu Ile Gly Arg His Gly Pro Gln Asn Lys Gln Pro Phe Met
                   265                 270                 275
   GTG GCC TTC TTC AAG GCC ACG GAA GTC CAT CTC CGT AGT ATC CGG TCC        979
   Val Ala Phe Phe Lys Ala Thr Glu Val His Leu Arg Ser Ile Arg Ser
               280                 285                 290
   ACG GGG GGC AAG CAG CGC AGC CAG AAT CGC TCC AAG ACG CCA AAG AAC       1027
   Thr Gly Gly Lys Gln Arg Ser Gln Asn Arg Ser Lys Thr Pro Lys Asn
           295                 300                 305
   CAA GAG GCC CTG AGG ATG GCC AGT GTG GCA GAA AAC AGC AGC AGT GAC        1075
   Gln Glu Ala Leu Arg Met Ala Ser Val Ala Glu Asn Ser Ser Ser Asp
       310                 315                 320
   CAG AGG CAG GCC TGC AAG AAA CAT GAG CTG TAC GTC AGC TTC CGA GAC        1123
   Gln Arg Gln Ala Cys Lys Lys His Glu Leu Tyr Val Ser Phe Arg Asp
   325                 330                 335                 340
   CTT GGC TGG CAG GAC TGG ATC ATT GCA CCT GAA GGC TAT GCT GCC TAC        1171
   Leu Gly Trp Gln Asp Trp Ile Ile Ala Pro Glu Gly Tyr Ala Ala Tyr
                   345                 350                 355
   TAC TGT GAG GGA GAG TGC GCC TTC CCT CTG AAC TCC TAC ATG AAC GCC        1219
   Tyr Cys Glu Gly Glu Cys Ala Phe Pro Leu Asn Ser Tyr Met Asn Ala
               360                 365                 370
   ACC AAC CAC GCC ATC GTC CAG ACA CTG GTT CAC TTC ATC AAC CCA GAC        1267
   Thr Asn His Ala Ile Val Gln Thr Leu Val His Phe Ile Asn Pro Asp
           375                 380                 385
   ACA GTA CCC AAG CCC TGC TGT GCG CCC ACC CAG CTC AAC GCC ATC TCT        1315
   Thr Val Pro Lys Pro Cys Cys Ala Pro Thr Gln Leu Asn Ala Ile Ser
       390                 395                 400
   GTC CTC TAC TTC GAC GAC AGC TCT AAT GTC ATC CTG AAG AAG TAC AGA        1363
   Val Leu Tyr Phe Asp Asp Ser Ser Asn Val Ile Leu Lys Lys Tyr Arg
   405                 410                 415                 420
   AAC ATG GTG GTC CGG GCC TGT GGC TGC CAC TAGCTCTTCC TGAGACCCTG          1413
   Asn Met Val Val Arg Ala Cys Gly Cys His
                   425                 430
   ACCTTTGCGG GGCCACACCT TTCCAAATCT TCGATGTCTC ACCATCTAAG TCTCTCACTG      1473
   CCCACCTTGG CGAGGAGAAC AGACCAACCT CTCCTGAGCC TTCCCTCACC TCCCAACCGG      1533
   AAGCATGTAA GGGTTCCAGA AACCTGAGCG TGCAGCAGCT GATGAGCGCC CTTTCCTTCT      1593
   GGCACGTGAC GGACAAGATC CTACCAGCTA CCACAGCAAA CGCCTAAGAG CAGGAAAAAT      1653
   GTCTGCCAGG AAAGTGTCCA GTGTCCACAT GGCCCCTGGC GCTCTGAGTC TTTGAGGAGT      1713
   AATCGCAAGC CTCGTTCAGC TGCAGCAGAA GGAAGGGCTT AGCCAGGGTG GGCGCTGGCG      1773
   TCTGTGTTGA AGGGAAACCA AGCAGAAGCC ACTGTAATGA TATGTCACAA TAAAACCCAT      1833
   GAATGAAAAA AAAAAAAAAA AAAAAAAAAA AAAAGAATTC                            1873
(2) INFORMATION FOR SEQ ID N0:19:
   (i) SEQUENCE CHARACTERISTICS:
      (A) LENGTH: 430 amino acids
      (B) TYPE: amino acid
      (D) TOPOLOGY: linear
   (ii) MOLECULE TYPE: protein
   (ix) FEATURE:
      (D) OTHER INFORMATION: /product= "mOP1-PP"
   (xi) SEQUENCE DESCRIPTION: SEQ ID NO:19:
(2) INFORMATION FOR SEQ ID NO:20:
   (i)SEQUENCE CHARACTERISTICS:
      (A) LENGTH: 1723 base pairs
      (B) TYPE: nucleic acid
      (C) STRANDEDNESS: single
      (D) TOPOLOGY: linear
   (ii)MOLECULE TYPE: cDNA
   (vi)ORIGINAL SOURCE:
      (A) ORGANISM: Homo sapiens
      (F) TISSUE TYPE: HIPPOCAMPUS
   (ix) FEATURE:
      (A) NAME/KEY: CDS
      (B) LOCATION: 490..1696
      (D) OTHER INFORMATION: /note= "hOP2 (cDNA)"
   (xi)SEQUENCE DESCRIPTION: SEQ ID NO:20:
   GGCGCCGGCA GAGCAGGAGT GGCTGGAGGA GCTGTGGTTG GAGCAGGAGG TGGCACGGCA      60
   GGGCTGGAGG GCTCCCTATG AGTGGCGGAG ACGGCCCAGG AGGCGCTGGA GCAACAGCTC     120
   CCACACCGCA CCAAGCGGTG GCTGCAGGAG CTCGCCCATC GCCCCTGCGC TGCTCGGACC     180
   GCGGCCACAG CCGGACTGGC GGGTACGGCG GCGACAGAGG CATTGGCCGA GAGTCCCAGT     240
   CCGCAGAGTA GCCCCGGCCT CGAGGCGGTG GCGTCCCGGT CCTCTCCGTC CAGGAGCCAG     300
   GACAGGTGTC GCGCGGCGGG GCTCCAGGGA CCGCGCCTGA GGCCGGCTGC CCGCCCGTCC     360
   CGCCCCGCCC CGCCGCCCGC CGCCCGCCGA GCCCAGCCTC CTTGCCGTCG GGGCGTCCCC     420
   AGGCCCTGGG TCGGCCGCGG AGCCGATGCG CGCCCGCTGA GCGCCCCAGC TGAGCGCCCC     480
   CGGCCTGCC ATG ACC GCG CTC CCC GGC CCG CTC TGG CTC CTG GGC CTG        528
   GCG CTA TGC GCG CTG GGC GGG GGC GGC CCC GGC CTG CGA CCC CCG CCC      576
   GGC TGT CCC CAG CGA CGT CTG GGC GCG CGC GAG CGC CGG GAC GTG CAG      624
   CGC GAG ATC CTG GCG GTG CTC GGG CTG CCT GGG CGG CCC CGG CCC CGC      672
   GCG CCA CCC GCC GCC TCC CGG CTG CCC GCG TCC GCG CCG CTC TTC ATG      720
   CTG GAC CTG TAC CAC GCC ATG GCC GGC GAC GAC GAC GAG GAC GGC GCG      768
   CCC GCG GAG CGG CGC CTG GGC CGC GCC GAC CTG GTC ATG AGC TTC GTT      816
   AAC ATG GTG GAG CGA GAC CGT GCC CTG GGC CAC CAG GAG CCC CAT TGG      864
   AAG GAG TTC CGC TTT GAC CTG ACC CAG ATC CCG GCT GGG GAG GCG GTC      912
   ACA GCT GCG GAG TTC CGG ATT TAC AAG GTG CCC AGC ATC CAC CTG CTC      960
   AAC AGG ACC CTC CAC GTC AGC ATG TTC CAG GTG GTC CAG GAG CAG TCC     1008
   AAC AGG GAG TCT GAC TTG TTC TTT TTG GAT CTT CAG ACG CTC CGA GCT     1056
   GGA GAC GAG GGC TGG CTG GTG CTG GAT GTC ACA GCA GCC AGT GAC TGC     1104
   TGG TTG CTG AAG CGT CAC AAG GAC CTG GGA CTC CGC CTC TAT GTG GAG     1152
   ACT GAG GAC GGG CAC AGC GTG GAT CCT GGC CTG GCC GGC CTG CTG GGT     1200
   CAA CGG GCC CCA CGC TCC CAA CAG CCT TTC GTG GTC ACT TTC TTC AGG     1248
   GCC AGT CCG AGT CCC ATC CGC ACC CCT CGG GCA GTG AGG CCA CTG AGG     1296
   AGG AGG CAG CCG AAG AAA AGC AAC GAG CTG CCG CAG GCC AAC CGA CTC     1344
   CCA GGG ATC TTT GAT GAC GTC CAC GGC TCC CAC GGC CGG CAG GTC TGC     1392
   CGT CGG CAC GAG CTC TAC GTC AGC TTC CAG GAC CTC GGC TGG CTG GAC        1440
   TGG GTC ATC GCT CCC CAA GGC TAC TCG GCC TAT TAC TGT GAG GGG GAG        1488
   TGC TCC TTC CCA CTG GAC TCC TGC ATG AAT GCC ACC AAC CAC GCC ATC        1536
   CTG CAG TCC CTG GTG CAC CTG ATG AAG CCA AAC GCA GTC CCC AAG GCG        1584
   TGC TGT GCA CCC ACC AAG CTG AGC GCC ACC TCT GTG CTC TAC TAT GAC        1632
   AGC AGC AAC AAC GTC ATC CTG CGC AAA CAC CGC AAC ATG GTG GTC AAG        1680
   GCC TGC GGC TGC CAC T GAGTCAGCCC GCCCAGCCCT ACTGCAG                    1723
(2) INFORMATION FOR SEQ ID NO:21:
   (i)SEQUENCE CHARACTERISTICS:
      (A) LENGTH: 402 amino acids
      (B) TYPE: amino acid
      (D) TOPOLOGY: linear
   (ii)MOLECULE TYPE: protein
   (ix)FEATURE:
      (A)OTHER INFORMATION: /product= "hOP2-PP"
   (xi)SEQUENCE DESCRIPTION: SEQ ID NO:21:
(2) INFORMATION FOR SEQ ID NO:22:
   (i) SEQUENCE CHARACTERISTICS:
      (A) LENGTH: 1926 base pairs
      (B) TYPE: nucleic acid
      (C) STRANDEDNESS: single
      (D TOPOLOGY: linear
   (ii) MOLECULE TYPE; cDNA
   (vi) ORIGINAL SOURCE:
      (A) ORGANISM: MURIDAE
      (F) TISSUE TYPE: EMBRYO
   (ix) FEATURE:
      (A) NAME/KEY: CDS
      (B) LOCATION: 93..1289
      (D) OTHER INFORMATION: /note= "mOP2 cDNA"
   (xi) SEQUENCE DESCRIPTION: SEQ ID NO:22:
   GCCAGGCACA GGTGCGCCGT CTGGTCCTCC CCGTCTGGCG TCAGCCGAGC          50
   CCGACCAGCT ACCAGTGGAT GCGCGCCGGC TGAAAGTCCG AG ATG GCT ATG CGT     104
                                                  Met Ala Met Arg
                                                    1
   CCC GGG CCA CTC TGG CTA TTG GGC CTT GCT CTG TGC GCG CTG GGA GGC    152
   Pro Gly Pro Leu Trp Leu Leu Gly Leu Ala Leu Cys Ala Leu Gly Gly
     5                  10                  15                  20
   GGC CAC GGT CCG CGT CCC CCG CAC ACC TGT CCC CAG CGT CGC CTG GGA    200
   Gly His Gly Pro Arg Pro Pro His Thr Cys Pro Gln Arg Arg Leu Gly
                    25                  30                  35
   GCG CGC GAG CGC CGC GAC ATG CAG CGT GAA ATC CTG GCG GTG CTC GGG      248
   Ala Arg Glu Arg Arg Asp Met Gln Arg Glu Ile Leu Ala Val Leu Gly
                40                  45                  50
   CTA CCG GGA CGG CCC CGA CCC CGT GCA CAA CCC GCG GCT GCC CGG CAG      296
   Leu Pro Gly Arg Pro Arg Pro Arg Ala Gln Pro Ala Ala Ala Arg Gln
            55                  60                  65
   CCA GCG TCC GCG CCC CTC TTC ATG TTG GAC CTA TAC CAC GCC ATG ACC      344
   Pro Ala Ser Ala Pro Leu Phe Met Leu Asp Leu Tyr His Ala Met Thr
        70                  75                  80
   GAT GAC GAC GAC GGC GGG CCA CCA CAG GCT CAC TTA GGC CGT GCC GAC      392
   Asp Asp Asp Asp Gly Gly Pro Pro Gln Ala His Leu Gly Arg Ala Asp
    85                  90                  95                 100
   CTG GTC ATG AGC TTC GTC AAC ATG GTG GAA CGC GAC CGT ACC CTG GGC      440
   Leu Val Met Ser Phe Val Asn Met Val Glu Arg Asp Arg Thr Leu Gly
                   105                 110                 115
   TAC CAG GAG CCA CAC TGG AAG GAA TTC CAC TTT GAC CTA ACC CAG ATC      488
   Tyr Gln Glu Pro His Trp Lys Glu Phe His Phe Asp Leu Thr Gln Ile
               120                 125                 130
   CCT GCT GGG GAG GCT GTC ACA GCT GCT GAG TTC CGG ATC TAC AAA GAA      536
   Pro Ala Gly Glu Ala Val Thr Ala Ala Glu Phe Arg Ile Tyr Lys Glu
           135                 140                 145
   CCC AGC ACC CAC CCG CTC AAC ACA ACC CTC CAC ATC AGC ATG TTC GAA      584
   Pro Ser Thr His Pro Leu Asn Thr Thr Leu His Ile Ser Met Phe Glu
       150                 155                 160
   GTG GTC CAA GAG CAC TCC AAC AGG GAG TCT GAC TTG TTC TTT TTG GAT      632
   Val Val Gln Glu His Ser Asn Arg Glu Ser Asp Leu Phe Phe Leu Asp
   165                 170                 175                 180
   CTT CAG ACG CTC CGA TCT GGG GAC GAG GGC TGG CTG GTG CTG GAC ATC      680
   Leu Gln Thr Leu Arg Ser Gly Asp Glu Gly Trp Leu Val Leu Asp Ile
                   185                 190                 195
   ACA GCA GCC AGT GAC CGA TGG CTG CTG AAC CAT CAC AAG GAC CTG GGA      728
   Thr Ala Ala Ser Asp Arg Trp Leu Leu Asn His His Lys Asp Leu Gly
               200                 205                 210
   CTC CGC CTC TAT GTG GAA ACC GCG GAT GGG CAC AGC ATG GAT CCT GGC      776
   Leu Arg Leu Tyr Val Glu Thr Ala Asp Gly His Ser Met Asp Pro Gly
           215                 220                 225
   CTG GCT GGT CTG CTT GGA CGA CAA GCA CCA CGC TCC AGA CAG CCT TTC      824
   Leu Ala Gly Leu Leu Gly Arg Gln Ala Pro Arg Ser Arg Gln Pro Phe
       230                 235                 240
   ATG GTA ACC TTC TTC AGG GCC AGC CAG AGT CCT GTG CGG GCC CCT CGG        872
   Met Val Thr Phe Phe Arg Ala Ser Gln Ser Pro Val Arg Ala Pro Arg
   245                 250                 255                 260
   GCA GCG AGA CCA CTG AAG AGG AGG CAG CCA AAG AAA ACG AAC GAG CTT        920
   Ala Ala Arg Pro Leu Lys Arg Arg Gln Pro Lys Lys Thr Asn Glu Leu
                   265                 270                 275
   CCG CAC CCC AAC AAA CTC CCA GGG ATC TTT GAT GAT GGC CAC GGT TCC        968
   Pro His Pro Asn Lys Leu Pro Gly Ile Phe Asp Asp Gly His Gly Ser
               280                 285                 290
   CGC GGC AGA GAG GTT TGC CGC AGG CAT GAG CTC TAC GTC AGC TTC CGT       1016
   Arg Gly Arg Glu Val Cys Arg Arg His Glu Leu Tyr Val Ser Phe Arg
           295                 300                 305
   GAC CTT GGC TGG CTG GAC TGG GTC ATC GCC CCC CAG GGC TAC TCT GCC       1064
   Asp Leu Gly Trp Leu Asp Trp Val Ile Ala Pro Gln Gly Tyr Ser Ala
       310                 315                 320
   TAT TAC TGT GAG GGG GAG TGT GCT TTC CCA CTG GAC TCC TGT ATG AAC       1112
   Tyr Tyr Cys Glu Gly Glu Cys Ala Phe Pro Leu Asp Ser Cys Met Asn
   325                 330                 335                 340
   GCC ACC AAC CAT GCC ATC TTG CAG TCT CTG GTG CAC CTG ATG AAG CCA       1160
   Ala Thr Asn His Ala Ile Leu Gln Ser Leu Val His Leu Met Lys Pro
                   345                 350                 355
   GAT GTT GTC CCC AAG GCA TGC TGT GCA CCC ACC AAA CTG AGT GCC ACC       1208
   Asp Val Val Pro Lys Ala Cys Cys Ala Pro Thr Lys Leu Ser Ala Thr
               360                 365                 370
   TCT GTG CTG TAC TAT GAC AGC AGC AAC AAT GTC ATC CTG CGT AAA CAC       1256
   Ser Val Leu Tyr Tyr Asp Ser Ser Asn Asn Val Ile Leu Arg Lys His
           375                 380                 385
   CGT AAC ATG GTG GTC AAG GCC TGT GGC TGC CAC TGAGGCCCCG CCCAGCATCC     1309
   Arg Asn Met Val Val Lys Ala Cys Gly Cys His
       390                 395
   TGCTTCTACT ACCTTACCAT CTGGCCGGGC CCCTCTCCAG AGGCAGAAAC CCTTCTATGT     1369
   TATCATAGCT CAGACAGGGG CAATGGGAGG CCCTTCACTT CCCCTGGCCA CTTCCTGCTA     1429
   AAATTCTGGT CTTTCCCAGT TCCTCTGTCC TTCATGGGGT TTCGGGGCTA TCACCCCGCC     1489
   CTCTCCATCC TCCTACCCCA AGCATAGACT GAATGCACAC AGCATCCCAG AGCTATGCTA     1549
   ACTGAGAGGT CTGGGGTCAG CACTGAAGGC CCACATGAGG AAGACTGATC CTTGGCCATC     1609
   CTCAGCCCAC AATGGCAAAT TCTGGATGGT CTAAGAAGGC CGTGGAATTC TAAACTAGAT     1669
   GATCTGGGCT CTCTGCACCA TTCATTGTGG CAGTTGGGAC ATTTTTAGGT ATAACAGACA 1729
   CATACACTTA GATCAATGCA TCGCTGTACT CCTTGAAATC AGAGCTAGCT TGTTAGAAAA 1789
   AGAATCAGAG CCAGGTATAG CGGTGCATGT CATTAATCCC AGCGCTAAAG AGACAGAGAC 1849
   AGGAGAATCT CTGTGAGTTC AAGGCCACAT AGAAAGAGCC TGTCTCGGGA GCAGGAAAAA 1909
   AAAAAAAAAC GGAATTC 1926
   (2) INFORMATION FOR SEQ ID NO:23:
      (i) SEQUENCE CHARACTERISTICS:
         (A) LENGTH: 399 amino acids
         (B) TYPE: amino acid
         (D) TOPOLOGY: linear
      (ii) MOLECULE TYPE: protein
      (ix) FEATURE:
         (D) OTHER INFORMATION: /product= "m0P2-PP"
      (xi) SEQUENCE DESCRIPTION: SEQ ID NO:23:
(2) INFORMATION FOR SEQ ID NO:24:
   (i) SEQUENCE CHARACTERISTICS:
      (A) LENGTH: 1368 base pairs
      (B) TYPE: nucleic acid
      (C) STRANDEDNESS: single
      (D) TOPOLOGY: linear
   (ii) MOLECULE TYPE: cDNA
   (ix) FEATURE:
      (A) NAME/KEY: CDS
      (B) LOCATION: 1..1368
      (D) OTHER INFORMATION:/STANDARD NAME="60A"
   (x) PUBLICATION INFORMATION:
      (A) AUTHORS: VHARTON, KRISTI A.; THOMSEN, GERALD H.; GELBERT, WILLIAM M.
      (B) TITLE: DROSOPHILA 60A GENE...
      (C) JOURNAL: PROC. NAT'L ACAD. SCI. USA
      (D) VOLUME: 88
      (E) RELEVANT RESIDUES IN SEQ ID NO:3: FROM 1 TO 1368
      (F) PAGES: 9214-9218
      (G) DATE: OCT - 1991
   (xi) SEQUENCE DESCRIPTION: SEQ ID NO:24:
   ATG TCG GGA CTG CGA AAC ACC TCG GAG GCC GTT GCA GTG CTC GCC TCC      48
   Met Ser Gly Leu Arg Asn Thr Ser Glu Ala Val Ala Val Leu Ala Ser
     1               5                  10                  15
   CTG GGA CTC GGA ATG GTT CTG CTC ATG TTC GTG GCG ACC ACG CCG CCG      96
   Leu Gly Leu Gly Met Val Leu Leu Met Phe Val Ala Thr Thr Pro Pro
                20                  25                  30
   GCC GTT GAG GCC ACC CAG TCG GGG ATT TAC ATA GAC AAC GGC AAG GAC     144
   Ala Val Glu Ala Thr Gln Ser Gly Ile Tyr Ile Asp Asn Gly Lys Asp
            35                  40                  45
   CAG ACG ATC ATG CAC AGA GTG CTG AGC GAG GAC GAC AAG CTG GAC GTC     192
   Gln Thr Ile Met His Arg Val Leu Ser Glu Asp Asp Lys Leu Asp Val
        50                  55                  60
   TCG TAC GAG ATC CTC GAG TTC CTG GGC ATC GCC GAA CGG CCG ACG CAC     240
   Ser Tyr Glu Ile Leu Glu Phe Leu Gly Ile Ala Glu Arg Pro Thr His
    65                  70                  75                  80
   CTG AGC AGC CAC CAG TTG TCG CTG AGG AAG TCG GCT CCC AAG TTC CTG     288
   Leu Ser Ser His Gln Leu Ser Leu Arg Lys Ser Ala Pro Lys Phe Leu
                    85                  90                  95
   CTG GAC GTC TAC CAC CGC ATC ACG GCG GAG GAG GGT CTC AGC GAT CAG      336
   Leu Asp Val Tyr His Arg Ile Thr Ala Glu Glu Gly Leu Ser Asp Gln
           100                 105                 110
   GAT GAG GAC GAC GAC TAC GAA CGC GGC CAT CGG TCC AGG AGG AGC GCC      384
   Asp Glu Asp Asp Asp Tyr Glu Arg Gly His Arg Ser Arg Arg Ser Ala
       115                 120                 125
   GAC CTC GAG GAG GAT GAG GGC GAG CAG CAG AAG AAC TTC ATC ACC GAC      432
   Asp Leu Glu Glu Asp Glu Gly Glu Gln Gln Lys Asn Phe Ile Thr Asp
       130                 135                 140
   CTG GAC AAG CGG GCC ATC GAC GAG AGC GAC ATC ATC ATG ACC TTC CTG      480
   Leu Asp Lys Arg Ala Ile Asp Glu Ser Asp Ile Ile Met Thr Phe Leu
   145                 150                 155                 160
   AAC AAG CGC CAC CAC AAT GTG GAC GAA CTG CGT CAC GAG CAC GGC CGT      528
   Asn Lys Arg His His Asn Val Asp Glu Leu Arg His Glu His Gly Arg
                   165                 170                 175
   CGC CTG TGG TTC GAC GTC TCC AAC GTG CCC AAC GAC AAC TAC CTG GTG      576
   Arg Leu Trp Phe Asp Val Ser Asn Val Pro Asn Asp Asn Tyr Leu Val
               180                 185                 190
   ATG GCC GAG CTG CGC ATC TAT CAG AAC GCC AAC GAG GGC AAG TGG CTG      624
   Met Ala Glu Leu Arg Ile Tyr Gln Asn Ala Asn Glu Gly Lys Trp Leu
           195                 200                 205
   ACC GCC AAC AGG GAG TTC ACC ATC ACG GTA TAC GCC ATT GGC ACC GGC      672
   Thr Ala Asn Arg Glu Phe Thr Ile Thr Val Tyr Ala Ile Gly Thr Gly
       210                 215                 220
   ACG CTG GGC CAG CAC ACC ATG GAG CCG CTG TCC TCG GTG AAC ACC ACC      720
   Thr Leu Gly Gln His Thr Met Glu Pro Leu Ser Ser Val Asn Thr Thr
   225                 230                 235                 240
   GGG GAC TAC GTG GGC TGG TTG GAG CTC AAC GTG ACC GAG GGC CTG CAC      768
   Gly Asp Tyr Val Gly Trp Leu Glu Leu Asn Val Thr Glu Gly Leu His
                   245                 250                 255
   GAG TGG CTG GTC AAG TCG AAG GAC AAT CAT GGC ATC TAC ATT GGA GCA      816
   Glu Trp Leu Val Lys Ser Lys Asp Asn His Gly Ile Tyr Ile Gly Ala
               260                 265                 270
   CAC GCT GTC AAC CGA CCC GAC CGC GAG GTG AAG CTG GAC GAC ATT GGA      864
   His Ala Val Asn Arg Pro Asp Arg Glu Val Lys Leu Asp Asp Ile Gly
           275                 280                 285
   CTG ATC CAC CGC AAG GTG GAC GAC GAG TTC CAG CCC TTC ATG ATC GGC      912
   Leu Ile His Arg Lys Val Asp Asp Glu Phe Gln Pro Phe Met Ile Gly
       290                 295                 300
   TTC TTC CGC GGA CCG GAG CTG ATC AAG GCG ACG GCC CAC AGC AGC CAC      960
   Phe Phe Arg Gly Pro Glu Leu Ile Lys Ala Thr Ala His Ser Ser His
   305                 310                 315                 320
   CAC AGG AGC AAG CGA AGC GCC AGC CAT CCA CGC AAG CGC AAG AAG TCG     1008
   His Arg Ser Lys Arg Ser Ala Ser His Pro Arg Lys Arg Lys Lys Ser
                   325                 330                 335
   GTG TCG CCC AAC AAC GTG CCG CTG CTG GAA CCG ATG GAG AGC ACG CGC     1056
   Val Ser Pro Asn Asn Val Pro Leu Leu Glu Pro Met Glu Ser Thr Arg
               340                 345                 350
   AGC TGC CAG ATG CAG ACC CTG TAC ATA GAC TTC AAG GAT CTG GGC TGG     1104
   Ser Cys Gln Met Gln Thr Leu Tyr Ile Asp Phe Lys Asp Leu Gly Trp
           355                 360                 365
   CAT GAC TGG ATC ATC GCA CCA GAG GGC TAT GGC GCC TTC TAC TGC AGC     1152
   His Asp Trp Ile Ile Ala Pro Glu Gly Tyr Gly Ala Phe Tyr Cys Ser
       370                 375                 380
   GGC GAG TGC AAT TTC CCG CTC AAT GCG CAC ATG AAC GCC ACG AAC CAT     1200
   Gly Glu Cys Asn Phe Pro Leu Asn Ala His Met Asn Ala Thr Asn His
   385                 390                 395                 400
   GCG ATC GTC CAG ACC CTG GTC CAC CTG CTG GAG CCC AAG AAG GTG CCC     1248
   Ala Ile Val Gln Thr Leu Val His Leu Leu Glu Pro Lys Lys Val Pro
                   405                 410                 415
   AAG CCC TGC TGC GCT CCG ACC AGG CTG GGA GCA CTA CCC GTT CTG TAC     1296
   Lys Pro Cys Cys Ala Pro Thr Arg Leu Gly Ala Leu Pro Val Leu Tyr
               420                 425                 430
   CAC CTG AAC GAC GAG AAT GTG AAC CTG AAA AAG TAT AGA AAC ATG ATT     1344
   His Leu Asn Asp Glu Asn Val Asn Leu Lys Lys Tyr Arg Asn Met Ile
           435                 440                 445
   GTG AAA TCC TGC GGG TGC CAT TGA                                     1368
   Val Lys Ser Cys Gly Cys His
       450                 455
(2) INFORMATION FOR SEQ ID NO:25:
   (i) SEQUENCE CHARACTERISTICS:
      (A) LENGTH: 455 amino acids
      (B) TYPE: amino acid
      (D) TOPOLOGY: linear
   (ii) MOLECULE TYPE: protein
   (xi) SEQUENCE DESCRIPTION: SEQ ID NO:25:
   Met Ser Gly Leu Arg Asn Thr Ser Glu Ala Val Ala Val Leu Ala Ser
    1               5                  10                  15
   Leu Gly Leu Gly Met Val Leu Leu Met Phe Val Ala Thr Thr Pro Pro
               20                  25                  30
   Ala Val Glu Ala Thr Gln Ser Gly Ile Tyr Ile Asp Asn Gly Lys Asp
           35                  40                  45
   Gln Thr Ile Met His Arg Val Leu Ser Glu Asp Asp Lys Leu Asp Val
       50                  55                  60
   Ser Tyr Glu Ile Leu Glu Phe Leu Gly Ile Ala Glu Arg Pro Thr His
   65                  70                  75                  80
   Leu Ser Ser His Gln Leu Ser Leu Arg Lys Ser Ala Pro Lys Phe Leu
                   85                  90                  95
   Leu Asp Val Tyr His Arg Ile Thr Ala Glu Glu Gly Leu Ser Asp Gln
               100                 105                 110
   Asp Glu Asp Asp Asp Tyr Glu Arg Gly His Arg Ser Arg Arg Ser Ala
           115                 120                 125
   Asp Leu Glu Glu Asp Glu Gly Glu Gln Gln Lys Asn Phe Ile Thr Asp
       130                 135                 140
   Leu Asp Lys Arg Ala Ile Asp Glu Ser Asp Ile Ile Met Thr Phe Leu
   145                 150                 155                 160
   Asn Lys Arg His His Asn Val Asp Glu Leu Arg His Glu His Gly Arg
                   165                 170                 175
   Arg Leu Trp Phe Asp Val Ser Asn Val Pro Asn Asp Asn Tyr Leu Val
               180                 185                 190
   Met Ala Glu Leu Arg Ile Tyr Gln Asn Ala Asn Glu Gly Lys Trp Leu
           195                 200                 205
   Thr Ala Asn Arg Glu Phe Thr Ile Thr Val Tyr Ala Ile Gly Thr Gly
       210                 215                 220
   Thr Leu Gly Gln His Thr Met Glu Pro Leu Ser Ser Val Asn Thr Thr
   225                 230                 235                 240
   Gly Asp Tyr Val Gly Trp Leu Glu Leu Asn Val Thr Glu Gly Leu His
                   245                 250                 255
   Glu Trp Leu Val Lys Ser Lys Asp Asn His Gly Ile Tyr Ile Gly Ala
               260                 265                 270
   His Ala Val Asn Arg Pro Asp Arg Glu Val Lys Leu Asp Asp Ile Gly
   275 280 285
   Leu Ile His Arg Lys Val Asp Asp Glu Phe Gln Pro Phe Met Ile Gly
   290 295 300
   Phe Phe Arg Gly Pro Glu Leu Ile Lys Ala Thr Ala His Ser Ser His
   305 310 315 320
   His Arg Ser Lys Arg Ser Ala Ser His Pro Arg Lys Arg Lys Lys Ser
   325 330 335
   Val Ser Pro Asn Asn Val Pro Leu Leu Glu Pro Met Glu Ser Thr Arg
   340 345 350
   Ser Cys Gln Met Gln Thr Leu Tyr Ile Asp Phe Lys Asp Leu Gly Trp
   355 360 365
   His Asp Trp Ile Ile Ala Pro Glu Gly Tyr Gly Ala Phe Tyr Cys Ser
   370 375 380
   Gly Glu Cys Asn Phe Pro Leu Asn Ala His Met Asn Ala Thr Asn His
   385 390 395 400
   Ala Ile Val Gln Thr Leu Val His Leu Leu Glu Pro Lys Lys Val Pro
   405 410 415
   Lys Pro Cys Cys Ala Pro Thr Arg Leu Gly Ala Leu Pro Val Leu Tyr
   420 425 430
   His Leu Asn Asp Glu Asn Val Asn Leu Lys Lys Tyr Arg Asn Met Ile
   435 440 445
   Val Lys Ser Cys Gly Cys His
   450 455
(2) INFORMATION FOR SEQ ID NO:26:
   (i) SEQUENCE CHARACTERISTICS:
      (A) LENGTH: amino acids
      (B) TYPE: amino acid
      (D) TOPOLOGY: linear
   (ii) MOLECULE TYPE: protein
   (iii) ORIGINAL SOURCE:
      (A) ORGANISM: Homo Sapiens
   (ix) FEATURE:
      (A) NAME/KEY: Protein
      (B) LOCATION: 1..102
      (D) OTHER INFORMATION: /note="BMP3"
   (xi) SEQUENCE DESCRIPTION: SEQ ID NO:26:
      (i)SEQUENCE CHARACTERISTICS:
         (A) LENGTH: 104 amino acids
         (B) TYPE: amino acid
         (C) STRANDEDNESS: single
         (D) TOPOLOGY: linear
      (ii)MOLECULE TYPE: protein
      (ix)FEATURE:
         (A) NAME/KEY: Protein
         (B) LOCATION: 1..104
         (D) OTHER INFORMATION: /note="BMP3"
      (xi)SEQUENCE DESCRIPTION: SEQ ID NO:26:
(2) INFORMATION FOR SEQ ID NO:27:
   (i) SEQUENCE CHARACTERISTICS:
      (A) LENGTH: 102 amino acids
      (B) TYPE: amino acid
      (C) STRANDEDNESS: single
      (D) TOPOLOGY: linear
   (ii) MOLECULE TYPE: protein
   (vi) ORIGINAL SOURCE:
      (A) ORGAMISH: HOMO SAPIENS
   (ix) FEATURE:
      (A) NAME/KEY: Protein
      (B) LOCATION: 1..102
      (D) OTHER INFORMATION: /note= "BMP5"
   (xi) SEQUENCE DESCRIPTION: SEQ ID NO:27:
   Cys Lys Lys His Glu Leu Tyr Val Ser Phe Arg Asp Leu Gly Trp Gln
   1 5 10 15
   Asp Trp Ile Ile Ala Pro Glu Gly Tyr Ala Ala Phe Tyr Cys Asp Gly
   20 25 30
   Glu Cys Ser Phe Pro Leu Asn Ala His Met Asn Ala Thr Asn His Ala
   35 40 45
   Ile Val Gln Thr Leu Val His Leu Met Phe Pro Asp His Val Pro Lys
   50 55 60
   Pro Cys Cys Ala Pro Thr Lys Leu Asn Ala Ile Ser Val Leu Tyr Phe
   65 70 75 80
   Asp Asp Ser Ser Asn Val Ile Leu Lys Lys Tyr Arg Asn Met Val Val
   85 90 95
   Arg Ser Cys Gly Cys His
   100
(2) INFORMATION FOR SEQ ID NO:28:
   (i) SEQUENCE CHARACTERISTICS:
      (A) LENGTH: 102 amino acids
      (B) TYPE: amino acid
      (C) STRANDEDNESS: single
      (D) TOPOLOGY: linear
   (ii) MOLECULE TYPE: protein
   (vi) ORIGINAL SOURCE:
      (A) ORGANISM: HOMO SAPIENS
   (ix) FEATURE:
      (A) NAME/KEY: Protein
      (B) LOCATION: 1..102
      (D) OTHER INFORMATION: /note= "BMP6"
   (xi) SEQUENCE DESCRIPTION: SEQ ID NO:28:
   Cys Arg Lys His Glu Leu Tyr Val Ser Phe Gln Asp Leu Gly Trp Gln
   1 5 10 15
   Asp Trp Ile Ile Ala Pro Lys Gly Tyr Ala Ala Asn Tyr Cys Asp Gly
   20 25 30
   Glu Cys Ser Phe Pro Leu Asn Ala His Met Asn Ala Thr Asn His Ala
   35 40 45
   Ile Val Gln Thr Leu Val His Leu Met Asn Pro Glu Tyr Val Pro Lys
   50 55 60
   Pro Cys Cys Ala Pro Thr Lys Leu Asn Ala Ile Ser Val Leu Tyr Phe
   65 70 75 80
   Asp Asp Asn Ser Asn Val Ile Leu Lys Lys Tyr Arg Trp Met Val Val
   85 90 95
   Arg Ala Cys Gly Cys His
   100
(2) INFORMATION FOR SEQ ID NO:29:
   (i) SEQUENCE CHARACTERISTICS:
      (A) LENGTH: 102 amino acids
      (B) TYPE: amino acid
      (D) TOPOLOGY: linear
   (ii) MOLECULE TYPE: protein
   (ix) FEATURE:
      (A) NAME/KEY: Protein
      (B) LOCATION: 1..102
      (D) OTHER INFORMATION: /label= OPX /note= "WHEREIN XAA AT EACH POS'N IS INDEPENDENTLY SELECTED FROM THE RESIDUES OCCURRING AT THE CORRESPONDING POS'N IN THE C-TERMINAL SEQUENCE OF MOUSE OR HUMAN OP1 OR OP2 (SEE SEQ. ID NOS. 5,6,7 and 8 or 16,18,20 and 22.)"
   (xi) SEQUENCE DESCRIPTION: SEQ ID NO:29:
   Cys Xaa Xaa His Glu Leu Tyr Val Xaa Phe Xaa Asp Leu Gly Trp Xaa
   1 5 10 15
   Asp Trp Xaa Ile Ala Pro Xaa Gly Tyr Xaa Ala Tyr Tyr Cys Glu Gly
   20 25 30
   Glu Cys Xaa Phe Pro Leu Xaa Ser Xaa Met Asn Ala Thr Asn His Ala
   35 40 45
   Ile Xaa Gln Xaa Leu Val His Xaa Xaa Xaa Pro Xaa Xaa Val Pro Lys
   50 55 60
   Xaa Cys Cys Ala Pro Thr Xaa Leu Xaa Ala Xaa Ser Val Leu Tyr Xaa
   65 70 75 80
   Asp Xaa Ser Xaa Asn Val Xaa Leu Xaa Lys Xaa Arg Asn Met Val Val
   85 90 95
   Xaa Ala Cys Gly Cys His
   100
(2) INFORMATION FOR SEQ ID NO:30:
   (i)SEQUENCE CHARACTERISTICS:
      (A) LENGTH: 97 amino acids
      (B) TYPE: amino acids
      (C) TOPOLOGY: linear
   (ii)MOLECULE TYPE: protein
   (ix)FEATURE:
      (A) NAME: Generic Sequence 5
      (D) OTHER INFORMATION: wherein each Xaa is independently selected from a group of one or more specified amino acids as defined in the specification.
   (xi)SEQUENCE DESCRIPTION: SEQ ID NO:30:
   Leu Xaa Xaa Xaa Phe
   1 5
   Xaa Xaa Xaa Gly Trp Xaa Xaa Trp Xaa
   10
   Xaa Xaa Pro Xaa Xaa Xaa Xaa Ala
   15 20
   Xaa Tyr Cys Xaa Gly Xaa Cys Xaa
   25 30
   Xaa Pro Xaa Xaa Xaa Xaa Xaa
   35
   Xaa Xaa Xaa Asn His Ala Xaa Xaa
   40 45
   Xaa Xaa Xaa Xaa Xaa Xaa Xaa Xaa
   50
   Xaa Xaa Xaa Xaa Xaa Xaa Xaa Cys
   55 60
   Cys Xaa Pro Xaa Xaa Xaa Xaa Xaa
   65
   Xaa Xaa Xaa Leu Xaa Xaa Xaa
   70 75
   Xaa Xaa Xaa Xaa Val Xaa Leu Xaa
   80
   Xaa Xaa Xaa Xaa Met Xaa Val Xaa
   85 90
   Xaa Cys Xaa Cys Xaa
   95
(2) INFORMATION FOR SEQ ID NO:31:
   (i)SEQUENCE CHARACTERISTICS:
      (A) LENGTH: 102 amino acids
      (B) TYPE: amino acids
      (C) TOPOLOGY: linear
   (ii)MOLECULE TYPE: protein
   (ix)FEATURE:
      (A) NAME: Generic Sequence 6
      (D) OTHER INFORMATION: wherein each Xaa is independently selected from a group of one or more specified amino acids as defined in the specification.
   (xi) SEQUENCE DESCRIPTION: SEQ ID NO:31:
   Cys Xaa Xaa Xaa Xaa Leu Xaa Xaa Xaa Phe
    1                   5                   10
   Xaa Xaa Xaa Gly Trp Xaa Xaa Trp Xaa
                       15
   Xaa Xaa Pro Xaa Xaa Xaa Xaa Ala
    20                      25
   Xaa Tyr Cys Xaa Gly Xaa Cys Xaa
            30                      35
   Xaa Pro Xaa Xaa Xaa Xaa Xaa
                       40
   Xaa Xaa Xaa Asn His Ala Xaa Xaa
            45                      50
   Xaa Xaa Xaa Xaa Xaa Xaa Xaa Xaa
                       55
   Xaa Xaa Xaa Xaa Xaa Xaa Xaa Cys
        60                      65
   Cys Xaa Pro Xaa Xaa Xaa Xaa Xaa
                   70
   Xaa Xaa Xaa Leu Xaa Xaa Xaa
    75                      80
   Xaa Xaa Xaa Xaa Val Xaa Leu Xaa
                   85
   Xaa Xaa Xaa Xaa Met Xaa Val Xaa
    90                      95
   Xaa Cys Xaa Cys Xaa
           100
(2) INFORMATION FOR SEQ ID NO:32:
   (i) SEQUENCE CHARACTERISTICS:
      (A) LENGTH: 1238 base pairs, 372 amino acids
      (B) TYPE: nucleic acid, amino acid
      (C) STRANDEDNESS: single
      (D) TOPOLOGY: linear
   (ii) MOLECULE TYPE: cDNA
   (iii) ORIGINAL SOURCE:
      (A) ORGANISM: human
      (F) TISSUE TYPE: BRAIN
   (iv) FEATURE:
      (A) NAME/KEY: CDS
      (B) LOCATION:
      (D) OTHER INFORMATION: /product= "GDF-1" /note= "GDF-1 CDNA"
   (x) PUBLICATION INFORMATION:
      (A) AUTHORS: Lee, Se-Jin
      (B) TITTLE: Expression of Growth/Differentiation Factor 1
      (C) JOURNAL: Proc. Nat'l Acad. Sci.
      (D) VOLUME: 88
      (E) RELEVANT RESIDUES: 1-1238
      (F) PAGES: 4250-4254
      (G) DATE: May-1991
   (xi) SEQUENCE DESCRIPTION: SEQ ID NO:32:
(2) INFORMATION FOR SEQ ID NO:33:
   (i) SEQUENCE CHARACTERISTICS:
      (A) LENGTH: 372 amino acids
      (B) TYPE: amino acid
      (C) STRANDEDNESS: single
      (D) TOPOLOGY: linear
   (ii) MOLECULE TYPE: cDNA
   (iii) HYPOTHETICAL: NO
   (iv) ANTI-SENSE: NO
   (vi) ORIGINAL SOURCE:
      (A) ORGANISM: human
      (F) TISSUE TYPE: BRAIN
   (ix) FEATURE:
      (A) NAME/KEY: CDS
      (B) LOCATION:
      (D) OTHER INFORMATION: /function= /product= "GDF-1"
   (xi) SEQUENCE DESCRIPTION: SEQ ID NO:33: